(19) Europäisches Patentamt
European Patent Office
Office européen des brevets

(11) EP 2 338 488 A1

(12) EUROPEAN PATENT APPLICATION

(43) Date of publication:
29.06.2011 Bulletin 2011/26

(21) Application number: 10185819.9

(22) Date of filing: 25.05.2007

(51) Int Cl.:
A61K 31/435 (2006.01) A61K 31/337 (2006.01)
A61K 31/555 (2006.01) A61P 35/00 (2006.01)
A61P 35/04 (2006.01)

(84) Designated Contracting States:
AT BE BG CH CY CZ DE DK EE ES FI FR GB GR HU IE IS IT LI LT LU LV MC MT NL PL PT RO SE SI SK TR

(30) Priority: 26.05.2006 US 808555 P
16.11.2006 US 859241 P

(62) Document number(s) of the earlier application(s) in accordance with Art. 76 EPC:
07795337.0 / 2 094 268

(71) Applicant: Bayer HealthCare, LLC
Tarrytown,
New York 10591 (US)

(72) Inventor: Kelley, Susan
Woodbridge, CT 06525 (US)

(74) Representative: Kilger, Ute
Forrester & Boehmert
Pettenkoferstrasse 20-22
80336 München (DE)

Remarks:
This application was filed on 01-10-2010 as a divisional application to the application mentioned under INID code 62.

# (54) Drug combinations with substituted diaryl ureas for the treatment of cancer

(57) The present invention relates to drug combinations and pharmaceutical compositions for treating cancer such as non-small cell lung carcinoma, said combination comprising (1) at least one substituted-diaryl urea such as BAY 43-9006, (2) at least one taxane such as Paclitaxel (Taxol®), Docetaxel (Taxotere®) and AbraxaneTM and (3) at least one platinum complex antineoplastic nucleic acid binding agent such as carboplatin (Paraplatin®), oxaplatin (Eloxatin®) and cisplatin (Platinol®), where any of these components can be present in the form of a pharmaceutically acceptable salt or other derivative thereof.

EP 2 338 488 A1

## Description

FIELD OF THE INVENTION

This invention relates to drug combinations of substituted diaryl urea kinase inhibitors with two other chemotherapeutic agents and their use in treating hyper-proliferative disorders such as cancer, in humans and other mammals.

BACKGROUND OF THE INVENTION

Non-small cell lung cancer (NSCLC) is a heterogeneous aggregate of at least three different histologies of lung cancer including epidermoid or squamous carcinoma, adenocarcinoma, and large cell carcinoma. They are often classified together because, in their localized states, all have the potential for cure with surgical procedure. At diagnosis, patients with NSCLC can be divided into three groups that reflect the extent of disease and treatment approach. The first group is characterized by surgically resectable tumors, and can be staged I or II. This is the group with the best prognosis, depending on a variety of tumor and host factors. The second group includes patients with advanced lung cancer and can be sub-categorized as local or regional. Radiation therapy with or without chemotherapy or other therapy modalities is the preferred mode of treatment. The final group comprises patients with distant metastasis. This group can be treated with radiation therapy or chemotherapy for palliation of symptoms from the primary tumor. Cisplatin-based chemotherapy has been associated with short-term palliation of symptoms and a small survival advantage.

For operable patients, prognosis is adversely influenced by the presence of pulmonary symptoms, large tumor size (>3 centimeters), and presence of the Erb-2 oncoprotein.[1-6] Other factors that have been identified as adverse prognostic factors in some series of patients with resectable non-small cell lung cancer include mutation of the K-ras gene, vascular invasion, and increased numbers of blood vessels in the tumor specimen [3,7,8]

References:

1. Albain KS, Crowley JJ, LeBlanc M, et al.: Survival determinants in extensive-stage non-small-cell lung cancer: the Southwest Oncology Group experience. Journal of Clinical Oncology 9(9): 1618-1626, 1991.
2. Macchiarini P, Fontanini G, Hardin MJ, et al.: Blood vessel invasion by tumor cells predicts recurrence in completely resected Tl N0 M0 non-small-cell lung cancer. Journal of Thoracic and Cardiovascular Surgery 106(1): 80-89, 1993.
3. Harpole DH, Herndon JE, Wolfe WG, et al.: A prognostic model of recurrence and death in stage I non-small cell lung cancer utilizing presentation, histopathology, and oncoprotein expression. Cancer Research 55(1): 51-56, 1995.
4. Ichinose Y, Yano T, Asoh H, et al.: Prognostic factors obtained by a pathologic examination in completely resected non-small-cell lung cancer: an analysis in each pathologic stage. Journal of Thoracic and Cardiovascular Surgery 110(3): 601-605, 1995.
5. Martini N, Bains MS, Burt ME, et al.: Incidence of local recurrence and second primary tumors in resected stage I lung cancer. Journal of Thoracic and Cardiovascular Surgery 109(1): 120-129, 1995.
6. Strauss GM, Kwiatkowski DJ, Harpole DH, et al.: Molecular and pathologic markers in stage I non-small-cell carcinoma of the lung. Journal of Clinical Oncology 13(5): 1265-1279, 1995.
7. Slebos RJ, Kibbelaar RE, Dalesio O, et al.: K-RAS oncogene activation as a prognostic marker in adenocarcinoma of the lung. New England Journal of Medicine 323(9): 561-565,1990.
8. Fontanini G, Bigini D, Vignati S, et al.: Microvessel count predicts metastatic disease and survival in non-small cell lung cancer. Journal of Pathology 177: 57-63, 1995.


Prior to initiating treatment of any patient with lung cancer, a review of pathologic material by an experienced lung cancer pathologist can be important since the chemo-responsive small cell lung cancer can be confused with non-small cell carcinoma [1]. Histologic classification of non-small cell lung cancer can be squamous cell (epidermoid) carcinoma, adenocarcinoma, large cell carcinoma, adenosquamous carcinoma, and undifferentiated carcinoma. Similarly the staging procedure can be performed using the guidelines set by the American Joint Committee on Cancer (AJCC). Since the classification is based on characterization of the primary tumor (T), measurement of the size of lymph node (N), and assessment of distant metastasis (M), it is shortly known as TNM classification system for NSCLC.

In advanced-stage disease, chemotherapy has been reported to offer modest improvements in median survival. [1,2] Chemotherapy has been reported to produce short-term improvement in disease-related symptoms, while combination chemotherapy has been reported to yield symptomatic relief [3,4].

Several new agents, including paclitaxel (Taxol), docetaxel (Taxotere), topotecan, irinotecan, vinorelbine, and gemcitabine have been shown to be active in the treatment of advanced NSCLC.

References:

1. Souquet PJ, Chauvin F, Boissel JP, et al.: Polychemotherapy in advanced non small cell lung cancer: a meta-analysis. Lancet 342(8862): 19-21, 1993.
2. Non-small Cell Lung Cancer Collaborative Group: Chemotherapy in non-small cell lung cancer: a meta-analysis using updated data on individual patients from 52 randomised clinical trials. British Medical Journal 311(7010): 899-909,1995.
3. Hardy JR, Noble T, Smith IE: Symptom relief with moderate dose chemotherapy (mitomycin-C, vinblastine and cisplatin) in advanced non-small cell lung cancer. British Journal of Cancer 60(5): 764-766, 1989.
4. Ellis PA, Smith IE, Hardy JR, et al.: Symptom relief with MVP (mitomycin C, vinblastine and cisplatin) chemotherapy in advanced non-small-cell lung cancer. British Journal of Cancer 71(2): 366-370, 1995.

Cisplatin-containing and carboplatin-containing combination chemotherapy regimens are reported to produce objective response rates (including a few complete responses) that are higher than those achieved with single-agent chemotherapy. Although toxic effects may vary, outcome is similar with most cisplatin-containing regimens; a randomized trial comparing 5 cisplatin-containing regimens reported no significant difference in response, duration of response, or survival.[1] Patients with good performance status and a limited number of sites of distant metastases were reported to have superior response and survival when given chemotherapy when compared to other patients.[2]. Two small phase II studies reported that paclitaxel (Taxol) has single-agent activity, with response rates in the range of 21% to 24%.[4, 5] Reports of paclitaxel combinations have shown relatively high response rates, significant 1 year survival, and palliation of lung cancer symptoms.[6] With the paclitaxel plus carboplatin regimen, response rates have been in the range of 27% to 53% with 1-year survival rates of 32% to 54%.[6,7] The combination of cisplatin and paclitaxel was shown to have a higher response rate than the combination of cisplatin and etoposide.[8]

Although these results support further evaluation of chemotherapeutic approaches for both metastatic and locally advanced non-small cell lung cancer (NSCLC), efficacy of current programs, it is reported that no specific regimen can be regarded as standard therapy. Radiation therapy may be effective in palliating symptomatic local involvement with NSCLC such as tracheal, esophageal, or bronchial compression, bone or brain metastases, pain, vocal cord paralysis, hemoptysis, or superior vena cava syndrome.

The following Chemotherapy regimens have been associated with similar survival outcomes: cisplatin plus vinblastine plus mitomycin [15]
cisplatin plus vinorelbine [3]
cisplatin plus paclitaxel [8]
cisplatin plus gemcitabine [16]
carboplatin plus paclitaxel [6,7]

References:

1. Weick JK, Crowley J, Natale RB, et al.: A randomized trial of five cisplatin-containing treatments in patients with metastatic non-small-cell lung cancer: a Southwest Oncology Group study. Journal of Clinical Oncology 9(7): 1157-1162, 1991.
2. O'Connell JP, Kris MG, Gralla RJ, et al.: Frequency and prognostic importance of pretreatment clinical characteristics in patients with advanced non-small-cell lung cancer treated with combination chemotherapy. Journal of Clinical Oncology 4(11): 1604-1614,1986.
3. Le Chevalier T, Brisgand D, Douillard JY, et al.: Randomized study of vinorelbine and cisplatin versus vindesine and cisplatin versus vinorelbine alone in advanced non-small-cell lung cancer: results of a European multicenter trial including 612 patients. Journal of Clinical Oncology 12(2): 360-367, 1994.
4. Chang AY, Kim K, Glick J, et al.: Phase II study of taxol, merbarone, and piroxantrone in stage IV non-small-cell lung cancer: the Eastern Cooperative Oncology Group results. Journal of the National Cancer Institute 85(5): 388-394, 1993.
5. Murphy WK, Fossella FV, Winn RJ, et al.: Phase II study of taxol in patients with untreated advanced non-small-cell lung cancer. Journal of the National Cancer Institute 85(5): 384-388, 1993.
6. Johnson DH, Paul DM, Hande KR, et al.: Paclitaxel plus carboplatin in advanced non-small-cell lung cancer: a phase II trial. Journal of Clinical Oncology 14(7): 2054-2060, 1996.
7. Langer CJ, Leighton JC, Comis RL, et al.: Paclitaxel and carboplatin in combination in the treatment of advanced

non-small-cell lung cancer: a phase II toxicity, response, and survival analysis. Journal of Clinical Oncology 13(8): 1860-1870, 1995.
8. Bonomi P, Kim K, Chang A, et al.: Phase III trial comparing etoposide (E) cisplatin (C) versus taxol (T) with cisplatin-G-CSF(G) versus taxol-cisplatin in advanced non-small cell lung cancer. An Eastern Cooperative Oncology Group (ECOG) trial. Proceedings of the American Society of Clinical Oncology 15: A-1145, 382, 1996.
9. Souquet PJ, Chauvin F, Boissel JP, et al.: Polychemotherapy in advanced non small cell lung cancer: a meta-analysis. Lancet 342(8862): 19-21, 1993.

The lung is also frequently the site of second primary malignancies in patients with primary lung cancers. Determining whether the new lesion is a new primary cancer or a metastasis may be difficult. Studies have indicated that in the majority of patients the new lesion is a second primary tumor, and following resection some patients may achieve long-term survival. Thus, if the first primary tumor has been controlled, the second primary tumor should be resected if possible. [8,9]

It is reported that the use of chemotherapy has produced objective responses and small improvement in survival for patients with metastatic disease.[10]

Treatment options:

1. Palliative radiation therapy.
2. Chemotherapy alone. For patients who have not received prior chemotherapy, the following regimens are associated with similar survival outcomes:

    cisplatin plus vinblastine plus mitomycin [13]
    cisplatin plus vinorelbine [14]
    cisplatin plus paclitaxel [15]
    cisplatin plus gemcitabine [16]
    carboplatin plus paclitaxel [17,18]

3. Surgical resection of isolated cerebral metastasis (highly selected patients).[6]
4. Laser therapy or interstitial radiation therapy for endobronchial lesions.[19]
5. Stereotactic radiosurgery (highly selected patients).[3,5]

References:

1. Patchell RA, Tibbs PA, Walsh JW, et al.: A randomized trial of surgery in the treatment of single metastases to the brain. New England Journal of Medicine 322(8): 494-500, 1990.
2. Mandell L, Hilaris B, Sullivan M, et al.: The treatment of single brain metastasis from non-oat cell lung carcinoma: surgery and radiation versus radiation therapy alone. Cancer 58(3): 641-649, 1986.
3. Loeffler JS, Kooy HM, Wen PY, et al.: The treatment of recurrent brain metastases with stereotactic radiosurgery. Journal of Clinical Oncology 8(4): 576-582, 1990.
4. DeAngelis LM, Mandell LR, Thaler HT, et al.: The role of postoperative radiotherapy after resection of single brain metastases. Neurosurgery 24(6): 798-805, 1989.
5. Alexander E, Moriarty TM, Davis RB, et al.: Stereotactic radiosurgery for the definitive, noninvasive treatment of brain metastases. Journal of the National Cancer Institute 87(1): 34-40, 1995.
6. Arbit E, Wronski M, Burt M, et al.: The treatment of patients with recurrent brain metastases: a retrospective analysis of 109 patients with nonsmall cell lung cancer. Cancer 76(5): 765-773, 1995.
7. Hazuka MB, Kinzie JJ: Brain metastases: results and effects of re-irradiation. International Journal of Radiation Oncology, Biology, Physics 15(2): 433-437, 1988.
8. Salerno TA, Munro DD, Blundell PE, et al.: Second primary bronchogenic carcinoma: life-table analysis of surgical treatment. Annals of Thoracic Surgery 27(1): 3-6, 1979.
9. Yellin A, Hill LR, Benfield JR: Bronchogenic carcinoma associated with upper aerodigestive cancer. Journal ofThoracic and Cardiovascular Surgery 91(5): 674-683, 1986.
10. Souquet PJ, Chauvin F, Boissel JP, et al.: Polychemotherapy in advanced non small cell lung cancer: a meta-analysis. Lancet 342(8862): 19-21, 1993.

11. Ellis PA, Smith IE, Hardy JR, et al.: Symptom relief with MVP (mitomycin C, vinblastine and cisplatin) chemotherapy in advanced non-small-cell lung cancer. British Journal of Cancer 71(2): 366-370, 1995.
12. Medical Research Council Lung Cancer Working Party: Randomized trial of etoposide cyclophosphamide methotrexate and vincristine versus etoposide and vincristine in the palliative treatment of patients with small-cell lung cancer and poor prognosis. British Journal of Cancer 67(Suppl 20): A-4;2, 14, 1993.
13. Veeder MH, Jett JR, Su JQ, et al.: A phase III trial of mitomycin C alone versus mitomycin C, vinblastine, and cisplatin for metastatic squamous cell lung carcinoma. Cancer 70(9): 2281-2287,1992.
14. Le Chevalier T, Brisgand D, Douillard JY, et al.: Randomized study of vinorelbine and cisplatin versus vindesine and cisplatin versus vinorelbine alone in advanced non-small-cell lung cancer: results of a European multicenter trial including 612 patients. Journal of Clinical Oncology 12(2): 360-367, 1994.
15. Bonomi P, Kim K, Chang A, et al.: Phase III trial comparing etoposide (E) cisplatin (C) versus taxol (T) with cisplatin-G-CSF(G) versus taxol-cisplatin in advanced non-small cell lung cancer. An Eastern Cooperative Oncology Group (ECOG) trial. Proceedings of the American Society of Clinical Oncology 15: A-1145, 382, 1996.
16. Rosell R, Tonato M, Sandler A: The activity of gemcitabine plus cisplatin in randomized trials in untreated patients with advanced non-small cell lung cancer. Seminars in Oncology 25(4 suppl 9): 27-34, 1998.
17. Johnson DH, Paul DM, Hande KR, et al.: Paclitaxel plus carboplatin in advanced non-small-cell lung cancer: a phase II trial. Journal of Clinical Oncology 14(7): 2054-2060, 1996.
18. Langer CJ, Leighton JC, Comis RL, et al.: Paclitaxel and carboplatin in combination in the treatment of advanced non-small-cell lung cancer: a phase II toxicity, response, and survival analysis. Journal of Clinical Oncology 13(8): 1860-1870, 1995.
19. Miller JI, Phillips TW: Neodymium:YAG laser and brachytherapy in the management of inoperable bronchogenic carcinoma. Annals of Thoracic Surgery 50(2): 190-196, 1990.

Substituted Diarylureas are a class of serine-threonine kinase inhibitors as well as tyrosine kinase inhibitors known in the art (Smith et al., Bioorg. Med. Chem. Lett. 2001, 11, 2775-2778, Lowinger et al., Clin. Cancer Res. 2000, 6(suppl.), 335, Lyons et al., Endocr.-Relat. Cancer 2001, 8, 219-225, Lowinger et al., Curr. Pharm. Design 2002, 8, 99-110). Omega-Carboxyaryl diphenyl ureas are disclosed in WO00/42012 and WO00/41698. In particular, it has been discovered that the diphenyl urea of formula (A),

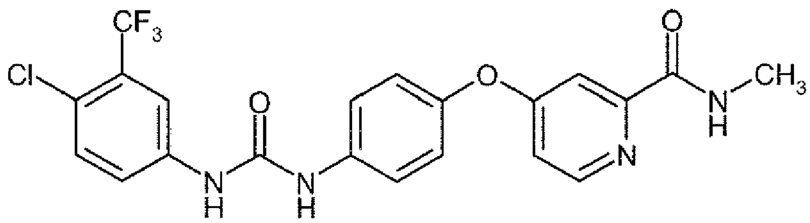

(A)

also referred as "BAY 43-9006" or "4-{4-[({[4-chloro-3-(trifluoromethyl)phenyl]amino}-carbonyl)amino]phenoxy}-N-methylpyridine-2-carboxamide" or "N-(4-chloro-3-(trifluoromethyl)phenyl-N'-(4-(2-(N-methylcarbamoyl)-4-pyridyloxy)phenyl) urea"or "4{4-[3-(4-chloro-3-trifluoromethylphenyl)-ureido]-phenoxy}-pyridine-2-carboxylic acid methyl amide"and its pharmaceutically acceptable salts are potent inhibitors of raf, VEGFR-2, p38, PDGFR and/or flt-3 signaling kinases. See, e.g., US 20050038080. These enzymes are all molecular targets of interest for the treatment of hyper-proliferative diseases, including cancer. Combinations of Sorafenib (a drug which contains the tosylate salt of BAY 43-9006 as an active ingredient) with carboplatin and paclitaxel are reported to be undergoing Phase III clinical trials for treating Unresectable Stage III or Stage IV Melanoma and Recurrent Ovarian Cancer, Primary Peritoneal Cancer and Fallopian Tube Cancer. (See www.clinicaltrials.gov.

The use of the substituted diaryl urea, BAY 43-9006, in treating non-small cell lung cancer has been disclosed. See Lee and McCubrey, Curr. Opin. Investig. Drugs, 4:657-63, June 2003 and Wilhelm et al., Cancer Res., 64:7099-7109, 2004, WO/04096224 (Boehringer; Hilberg et al.) published November 11,2004; April 29, 2003.

WO 03/047579 relates to the use of substituted diaryl ureas in combination with cytotoxic or cytostatic compounds for treating cancer.

Methods for preparing substituted diaryl ureas such as BAY 43-9006 are described in the following U.S. applications:

09/425,228, filed October 22, 1999;

09/722,418 filed November 28, 2000
09/758,547, filed January 12, 2001;
09/838,285, filed April 20, 2001;
09/838,286, filed April 20, 2001; and

DESCRIPTION OF THE INVENTION

The present invention provides drug combinations, pharmaceutical compositions, and methods for treating diseases and conditions, including, but not limited to, cell proliferative disorders such as cancer, including but not limited to colon, gastric, lung, pancreatic, ovarian, prostate, leukemia, melanoma, hepatocellular, renal, head and neck, glioma, and mammary cancers. The drug combinations comprise (1) at least one substituted-diaryl urea of Formula I (defined below), (2) at least one taxane such as Paclitaxel (Taxol®), Docetaxel (Taxotere®) and AbraxaneTM and (3) at least one platinum complex antineoplastic nucleic acid binding agent such as carboplatin (Paraplatin®), oxaplatin (Eloxatin®) and cisplatin (Platinol®), where any of these components can be present in the form of a pharmaceutically acceptable salt or other known derivative.

In a preferred embodiment of the drug combination, the substituted diaryl urea is Sorafenib, the taxane is paclitaxel and the platinum complex is carboplatin.

The invention also relates to pharmaceutical compositions which comprise one or more pharmaceutically acceptable carrier molecules and quantities of substituted diaryl urea compound of Formula I (defined below), a taxane (e.g., paclitaxel) and a platinum complex (e.g., carboplatin), in amounts which are jointly effective for treating a cancer, where any of these components can be present in the form of a pharmaceutically acceptable salt or other common derivative.

The methods can comprise, e.g., administering (1) a substituted diaryl urea compound of Formula I (e.g. Sorafenib); (2) a taxane (e.g. paclitaxel) and (3) a platinum complex (e.g., carboplatin) or pharmaceutically-acceptable salts or derivatives thereof, etc.

In particular embodiments of this invention, the active components of the drug combination are administered to a patient by oral delivery and/or by intravenous injection or infusion.

In particular embodiments of this invention, the substituted diaryl urea compound of Formula I is administered simultaneously with the taxane (e.g. paclitaxel) and platinum complex (e.g. carboplatin) to a patient with cancer, in the same formulation or in separate formulations, optionally using different administration routes. Administration can also be sequentially, in any order.

In particular embodiments of this invention, the substituted diaryl urea compounds of Formula I (e.g., Sorafenib) are administered in tandem with the taxane (e.g., paclitaxel) and platinum complex (e.g., carboplatin), wherein the substituted diaryl urea compound of Formula I is administered to a patient once or more per day for up to 28 consecutive days with the concurrent or intermittent administration of the taxane and platinum complex over the same total time period.

In particular embodiments of this invention, the substituted diaryl urea compound of Formula I (e.g., Sorafenib) can be administered to a patient as an oral, intravenous, intramuscular, subcutaneous, or parenteral dosage which can range from about 0.1 to about 300 mg/kg of total body weight.

In particular embodiments of this invention, the taxane (e.g., paclitaxel) is administered to a patient as an intravenous, intramuscular, subcutaneous, or parenteral dosage which can range from about 10-300 $mg/m^2$ of patient surface area.

In particular embodiments of this invention, the platinum complex (e.g., carboplatin) is administered to a patient at an intravenous, intramuscular, subcutaneous, or parenteral dosage which can range from about 100-500 $mg/m^2$ of patient surface area.

In particular embodiments of this invention, the substituted diaryl urea compound of Formula I is Sorafenib, a tosylate salt of "Bay 43-9006," N- (4-chloro-3- (trifluoromethyl) phenyl)-N'-(4-(2-(N-methylearbamoyl)-4-pyridyloxy) phenyl) urea. The scaleable synthesis of the aryl urea compound is disclosed in Organic Process Research and Development (2002), Vol.6, Issue #6, 777-781, and copending patent application serial no. 09/948,915 filed September 10, 2001 which are incorporated herein by reference.

This invention also relates to compositions containing a substituted diaryl urea compound of formula I, paclitaxel and carboplatin in amounts consistent with the objectives of this invention. This invention further relates to kits comprising separate doses of the three chemotherapeutic agents in separate containers. The combinations of the invention can also be formed in vivo, e.g., in a patient's body.

Paclitaxel is sold under the tradename Taxol® by the Bristol-Myers Squibb Company. Paclitaxel (5∃,20-Epoxy-1,2∀,4,7∃,10∃,13∀-hexahydroxytax-11-en-9-one 4,10-diacetate 2-benzoate 13-ester with (2R,3S)- N-benzoyl-3-phenylisoserine) has the empirical formula $C_{47}H_{51}NO_{14}$ and a molecular weight of 853.9. It is highly lipophilic in water. Paclitaxel is an antimicrotubule agent that promotes the assembly of microtubules from tubulin dimers and stabilizes microtubules by preventing depolymerization. While not bound by a theory, it is believed that this stability results in the

inhibition in the normal dynamic reorganization of the microtubule network that is essential for vital interphase and mitotic cellular functions. Also, paclitaxel is believed to induce abnormal arrays or bundles of microtubules throughout the cell cycle and multiple asters of microtubules during mitosis. Paclitaxel is administered by intravenous injection or by other appropriate infusion techniques in the conventional formulations and regimens in which they are known for use alone.

The substituted diaryl urea compounds of formula (I), salts, polymorphs, solvates, hydrates metabolites and prodrugs thereof, including diastereoisomeric forms (both isolated stereoisomers and mixtures of stereoisomers) are collectively referred to herein as the "Compounds of Formula I". Formula (I) is as follows:

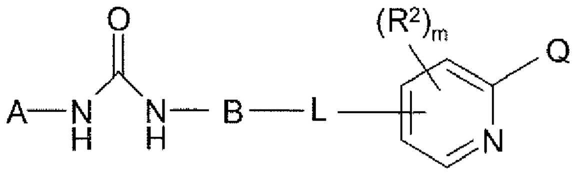

(Formula (I))

wherein

Q is -C(O)R)$_x$
$R_x$ is hydroxy, $C_{1-4}$ alkyl, $C_{1-4}$ alkoxy or $NR_aR_b$,
$R_a$ and $R_b$ are independently:

a) hydrogen;
b) $C_{1-4}$ alkyl, optionally substituted by

- hydroxy,
- $C_{1-4}$ alkoxy,
- a heteroaryl group selected from pyrrole, furan, thiophene, imidazole, pyrazole, thiazole, oxazole, isoxazole, isothiazole, triazole, tetrazole, thiadiazole, oxadiazole, pyridine, pyrimidine, pyridazine, pyrazine, triazine, benzoxazole, isoquioline, quinolines and imidazopyrimidine
- a heterocyclic group selected from tetrahydropyran, tetrahydrofuran, 1,3-dioxolane, 1,4-dioxane, morpholine, thiomorpholine, piperazine, piperidine, piperidinone, tetrahydropyrimidone, pentamethylene sulfide, tetramethylene sulfide, dihydropyrane, dihydrofuran, and dihydrothiophene,
- amino,-$NH_2$, optionally substituted by one or two $C_{1-4}$ alkyl groups, or
- phenyl,

c) phenyl optionally substituted with

- halogen, or
- amino,-$NH_2$, optionally substituted by one or two $C_{1-4}$ alkyl, or

d) - a heteroaryl group selected from pyrrole, furan, thiophene, imidazole, pyrazole, thiazole, oxazole, isoxazole, isothiazole, triazole, tetrazole, thiadiazole, oxadiazole, pyridine, pyrimidine, pyridazine, pyrazine, triazine, benzoxazole, isoquioline, quinoline and imidazopyrimidine;

A is optionally substituted phenyl, pyridinyl, naphthyl, benzoxazole, isoquioline, quinoline or imidazopyrimidine;
B is optionally substituted phenyl or naphthyl:
L is a bridging group which is -S- or -O-;
M is 0,1,2 or 3, and

each $R^2$ is independently $C_{1-5}$ alkyl, $C_{1-5}$ haloalkyl, $C_{1-3}$ alkoxy, N-oxo or N-hydroxy.

Structures of optionally substituted phenyl moieties for A of formula (I) which are of particular interest include structures of formula 1xx:

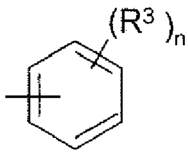

1xx

Structures of optionally substituted pyridinyl moieties for A of formula (I) which are of particular interest include structures of formula 1x:

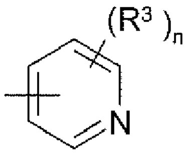

1x

Structures of optionally substituted naphthyl moieties for A of formula (I) which are of particular interest include structures of formula 1y:

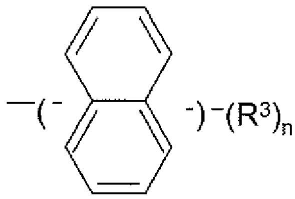

1y

The structure 1y represents that the substituents $R^3$ can appear on any carbon atom in either ring which has a valence that is otherwise complete with a hydrogen atom as a substituent. The bond to the urea group can also be through any carbon atom on either ring which has a valence that is otherwise complete with a hydrogen atom as a substituent.

B is optionally substituted phenyl or naphthyl. Structures of optionally substituted phenyl or naphthyl moieties for B of formula (I) which are of particular interest include structures 2a and 2b:

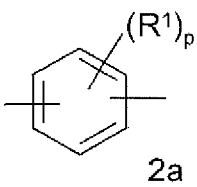

and

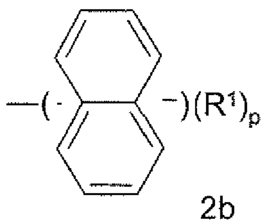

2a 2b .

The structures 2a and 2b represent that the substituents $R^1$ can appear on any carbon atom in the structure which has a valence that is otherwise complete with a hydrogen atom as a substituent and the bond to the urea group can be through any carbon atom in the structure which has a valence that is otherwise complete with a hydrogen atom as a substituent.

In a class of embodiments of this invention, B is substituted by at least one halogen substituent. In another class of embodiments, $R_x$ is $NR_aR_b$, and $R_a$ and $R_b$ are independently hydrogen or $C_{1-4}$ alkyl optionally substituted by hydroxy and L is a bridging group which is -S- or -O-.

The variable p is 0, 1, 2, 3, or 4, typically 0 or 1. The variable n is 0, 1, 2, 3, 4, 5 or 6, typically 0,1,2,3 or 4. The variable m is 0,1,2 or 3, typically 0.

Each $R^1$ is independently: halogen, $C_{1-5}$ haloalkyl, $NO_2$ $C(O)NR^4R^5$, $C_{1-6}$ alkyl, $C_{1-6}$ dialkylamine, $C_{1-3}$ alkylamine, CN, amino, hydroxy or $C_{1-3}$ alkoxy. Where present, $R^1$ is more commonly halogen and of the halogens, typically chlorine or fluorine, and more commonly fluorine.

Each $R^2$ is independently: $C_{1-5}$ alkyl, $C_{1-5}$ haloalkyl, $C_{1-3}$ alkoxy, N-oxo or N-hydroxy. Where present, $R^2$ is typically methyl or trifluoromethyl.

Each $R^3$ is independently selected from halogen, $R^4$, $OR^4$, $S(O)R^4$ ,$C(O)R^4$, $C(O)NR^4R^5$, oxo, cyano or nitro ($NO_2$).

$R^4$ and $R^5$ are independently selected from hydrogen, $C_{1-6}$ alkyl, and up to per-halogenated $C_{1-6}$ alkyl.

Other examples of A include: 3-tert butyl phenyl, 5-tert butyl-2-methoxyphenyl, 5-(trifluoromethyl)-2 phenyl, 3-(trifluoromethyl) -4 chlorophenyl, 3-(trifluoromethyl)-4-bromophenyl and 5-(trifluoromethyl)-4-chloro-2 methoxyphenyl.

Other examples of B include:

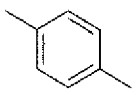 , 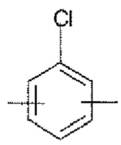 , 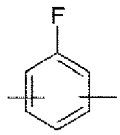 ,

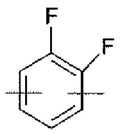 , 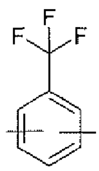 , 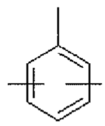 , 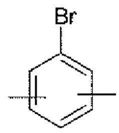 ,

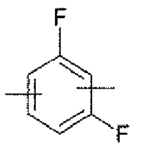 , 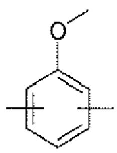 , 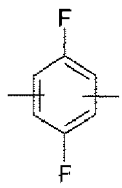 and 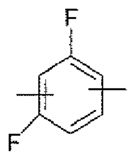 ,

Preferably the urea group -NH-C(O)-NH- and the bridging group, L, are not bound to contiguous ring carbons of B, but rather have 1 or 2 ring carbons separating them.

Examples of $R^1$ groups include fluorine, chorine, bromine, methyl, $NO_2$, $C(O)NH_2$, methoxy, $SCH_3$, trifluoromethyl, and methanesulfonyl.

Examples of $R^2$ groups include methyl, ethyl, propyl, oxygen, and cyano.

Examples of $R^3$ groups include trifluoromethyl, methyl, ethyl, propyl, butyl, isopropyl, tert-butyl, chlorine, fluorine, bromine, cyano, methoxy, acetyl, trifluoromethanesulfonyl, trifluoromethoxy, and trifluoromethylthio.

A class of compounds of interest are of formula II below

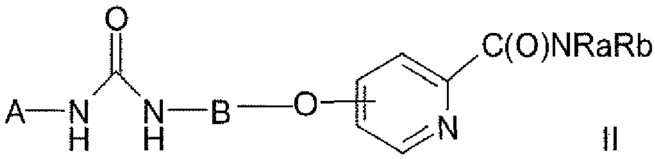
II

wherein Ra and Rb are independently hydrogen and $C_1$-$C_4$ alkyl,
B of formula II is

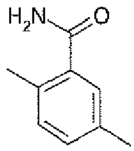 , 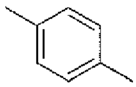 , 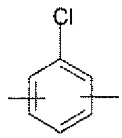 , 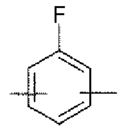 ,

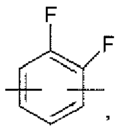 , 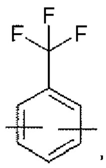 , 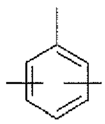 , 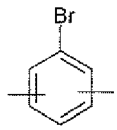 ,

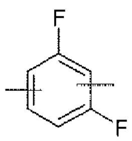 , 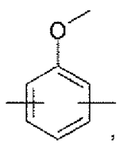 , 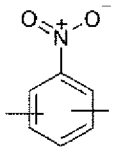 , 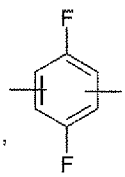 or 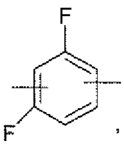 ,

wherein the urea group, -NH-C(O)-NH-, and the oxygen bridging group are not bound to contiguous ring carbons of B, but rather have 1 or 2 ring carbons separating them,
and A of formula (II) is

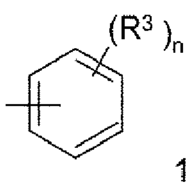
1xx

or

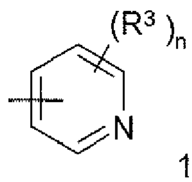
1x

wherein the variable n is 0, 1, 2, 3 or 4.

$R^3$ is trifluoromethyl, methyl, ethyl, propyl, butyl, isopropyl, tert-butyl, chlorine, fluorine, bromine, cyano, methoxy, acetyl, trifluoromethanesulfonyl, trifluoromethoxy, or trifluoromethylthio.

In a subclass of such compounds, each $R^3$ substituent on A of formula II is selected from chlorine, trifluoromethyl, tert-butyl or methoxy.

In another subclass of such compounds, A of formula II is

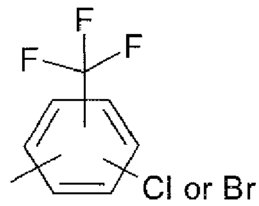

and B of formula II is phenylene, fluoro substituted phenylene or difluoro substituted phenylene.

Another class of compounds of interest includes compounds having the structure of formulae X below wherein phenyl ring "B" optionally has one halogen substituent, preferably Cl or F, more preferably F.

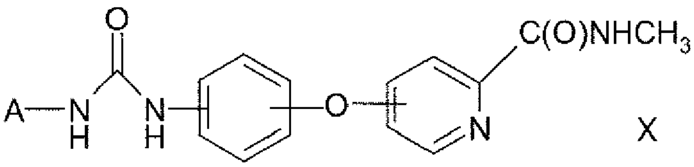

X

For the compounds of formula X, $R^2$, A is as defined above for formula I. The moiety $C(O)NHCH_3$ is the only substituent on the pyridinyl moiety. Preferred values for A are substituted phenyl which have at least one substituent, $R^3$, $R^3$ is preferably halogen, preferably Cl or F, trifluoromethyl and/or methoxy.

A subclass of compounds of interest includes compounds having the structure of formulas Z1, Z2, and Z3 below :

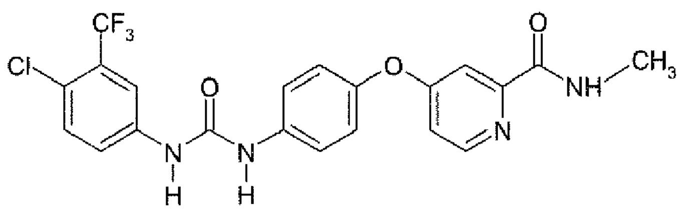

Z1

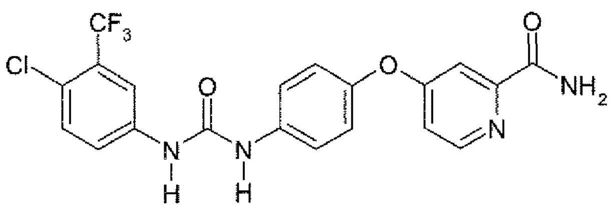

Z3

The compound Z1 is described in WO 00/42012. Both WO 00/42012 and Bankston et al. (Organic Process Research & Development, 2002, 6, 777-781) describe a process for preparing compound Z1, which is illustrated in the following scheme:

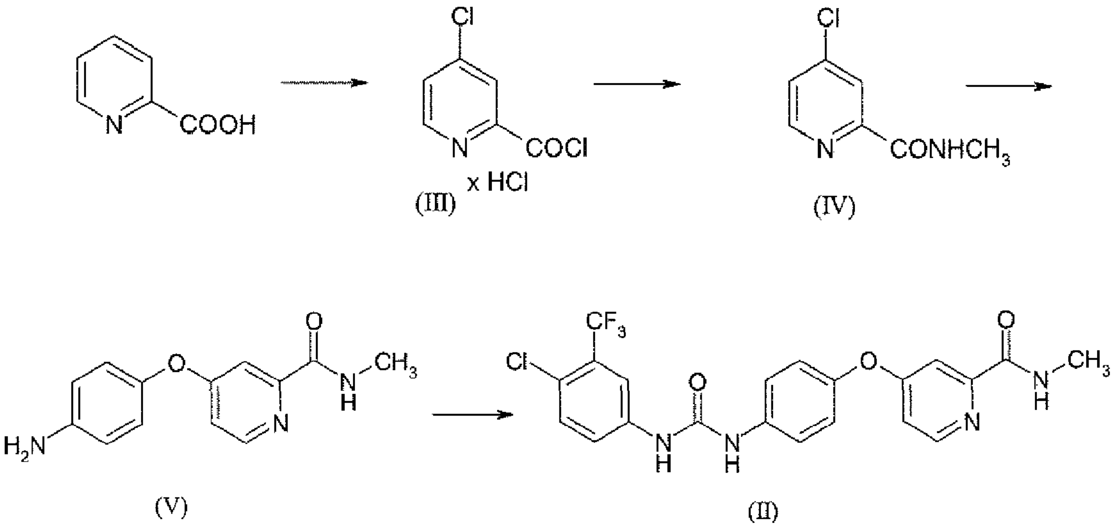

(III) (IV) (V) (II)

Preferably used in the pharmaceutical composition according to the invention is the p-toluenesulfonic acid salt of 4{4-[3-(4-chloro-3-trifluoromethylphenyl)-ureido]-phenoxy}-pyridine-2-carboxylic acid methyl amide (tosylate salt of compound (I)). More preferably the p-toluenesulfonic acid salt of 4{4-[3-(4-chloro-3-trifluoromethylphenyl)-ureido]-phenoxy}-pyridine-2-carboxylic acid methyl amide exists for at least 80% in the stable polymorph I. Most preferably the p-toluenesulfonic acid salt of 4 {4-[3-(4-chloro-3-trifluoromethylphenyl)-ureido]-phenoxy}-pyridine-2-carboxylic acid methyl amide exists for at least 80% in the stable polymorph I and in a micronized form.

Micronization can be achieved by standard milling methods, preferably by air chat milling, known to a skilled person. The micronized form can have a mean particle size of from 0.5 to 10 μm, preferably from 1 to 6 μm, more preferably from 1 to 3 μm. The indicated particle size is the mean of the particle size distribution measured by laser diffraction known to a skilled person (measuring device: HELOS, Sympatec).

The process for preparing the p-toluenesulfonic acid salt of 4{4-[3-(4-chloro-3-trifluoromethylphenyl)-ureido]-phenoxy}-pyridine-2-carboxylic acid methyl amide and its stable polymorph I are described in the patent applications EP 04023131.8 and EP 04023130.0.

When any moiety is "substituted", it can have up to the highest number of indicated substituents and each substituent can be located at any available position on the moiety and can be attached through any available atom on the substituent. "Any available position" means any position on the moiety that is chemically accessible through means known in the art or taught herein and that does not create an unstable molecule, e.g., incapable of administration to a human. When there are two or more substituents on any moiety, each substituent is defined independently of any other substituent and can, accordingly, be the same or different.

The term "optionally substituted" means that the moiety so modified may be either unsubstituted, or substituted with the identified substituent(s).

It is understood that the term "hydroxy" as a pyridine substituent includes 2-, 3-, and 4-hydroxypyridine, and also includes those structures referred to in the art as 1-oxo-pyridine, 1-hydroxy-pyridine or pyridine N-oxide.

Where the plural form of the word compounds, salts, and the like, is used herein, this is taken to mean also a single compound, salt, or the like.

The term $C_{1-6}$ alkyl, unless indicated otherwise, means straight, branched chain or cyclic alkyl groups having from one to six carbon atoms, which may be cyclic, linear or branched with single or multiple branching. Such groups include for example methyl, ethyl, n-propyl, isopropyl, n-butyl, isobutyl, sec-butyl, tert-butyl, cyclopropyl, cyclobutyl and the like.

The term $C_{1-6}$ haloalkyl, unless indicated otherwise, means a saturated hydrocarbon radical having up to six carbon atoms, which is substituted with a least one halogen atom, up to perhalo. The radical may be cyclic, linear or branched with single or multiple branching. The halo substituent(s) include fluoro, chloro, bromo, or iodo. Fluoro, chloro and bromo are preferred, and fluoro and chloro are more preferred. The halogen substituent(s) can be located on any available carbon. When more than one halogen substituent is present on this moiety, they may be the same or different. Examples of such halogenated alkyl substituents include but are not limited to chloromethyl, dichloromethyl, trichloromethyl, fluoromethyl, difluoromethyl, trifluoromethyl, 2,2,2-trifluoroethyl, and 1,1,2,2-tetrafluoroethyl, and the like.

The term $C_{1-6}$ alkoxy, unless indicated otherwise, means a cyclic, straight or branched chain alkoxy group having from one to six saturated carbon atoms which may be cyclic, linear or branched with single or multiple branching,

and includes such groups as methoxy, ethoxy, n-propoxy, isopropoxy, butoxy, pentoxy and the like. It also includes halogenated groups such as 2, 2-dichloroethoxy, trifluoromethoxy, and the like.

Halo or halogen means fluoro, chloro, bromo, or iodo. Fluoro, chloro and bromo are preferred, and fluoro and chloro are more preferred.

$C_{1-3}$alkylamine, unless indicated otherwise, means methylamino, ethylamino, propylamino or isopropylamino.

Examples of $C_{1-6}$ dialkylamine include but are not limited to diethylamino, ethyl-isopropylamino, methyl-isobutylamino and dihexylamino.

The term heteroaryl refers to both monocyclic and bicyclic heteroaryl rings. Monocyclic heteroaryl means an aromatic monocyclic ring having 5 to 6 ring atoms and 1-4 hetero atoms selected from N, O and S, the remaining atoms being carbon. When more than one hetero atom is present in the moiety, they are selected independently from the other (s) so that they may be the same or different. Monocyclic heteroaryl rings include, but are not limited to pyrrole, furan, thiophene, imidazole, pyrazole, thiazole, oxazole, isoxazole, isothiazole, triazole, tetrazole, thiadiazole, oxadiazole, pyridine, pyrimidine, pyridazine, pyrazine, and triazine.

Bicyclic heteroaryl means fused bicyclic moieties where one of the rings is chosen from the monocyclic heteroaryl rings described above and the second ring is either benzene or another monocyclic heteroaryl ring described above. When both rings in the bicyclic moiety are heteroaryl rings, they may be the same or different, as long as they are chemically accessible by means known in the art. Bicyclic heteroaryl rings include synthetically accessible 5-5, 5-6, or 6-6 fused bicyclic aromatic structures including, for example but not by way of limitation, benzoxazole (fused phenyl and oxazole), quinoline (fused phenyl and pyridine), imidazopyrimidine (fused imidazole and pyrimidine), and the like.

Where indicated, the bicyclic heteroaryl moieties may be partially saturated. When partially saturated either the monocyclic heteroaryl ring as described above is fully or partially saturated, the second ring as described above is either fully or partially saturated or both rings are partially saturated. The term "5 or 6 membered heterocyclic ring, containing at least one atom selected from oxygen, nitrogen and sulfur, which is saturated, partially saturated, or aromatic" includes, by no way of limitation, tetrahydropyran, tetrahydrofuran, 1,3-dioxolane, 1,4-dioxane, morpholine, thiomorpholine, piperazine, piperidine, piperidinone, tetrahydropyrimidone, pentamethylene sulfide, tetramethylene sulfide, dihydropyrane, dihydrofuran, dihydrothiophene, pyrrole, furan, thiophene, imidazole, pyrazole, thiazole, oxazole, isoxazole, isothiazole, triazole, pyridine, pyrimidine, pyridazine, pyrazine, triazine, and the like.

The term "$C_{1-3}$ alkyl-phenyl" includes, for example, 2-methylphenyl, isopropylphenyl, 3-phenylpropyl, or 2-phenyl-1-methylethyl. Substituted examples include 2-[2-chlorophenyl] ethyl, 3,4-dimethylphenylmethyl, and the like.

Unless otherwise stated or indicated, the term "aryl" includes 6-12 membered mono or bicyclic aromatic hydrocarbon groups (e.g., phenyl, naphthalene, azulene, indene group ) having 0, 1, 2, 3, 4, 5 or 6 substituents.

The compounds of Formula (I) may contain one or more asymmetric centers, depending upon the location and nature of the various substituents desired. Asymmetric carbon atoms may be present in the (R) or (S) configuration or (R,S) configuration. In certain instances, asymmetry may also be present due to restricted rotation about a given bond, for example, the central bond adjoining two substituted aromatic rings of the specified compounds. Substituents on a ring may also be present in either cis or trans form. It is intended that all such configurations (including enantiomers and diastereomers), are included within the scope of the present invention. Preferred compounds are those with the absolute configuration of the compound of Formula (I) which produces the more desirable biological activity. Separated, pure or partially purified isomers or racemic mixtures of the compounds of this invention are also included within the scope of the present invention. The purification of said isomers and the separation of said isomeric mixtures can be accomplished by standard techniques known in the art.

The optical isomers can be obtained by resolution of the racemic mixtures according to conventional processes, for example, by the formation of diastereoisomeric salts using an optically active acid or base or formation of covalent diastereomers. Examples of appropriate acids are tartaric, diacetyltartaric, ditoluoyltartaric and camphorsulfonic acid. Mixtures of diastereoisomers can be separated into their individual diastereomers on the basis of their physical and/or chemical differences by methods known in the art, for example, by chromatography or fractional crystallization. The optically active bases or acids are then liberated from the separated diastereomeric salts. A different process for separation of optical isomers involves the use of chiral chromatography (e.g., chiral HPLC columns), with or without conventional derivation, optimally chosen to maximize the separation of the enantiomers. Suitable chiral HPLC columns are manufactured by Diacel, e.g., Chiracel OD and Chiracel OJ among many others, all routinely selectable. Enzymatic separations, with or without derivitization, are also useful. The optically active compounds of Formula I can likewise be obtained by chiral syntheses utilizing optically active starting materials.

The present invention also relates to useful forms of the compounds as disclosed herein, such as pharmaceutically acceptable salts, metabolites and prodrugs of all the compounds Formula (I). The term "pharmaceutically acceptable salt" refers to a relatively non-toxic, inorganic or organic acid addition salt of a compound of the present invention. For example, see S. M. Berge, et al. "Pharmaceutical Salts," J. Pharm. Sci. 1977, 66, 1-19. Pharmaceutically acceptable salts include those obtained by reacting the main compound, functioning as a base, with an inorganic or organic acid to form a salt, for example, salts of hydrochloric acid, sulfuric acid, phosphoric acid, methane sulfonic acid, camphor sulfonic

acid, oxalic acid, maleic acid, succinic acid and citric acid. Pharmaceutically acceptable salts also include those in which the main compound functions as an acid and is reacted with an appropriate base to form, e.g., sodium, potassium, calcium, mangnesium, ammonium, and choline salts. Those skilled in the art will further recognize that acid addition salts of the claimed compounds may be prepared by reaction of the compounds with the appropriate inorganic or organic acid via any of a number of known methods. Alternatively, alkali and alkaline earth metal salts are prepared by reacting the compounds of the invention with the appropriate base via a variety of known methods.

Representative salts of the compounds of this invention include the conventional non-toxic salts and the quaternary ammonium salts which are formed, for example, from inorganic or organic acids or bases by means well known in the art. For example, such acid addition salts include acetate, adipate, alginate, ascorbate, aspartate, benzoate, benzenesulfonate, bisulfate, butyrate, citrate, camphorate, camphorsulfonate, cinnamate, cyclopentanepropionate, digluconate, dodecylsulfate, ethanesulfonate, fumarate, glucoheptanoate, glycerophosphate, hemisulfate, heptanoate, hexanoate, hydrochloride, hydrobromide, hydroiodide, 2-hydroxyethanesulfonate, itaconate, lactate, maleate, mandelate, methanesulfonate, 2-naphthalenesulfonate, nicotinate, nitrate, oxalate, pamoate, pectinate, persulfate, 3-phenylpropionate, picrate, pivalate, propionate, succinate, sulfonate, tartrate, thiocyanate, tosylate, trifluoromethanesulfonate, and undecanoate.

Base salts include alkali metal salts such as potassium and sodium salts, alkaline earth metal salts such as calcium and magnesium salts, and ammonium salts with organic bases such as dicyclohexylamine and N-methyl-D-glucamine. Additionally, basic nitrogen containing groups may be quaternized with such agents as lower alkyl halides such as methyl, ethyl, propyl, and butyl chlorides, bromides and iodides; dialkyl sulfates like dimethyl, diethyl, and dibutyl sulfate; and diamyl sulfates, long chain halides such as decyl, lauryl, myristyl and strearyl chlorides, bromides and iodides, aryl or aralkyl halides like benzyl and phenethyl bromides and others monosubstituted aralkyl halides or polysubstituted aralkyl halides. Solvates for the purposes of the invention are those forms of the compounds where solvent molecules form a complex in the solid state and include, but are not limited to for example ethanol and ethanol. Hydrates are a specific form of solvates, where the solvent molecule is water.

Certain pharmacologically active agents can be further modified with labile functional groups that are cleaved after in vivo administration to furnish the parent active agent and the pharmacologically inactive derivatizing group. These derivatives, commonly referred to as prodrugs, can be used, for example, to alter the physicochemical properties of the active agent, to target the active agent to a specific tissue, to alter the pharmacokinetic and pharmacodynamic properties of the active agent, and to reduce undesirable side effects. Prodrugs of the invention include, e.g., the esters of appropriate compounds of this invention that are well-tolerated, pharmaceutically acceptable esters such as alkyl esters including methyl, ethyl, propyl, isopropyl, butyl, isobutyl or pentyl esters. Additional esters such as phenyl-$C_1$-$C_5$ alkyl may be used, although methyl ester is preferred.

Methods which can be used to synthesize other prodrugs are described in the following reviews on the subject, which are incorporated herein by reference for their description of these synthesis methods:

- Higuchi, T.; Stella, V. eds. Prodrugs As Novel Drug Delivery Systems. ACS Symposium Series. American Chemical Society: Washington, DC (1975).
- Roche, E. B. Design of Biopharmaceutical Properties through Prodrugs and Analogs. American Pharmaceutical Association: Washington, DC (1977).
- Sinkula, A. A.; Yalkowsky, S. H. J Pharm Sci. 1975, 64, 181-210.
- Stella, V. J.; Charman, W. N. Naringrekar, V. H. Drugs 1985,29,455-473.
- Bundgaard, H., ed. Design of Prodrugs. Elsevier: New York (1985).
- Stella, V. J.; Himmelstein, K. J. J. Med. Chem. 1980, 23, 1275-1282.
- Han, H-K; Amidon, G. L. AAPS Pharmsci 2000, 2, 1- 11.
- Denny, W. A. Eur. J. Med. Chem. 2001, 36, 577-595.
- Wermuth, C. G. in Wermuth, C. G. ed. The Practice of Medicinal Chemistry Academic Press: San Diego (1996), 697-715.
- Balant, L. P.; Doelker, E. in Wolff, M. E. ed. Burgers Medicinal Chemistry And Drug Discovery John Wiley & Sons: New York (1997), 949-982.

The metabolites of the compounds of this invention include oxidized derivatives of the compounds of Formula I, II, X, Y, Za, Zb, Zc and Zd, wherein one or more of the nitrogens are substituted with a hydroxy group; which includes derivatives where the nitrogen atom of the pyridine group is in the oxide form, referred to in the art as 1-oxo-pyridine or has a hydroxy substituent, referred to in the art as 1-hydroxy-pyridine.

General Preparative Methods

The particular process to be utilized in the preparation of the compounds used in this embodiment of the

invention depends upon the specific compound desired. Such factors as the selection of the specific substituents play a role in the path to be followed in the preparation of the specific compounds of this invention. Those factors are readily recognized by one of ordinary skill in the art.

The compounds of the invention may be prepared by use of known chemical reactions and procedures as described in the following published international applications WO 00/42012, WO03/047579, WO 2005/009961, WO 2004/078747 and WO05/000284 and European patent applications EP 04023131.8 and EP 04023130.0.

The compounds of the invention can be made according to conventional chemical methods, and/or as disclosed below, from starting materials which are either commercially available or producible according to routine, conventional chemical methods. General methods for the preparation of the compounds are given below, and the preparation of representative compounds is specifically illustrated in examples.

The preparation of ureas of formula (I) can be prepared from the condensation of the two arylamine fragments and in the presence of phosgene, di-phosgene, tri-phosgene, carbonyldiimidazole, or equivalents in a solvent that does not react with any of the starting materials, as described in one or more of these published. Alternatively, compounds of formula (I) can be synthesized by reacting amino compounds) with isocyanate compounds as described in one or more of the published international applications described above.

The isocyanates are commercially available or can be synthesized from heterocyclic amines according to methods commonly known to those skilled in the art [e.g. from treatment of an amine with phosgene or a phosgene equivalent such as trichloromethyl chloroformate (diphosgene), bis(trichloromethyl)carbonate (triphosgene), or N,N'-carbonyldiimidazole (CDI); or, alternatively by a Curtius-type rearrangement of an amide, or a carboxylic acid derivative, such as an ester, an acid halide or an anhydride].

Aryl amines of formulas are commercially available, or can be synthesized according to methods commonly known to those skilled in the art. Aryl amines are commonly synthesized by reduction of nitroaryls using a metal catalyst, such as Ni, Pd, or Pt, and $H_2$ or a hydride transfer agent, such as formate, cyclohexadiene, or a borohydride (Rylander. Hydrogenation Methods; Academic Press: London, UK (1985)). Nitroaryls may also be directly reduced using a strong hydride source, such as $LiAlH_4$ (Seyden-Penne. Reductions by the Alumino- and borohydrides in Organic Synthesis; VCH Publishers: New York (1991)), or using a zero valent metal, such as Fe, Sn or Ca, often in acidic media. Many methods exist for the synthesis of nitroaryls (March. Advanced Organic Chemistry, 3rd Ed.; John Wiley: New York (1985). Larock. Comprehensive Organic Transformations; VCH Publishers: New York (1989)). Nitro aryls are commonly formed by electrophilic aromatic nitration using $HNO_3$, or an alternative $NO_2^+$ source.

Pyridine-1-oxides of Formula (I) where the pyridine ring carries a hydroxy substituent on its nitrogen atom, and A, B, L are broadly defined as above can be prepared from the corresponding pyridines using oxidation conditions know in the art. Some examples are as follows:

- peracids such as meta chloroperbenzoic acids in chlorinated solvents such as dichloromethane, dichloroethane, or chloroform (Markgraf et al., Tetrahedron 1991, 47, 183);
- $(Me_3SiO)_2$ in the presence of a catalytic amount of perrhenic acid in chlorinated solvents such as dichloromethane (Coperet et al., Terahedron Lett. 1998, 39, 761);
- Perfluoro-cis-2-butyl-3-propyloxaziridine in several combinations of halogenated solvents (Amone et al., Tetrahedron 1998, 54, 7831);
- Hypofluoric acid - acetonitrile complex in chloroform (Dayan et al., Synthesis 1999, 1427);
- Oxone, in the presence of a base such as KOH, in water (Robker et al., J. Chem. Res., Synop. 1993, 10, 412);
- Magnesium monoperoxyphthalate, in the presence of glacial acetic acid (Klemm et al., J. Heterocylic Chem. 1990, 6,1537);
- Hydrogen peroxide, in the presence of water and acetic acid (Lin A.J., Org. Prep. Proced, Int. 1991, 23(1), 114);
- Dimethyldioxirane in acetone (Boyd et al., J. Chem. Soc., Perkin Trans. 1991, 9, 2189).

In addition, specific methods for preparing diaryl ureas and intermediate compounds are already described elsewhere in the patent literature, and can be adapted to the compounds of the present invention. For example, Miller S. et al, "Inhibition of p38 Kinase using Symmetrical and Unsymmetrical Diphenyl Ureas" PCT Int. Appl. WO 99 32463, Miller, S et al. "Inhibition of raf Kinase using Symmetrical and Unsymmetrical Substituted Diphenyl Ureas" PCT Int. Appl., WO 99 32436, Dumas, J. et al., "Inhibition of p38 Kinase Activity using Substituted Heterocyclic Ureas" PCT Int. Appl., WO 99 32111, Dumas, J. et al., "Method for the Treatment of Neoplasm by Inhibition of raf Kinase using N-Heteroaryl-N'-(hetero)arylureas" PCT Int. Appl., WO 99 32106, Dumas, J. et al., "Inhibition of p38 Kinase Activity using Aryl- and Heteroaryl- Substituted Heterocyclic Ureas" PCT Int. Appl., WO 99 32110, Dumas, J., et al., "Inhibition of raf Kinase using Aryl- and Heteroaryl- Substituted Heterocyclic Ureas" PCT Int. Appl., WO 99 32455, Riedl, B., et al., "O-Carboxy Aryl Substituted Diphenyl Ureas as raf Kinase Inhibitors" PCT Int. Appl., WO 00 42012, Riedl, B., et al., "O-Carboxy Aryl Substituted Diphenyl Ureas as p38 Kinase Inhibitors" PCT Int. Appl., WO 00 41698, Dumas, J. et al. "Heteroaryl ureas containing nitrogen hetero-atoms as p38 kinase inhibitors" U.S. Pat. Appl. Publ., US 20020065296, Dumas, J. et al.

"Preparation of N-aryl-N'-[(acylphenoxy) phenyl]ureas as raf kinase inhibitors" PCT Int. Appl., WO 02 62763, Dumas, J. et al. "Inhibition of raf kinase using quinolyl, isoquinolyl or pyridyl ureas" PCT Int. Appl., WO 02 85857, Dumas, J. et al. "Preparation of quinolyl, isoquinolyl or pyridyl-ureas as inhibitors of raf kinase for the treatment of tumors and/or cancerous cell growth" U.S. Pat. Appl. Publ., US 20020165394. All the preceding patent applications are hereby incorporated by reference.

Synthetic transformations that may be employed in the synthesis of compounds of Formula (I) and in the synthesis of intermediates involved in the synthesis of compounds of Formula (I) are known by or accessible to one skilled in the art. Collections of synthetic transformations may be found in compilations, such as:

- J. March. Advanced Organic Chemistry, 4th ed.; John Wiley: New York (1992);
- R.C. Larock. Comprehensive Organic Transformations, 2nd ed.; Wiley-VCH: New York (1999);
- F.A. Carey; R.J. Sundberg. Advanced Organic Chemistry, 2nd ed.; Plenum Press: New York (1984);
- T.W. Greene; P.G.M. Wuts. Protective Groups in Organic Synthesis, 3rd ed.; John Wiley: New York (1999);
- L.S. Hegedus. Transition Metals in the Synthesis of Complex Organic Molecules, 2nd ed.; University Science Books: Mill Valley, CA (1994);
- L.A. Paquette, Ed. The Encyclopedia of Reagents for Organic Synthesis; John Wiley: New York (1994);
- A.R. Katritzky; O. Meth-Cohn; C.W. Rees, Eds. Comprehensive Organic Functional Group Transformations; Pergamon Press: Oxford, UK (1995);
- G. Wilkinson; F.G A. Stone; E.W. Abel, Eds. Comprehensive Organometallic Chemistry; Pergamon Press: Oxford, UK (1982);
- B.M. Trost; I. Fleming. Comprehensive Organic Synthesis; Pergamon Press: Oxford, UK (1991);
- A.R. Katritzky; C.W. Rees Eds. Comprehensive Heterocylic Chemistry; Pergamon Press: Oxford, UK (1984);
- A.R. Katritzky; C.W. Rees; E.F.V. Scriven, Eds. Comprehensive Heterocylic Chemistry II; Pergamon Press: Oxford, UK (1996); and
- C. Hansch; P.G. Sammes; J.B. Taylor, Eds. Comprehensive Medicinal Chemistry: Pergamon Press: Oxford, UK (1990).

In addition, recurring reviews of synthetic methodology and related topics include Organic Reactions; John Wiley: New York; Organic Syntheses; John Wiley: New York; Reagents for Organic Synthesis: John Wiley: New York; The Total Synthesis of Natural Products; John Wiley: New York; The Organic Chemistry of Drug Synthesis; John Wiley: New York; Annual Reports in Organic Synthesis; Academic Press: San Diego CA; and Methoden der Organischen Chemie (Houben-Weyl); Thieme: Stuttgart, Germany. Furthermore, databases of synthetic transformations include Chemical Abstracts, which may be searched using either CAS OnLine or SciFinder, Handbuch der Organischen Chemie (Beilstein), which may be searched using SpotFire, and REACCS.

The compounds of Formula I have been previously characterized as having various activities, including for inhibiting the Raf/MEK/ERK pathway, c-raf, b-raf, p38, VEGFR, VEGFR2, VEGR3, FLT3, PDGFR, PDGFR-beta, and c-kit. These activities and their use in treating various diseases and conditions are disclosed in, e.g., WO 00/42021, WO 00/41698, WO03/068228, WO 03/047579, WO 2005/00996, WO 2005/000284 and U.S. Application No. 20050038080, which are hereby incorporated by reference in their entirety. Pharmacuetical compositions intended for oral use may be prepared according to any suitable method known to the art for the manufacture of pharmaceutical compositions. The pharmaceutical composition comprises suitable administration forms which deliver the compound of the invention in a rapid manner, for example tablets (uncoated or coated tablets), tablets which disintegrate rapidly in the oral cavity or capsules optionally filled with granules (for example hard or soft gelatin capsules), sugar-coated tablets, powders, sachets, granules, pellets, dragées, chewable tablets, dispersible tables, troches and lozenges. Such compositions may contain one or more agents selected from the group consisting of diluents, sweetening agents, flavoring agents, coloring agents and preserving agents in order to provide palatable preparations. Tablets contain the active ingredient in admixture with non-toxic pharmaceutically acceptable excipients which are suitable for the manufacture of tablets. These excipients may be, for example, inert diluents, such as calcium carbonate, sodium carbonate, lactose, calcium phosphate or sodium phosphate; granulating and disintegrating agents, for example, corn starch, or alginic acid; and binding agents, for example magnesium stearate, stearic acid or talc. The tablets may be uncoated or they may be coated by known techniques to delay disintegration and adsorption in the gastrointestinal tract and thereby provide a sustained action over a longer period. For example, a time delay material such as glyceryl monostearate or glyceryl distearate may be employed. These compounds may also be prepared in solid, rapidly released form.

Pharmacuetical compositions for oral use may also be presented as hard gelatin capsules wherein the active ingredient is mixed with an inert solid diluent, for example, calcium carbonate, calcium phosphate or kaolin, or as soft gelatin capsules wherein the active ingredient is mixed with water or an oil medium, for example peanut oil, liquid paraffin or olive oil.

Aqueous suspensions containing at least one of the active materials in admixture with excipients suitable for

the manufacture of aqueous suspensions may also be used. Such excipients are suspending agents, for example sodium carboxymethylcellulose, methylcellulose, hydroxypropyl-methylcellulose, sodium alginate, polyvinylpyrrolidone, gum tragacanth and gum acacia; dispersing or wetting agents may be a naturally-occurring phosphatide, for example, lecithin, or condensation products of an alkylene oxide with fatty acids, for example polyoxyethylene stearate, or condensation products of ethylene oxide with long chain aliphatic alcohols, for example heptadecaethylene oxycetanol, or condensation products of ethylene oxide with partial esters derived from fatty acids and hexitol such as polyoxyethylene sorbitol monooleate, or condensation products of ethylene oxide with partial esters derived from fatty acids and hexitol anhydrides, for example polyethylene sorbitan monooleate. The aqueous suspensions may also contain one or more preservatives, for example ethyl, or n-propyl p-hydroxybenzoate, one or more coloring agents, one or more flavoring agents, and one or more sweetening agents, such as sucrose or saccharin.

A pharmaceutically acceptable carrier is any carrier which is relatively non-toxic and innocuous to a patient at concentrations consistent with effective activity of the active ingredient so that any side effects ascribable to the carrier do not vitiate the beneficial effects of the active ingredient.

A pharmaceutically effective amount of compound is that amount which produces a result or exerts an influence on the particular condition being treated. The compounds of the present invention can be administered with pharmaceutically-acceptable carriers well known in the art using any effective conventional dosage unit forms, including immediate, slow and timed release preparations.

A pharmaceutically acceptable excipient is any excipient which is relatively non-toxic and innocuous to a patient at concentrations consistent with effective activity of the active ingredient so that any side effects ascribable to the excipient do not vitiate the beneficial effects of the active ingredient.

Pharmaceutically acceptable excipients according to the invention are for example disintegrants, binders, lubricants, fillers, plasticizers, surfactants and wetting agents, film-forming agents and coating materials, and coloring agents for example pigments.

Disintegrants include, but are not limited to croscarmellose sodium, crospovidone, alginic acid, , carboxymethylcellulose calcium, carboxymethylcellulose sodium, microcrystalline cellulose, hydroxypropyl cellulose, low substituted hydroxypropyl cellulose, polacrillin potassium, cross-linked polyvinylpyrrolidone, sodium alginate, sodium starch glycollate, partially hydrolysed starch, sodium carboxymethyl starch and starch. Preference is given to croscarmellose sodium and/or cross-linked polyvinylpyrrolidone, more preference is given to croscarmellose sodium.

The amount of the disintegrant contained in the pharmaceutical composition of can be from 0 to 15%, preferably from 5 to 12% by the total weight of the composition.

Binders include, but are not limited to hydroxypropyl cellulose, hypromellose (hydroxypropyl methylcellulose, HPMC), microcrystalline cellulose, acacia, alginic acid, carboxymethylcellulose, ethylcellulose, methylcellulose, hydroxaethylcellulose, ethylhydroxyethylcellulose, polyvinyl alcohol, polyacrylates, carboxymethylcellulose calcium, carboxymethylcellulose sodium, compressible sugar, ethylcellulose, gelatin, liquid glucose, methylcellulose, polyvinyl pyrrolidone and pregelatinized starch. Preference is given to a hydrophilic binder which is soluble in the granulation liquid, more preference is given to hypromellose (hydroxypropyl methylcellulose, HPMC) and/or polyvinylpyrrolidone, most preference is given to hypromellose.

The amount of the binder contained in the pharmaceutical composition of can be from 0 to 15%, preferably from 0.5 to 8% by the total weight of the composition.

Lubricants include, but are not limited to calcium stearate, magnesium stearate, mineral oil, stearic acid, fumaric acid, sodium stearylfumarate, zinc stearate and polyethyleneglycol. Preference is given to magnesium stearate.

The amount of the lubricant contained in the pharmaceutical composition of can be from 0 to 2%, preferably from 0.2 to 0.8% by the total weight of the composition.

Fillers include, but are not limited to dibasic calcium phosphate, kaolin, lactose, mannitol, microcrystalline cellulose, silicated microcrystalline cellulose, dicalcium phosphate, tricalcium phosphate, magnesium trisilicate, mannitol, maltitol, sorbitol, xylitol, lactose for example the anhydrous form or the hydrate form such as the monohydrate form, dextrose, maltose, saccharose, glucose, fructose or maltodextrine, powdered cellulose, precipitated calcium carbonate, sodium carbonate, sodium phosphate and starch. Preference is given to microcrystalline cellulose, mannitol, lactose and/or dicalcium phosphate, more preference is given to microcrystalline cellulose.

The amount of the filler contained in the pharmaceutical composition of can be from 0 to 60%, preferably from 3 to 20 % by the total weight of the composition.

Surfactants and Wetting agents include, but are not limited to heptadecaethylene oxycetanol, lecithins, sorbitol monooleate, polyoxyethylene sorbitol monooleate, polyoxyethylene stearate, polyoxyethylen sorbitan monolaurate, benzalkonium chloride, nonoxynol 10, oxtoxynol 9, polysorbates for example 20, 40, 60 or 80, sorbitan mono-palmitate, sodium salts of fatty alcoholsulaftes such as sodium lauryl sulfate, sodium dodecylsulfate, sodium salts of sulfosuccinates such as sodium dioctylsulfosuccinate, partially esters of fatty acids with alcohols such as glycerine monostearate, partially esters of fatty acids with sorbitans such as sorbitan monolaurate, partially esters of fatty acids with polyhydroxyethylene sorbitans such as polyethyleneglycol sorbitan monolaurate, -monostearate or -monooleate, ethers of fatty alcohols with

polyhydroxyethylene, esters of fatty acids with polyhydroxyethylene, copolymers of ethylenoxide and propylenoxide (Pluronic®) and ethoxylated triglycerides. Preference is given to sodium lauryl sulfate.

The amount of the surfactant contained in the pharmaceutical composition of can be from 0 to 5 %, preferably from 0.1 to 2 % by the total weight of the composition.

Film-forming agents and coating materials include, but are not limited to liquid glucose, hydroxyethyl cellulose, hydroxypropyl cellulose, hydroxypropyl methylcellulose (hypromellose, HPMC), methyl cellulose, ethylcellulose, cellulose acetate phthalate, shellac, polyvinylpyrrolidone, copolymers of vinylpyrrolidone and vinylacetate such as Kollidon® VA64 BASF, copolymers of acrylic- and/or methacrylic acid esters with trimethylammoniummethylacrylate, copolymers of dimethylaminomethacrylic acid and neutral methacrylic acid esters, polymers of methacrylic acid or methacrylic acid esters, copolymers of acrylic acid ethylester and methacrylic acid methyl ester, and copolymers of acrylic acid and acrylic acid methylester. Preference is given to hydroxypropyl methylcellulose (hypromellose, HPMC) as film-forming agent.

Plasticizers include, but are not limited to polyethylene glycol, diethyl phthalate and glycerol. Preference is given to polyethylene glycol.

Coloring agents include, but are not limited to pigments, inorganic pigments, FD&C Red No. 3, FD&C Red No. 20, FD&C Yellow No. 6, FD&C Blue No. 2, D&C Green No. 5, D&C Orange No. 5, D&C Red No. 8, caramel, ferric oxide red, ferric oxide yellow and titanium dioxide. Preference is given to ferric oxide red, ferric oxide yellow and titanium dioxide.

Further commonly used pharmaceutical exipients which can be used as appropriate to formulate the composition for its intended route of administration include, but is not limited to: Acidifying agents for example acetic acid, citric acid, fumaric acid, hydrochloric acid and nitric acid; alkalizing agents for example ammonia solution, ammonium carbonate, diethanolamine, monoethanolamine, potassium hydroxide, sodium borate, sodium carbonate, sodium hydroxide, triethanolamine and trolamine; adsorbents for example powdered cellulose and activated charcoal; stabilizers and antioxidants for example ascorbic acid, ascorbyl palmitate, butylated hydroxyanisole, butylated hydroxytoluene, hypophosphorus acid, monothioglycerol, propyl gallate, sodium ascorbate, sodium bisulfite, sodium formaldehyde sulfoxylate and sodium metabisulfite; other binding materials for example block polymers, natural and synthetic rubber, polyacrylates, polyurethanes, silicones, polysiloxanes and styrene-butadiene copolymers; buffering agents for examples potassium metaphosphate, dipotassium phosphate, sodium acetate, sodium citrate anhydrous and sodium citrate hydrates; encapsulating agents for example gelatin, starch and cellulose derivates); flavorants, masking agents and odors for example anise oil, cinnamon oil, cocoa, menthol, orange oil, peppermint oil and vanillin; humectants for example glycerol, propylene glycol and sorbitol; sweeteners for example aspartame, dextrose, glycerol, mannitol, propylene glycol, saccharin sodium, sorbitol and sucrose; anti-adherents for example magnesium stearate and talc; direct compression excipients for example dibasic calcium phosphate, lactose and microcrystalline cellulose; tablet polishing agents for example carnauba wax and white wax.

Commonly used pharmaceutical ingredients which can be used as appropriate to formulate the composition for its intended route of administration include:

acidifying agents (examples include but are not limited to acetic acid, citric acid, fumaric acid, hydrochloric acid, nitric acid);

alkalinizing agents (examples include but are not limited to ammonia solution, ammonium carbonate, diethanolamine, monoethanolamine, potassium hydroxide, sodium borate, sodium carbonate, sodium hydroxide, triethanolamine, trolamine);

adsorbents (examples include but are not limited to powdered cellulose and activated charcoal);

aerosol propellants (examples include but are not limited to carbon dioxide, $CCl_2F_2$, $F_2ClC\text{-}CClF_2$ and $CClF_3$)

air displacement agents (examples include but are not limited to nitrogen and argon); antifungal preservatives (examples include but are not limited to benzoic acid, butylparaben, ethylparaben, methylparaben, propylparaben, sodium benzoate);

antimicrobial preservatives (examples include but are not limited to benzalkonium chloride, benzethonium chloride, benzyl alcohol, cetylpyridinium chloride, chlorobutanol, phenol, phenylethyl alcohol, phenylmercuric nitrate and thimerosal);

antioxidants (examples include but are not limited to ascorbic acid, ascorbyl palmitate, butylated hydroxyanisole, butylated hydroxytoluene, hypophosphorus acid, monothioglycerol, propyl gallate, sodium ascorbate, sodium bisulfite, sodium formaldehyde sulfoxylate, sodium metabisulfite);

binding materials (examples include but are not limited to block polymers, natural and synthetic rubber, polyacrylates, polyurethanes, silicones, polysiloxanes and styrene-butadiene copolymers);

buffering agents (examples include but are not limited to potassium metaphosphate, dipotassium phosphate, sodium acetate, sodium citrate anhydrous and sodium citrate dihydrate)

carrying agents (examples include but are not limited to acacia syrup, aromatic syrup, aromatic elixir, cherry syrup, cocoa syrup, orange syrup, syrup, corn oil, mineral oil, peanut oil, sesame oil, bacteriostatic sodium chloride injection and bacteriostatic water for injection) chelating agents (examples include but are not limited to edetate disodium

and edetic acid)

colorants (examples include but are not limited to FD&C Red No. 3, FD&C Red No. 20, FD&C Yellow No. 6, FD&C Blue No. 2, D&C Green No. 5, D&C Orange No. 5, D&C Red No. 8, caramel and ferric oxide red);

clarifying agents (examples include but are not limited to bentonite);

emulsifying agents (examples include but are not limited to acacia, cetomacrogol, cetyl alcohol, glyceryl monostearate, lecithin, sorbitan monooleate, polyoxyethylene 50 monostearate);

encapsulating agents (examples include but are not limited to gelatin and cellulose acetate phthalate)

flavorants (examples include but are not limited to anise oil, cinnamon oil, cocoa, menthol, orange oil, peppermint oil and vanillin);

humectants (examples include but are not limited to glycerol, propylene glycol and sorbitol); levigating agents (examples include but are not limited to mineral oil and glycerin);

oils (examples include but are not limited to arachis oil, mineral oil, olive oil, peanut oil, sesame oil and vegetable oil);

ointment bases (examples include but are not limited to lanolin, hydrophilic ointment, polyethylene glycol ointment, petrolatum, hydrophilic petrolatum, white ointment, yellow ointment, and rose water ointment);

penetration enhancers (transdermal delivery) (examples include but are not limited to monohydroxy or polyhydroxy alcohols, mono-or polyvalent alcohols, saturated or unsaturated fatty alcohols, saturated or unsaturated fatty esters, saturated or unsaturated dicarboxylic acids, essential oils, phosphatidyl derivatives, cephalin, terpenes, amides, ethers, ketones and ureas)

plasticizers (examples include but are not limited to diethyl phthalate and glycerol);

solvents (examples include but are not limited to ethanol, corn oil, cottonseed oil, glycerol, isopropanol, mineral oil, oleic acid, peanut oil, purified water, water for injection, sterile water for injection and sterile water for irrigation);

stiffening agents (examples include but are not limited to cetyl alcohol, cetyl esters wax, microcrystalline wax, paraffin, stearyl alcohol, white wax and yellow wax);

suppository bases (examples include but are not limited to cocoa butter and polyethylene glycols (mixtures));

surfactants (examples include but are not limited to benzalkonium chloride, nonoxynol 10, oxtoxynol 9, polysorbate 80, sodium lauryl sulfate and sorbitan mono-palmitate); suspending agents (examples include but are not limited to agar, bentonite, carbomers, carboxymethylcellulose sodium, hydroxyethyl cellulose, hydroxypropyl cellulose, hydroxypropyl methylcellulose, kaolin, methylcellulose, tragacanth and veegum); sweetening agents (examples include but are not limited to aspartame, dextrose, glycerol, mannitol, propylene glycol, saccharin sodium, sorbitol and sucrose);

tablet anti-adherents (examples include but are not limited to magnesium stearate and talc);

tablet binders (examples include but are not limited to acacia, alginic acid, carboxymethylcellulose sodium, compressible sugar, ethylcellulose, gelatin, liquid glucose, methylcellulose, non-crosslinked polyvinyl pyrrolidone, and pregelatinized starch);

tablet and capsule diluents (examples include but are not limited to dibasic calcium phosphate, kaolin, lactose, mannitol, microcrystalline cellulose, powdered cellulose, precipitated calcium carbonate, sodium carbonate, sodium phosphate, sorbitol and starch);

tablet coating agents (examples include but are not limited to liquid glucose, hydroxyethyl cellulose, hydroxypropyl cellulose, hydroxypropyl methylcellulose, methylcellulose, ethylcellulose, cellulose acetate phthalate and shellac);

tablet direct compression excipients (examples include but are not limited to dibasic calcium phosphate);

tablet disintegrants (examples include but are not limited to alginic acid, carboxymethylcellulose calcium, microcrystalline cellulose, polacrillin potassium, cross-linked polyvinylpyrrolidone, sodium alginate, sodium starch glycollate and starch);

tablet glidants (examples include but are not limited to colloidal silica, corn starch and talc);

tablet lubricants (examples include but are not limited to calcium stearate, magnesium stearate, mineral oil, stearic acid and zinc stearate);

tablet/capsule opaquants (examples include but are not limited to titanium dioxide);

tablet polishing agents (examples include but are not limited to carnauba wax and white wax);

thickening agents (examples include but are not limited to beeswax, cetyl alcohol and paraffin);

tonicity agents (examples include but are not limited to dextrose and sodium chloride); viscosity increasing agents (examples include but are not limited to alginic acid, bentonite, carbomers, carboxymethylcellulose sodium, methylcellulose, polyvinyl pyrrolidone, sodium alginate and tragacanth); and

wetting agents (examples include but are not limited to heptadecaethylene oxycetanol, lecithins, sorbitol monooleate, polyoxyethylene sorbitol monooleate, and polyoxyethylene stearate).

Dispersible powders and granules suitable for preparation of an aqueous suspension by the addition of water provide the active ingredient in admixture with a dispersing or wetting agent, suspending agent and one or more preservatives. Suitable dispersing or wetting agents and suspending agents are exemplified by those already mentioned

above. Additional excipients, for example, sweetening, flavoring and coloring agents, may also be present.

The pharmacuetical compositions may also be in the form of non-aqueous liquid formulations, e.g., oily suspensions which may be formulated by suspending the active ingredients in polyethyleneglycol, a vegetable oil, for example arachis oil, olive oil, sesame oil or peanut oil, or in a mineral oil such as liquid paraffin. The oily suspensions may contain a thickening agent, for example beeswax, hard paraffin or cetyl alcohol. Sweetening agents such as those set forth above, and flavoring agents may be added to provide palatable oral preparations. These compositions may be preserved by the addition of an anti-oxidant such as ascorbic acid.

Pharmaceutical compositions of the invention may also be in the form of oil-in-water emulsions. The oily phase may be a vegetable oil, for example olive oil or arachis oil, or a mineral oil, for example liquid paraffin or mixtures of these. Suitable emulsifying agents may be naturally-occurring gums, for example gum acacia or gum tragacanth, naturally-occurring phosphatides, for example soy bean, lecithin, and esters or partial esters derived from fatty acids and hexitol anhydrides, for example sorbitan monooleate, and condensation products of the said partial esters with ethylene oxide, for example polyoxyethylene sorbitan monooleate. The emulsions may also contain sweetening and flavoring agents.

Syrups and elixirs may be formulated with sweetening agents, for example glycerol, propylene glycol, sorbitol or sucrose. Such formulations may also contain a demulcent, a preservative and flavoring and coloring agents.

The active ingredients of the drug combination of the invention may also be administered transdermally using methods known to those skilled in the art (see, for example: Chien; "Transdermal Controlled Systemic Medications"; Marcel Dekker, Inc.; 1987. Lipp et al. WO94/04157 3Mar94). For example, a solution or suspension of an aryl urea compound in a suitable volatile solvent optionally containing penetration enhancing agents can be combined with additional additives known to those skilled in the art, such as matrix materials and bacteriocides. After sterilization, the resulting mixture can be formulated following known procedures into dosage forms. In addition, on treatment with emulsifying agents and water, a solution or suspension of an aryl urea compound may be formulated into a lotion or salve.

Suitable solvents for processing transdermal delivery systems are known to those skilled in the art, and include dimethylsulfoxide, lower alcohols such as ethanol or isopropyl alcohol, lower ketones such as acetone, lower carboxylic acid esters such as ethyl acetate, polar ethers such as tetrahydrofuran, lower hydrocarbons such as hexane, cyclohexane or benzene, or halogenated hydrocarbons such as dichloromethane, chloroform, trichlorotrifluoroethane, or trichlorofluoroethane. Suitable solvents may also include mixtures of one or more materials selected from lower alcohols, lower ketones, lower carboxylic acid esters, polar ethers, lower hydrocarbons, halogenated hydrocarbons.

Suitable penetration enhancing materials for transdermal delivery systems are known to those skilled in the art, and include, for example, monohydroxy or polyhydroxy alcohols such as ethanol, propylene glycol or benzyl alcohol, saturated or unsaturated $C_8$-$C_{18}$ fatty alcohols such as lauryl alcohol or cetyl alcohol, saturated or unsaturated $C_8$-$C_{18}$ fatty acids such as stearic acid, saturated or unsaturated fatty esters with up to 24 carbons such as methyl, ethyl, propyl, isopropyl, n-butyl, sec-butyl, isobutyl, tertbutyl or monoglycerin esters of acetic acid, capronic acid, lauric acid, myristinic acid, stearic acid, or palmitic acid, or diesters of saturated or unsaturated dicarboxylic acids with a total of up to 24 carbons such as diisopropyl adipate, diisobutyl adipate, diisopropyl sebacate, diisopropyl maleate, or diisopropyl fumarate. Additional penetration enhancing materials include phosphatidyl derivatives such as lecithin or cephalin, terpenes, amides, ketones, ureas and their derivatives, and ethers such as dimethyl isosorbid and diethyleneglycol monoethyl ether. Suitable penetration enhancing formulations may also include mixtures of one or more materials selected from monohydroxy or polyhydroxy alcohols, saturated or unsaturated $C_8$-$C_{18}$ fatty alcohols, saturated or unsaturated $C_8$-$C_{18}$ fatty acids, saturated or unsaturated fatty esters with up to 24 carbons, diesters of saturated or unsaturated discarboxylic acids with a total of up to 24 carbons, phosphatidyl derivatives, terpenes, amides, ketones, ureas and their derivatives, and ethers.

Suitable binding materials for transdermal delivery systems are known to those skilled in the art and include polyacrylates, silicones, polyurethanes, block polymers, styrenebutadiene copolymers, and natural and synthetic rubbers. Cellulose ethers, derivatized polyethylenes, and silicates may also be used as matrix components. Additional additives, such as viscous resins or oils may be added to increase the viscosity of the matrix. Specific preparations of compounds within the drug combination of this invention can be adapted from others known in the art. For example, Riedl, B., et al., "O-Carboxy Aryl Substituted Diphenyl Ureas as raf Kinase Inhibitors" PCT Int. Appl., WO 00 42012, Riedl, B., et al., "O-Carboxy Aryl Substituted Diphenyl Ureas as p38 Kinase Inhibitors" PCT Int. Appl., WO 00 41698, incorporated herein by reference.

Pharmaceutical compositions according to the present invention can be illustrated as follows:

Sterile IV Solution: A 5 mg/ml solution of a desired compound of the drug combination of this invention is made using sterile, injectable water, and the pH is adjusted if necessary. The solution is diluted for administration to 1 - 2 mg/ml with sterile 5% dextrose and is administered as an IV infusion over 60 minutes.

Lyophilized powder for IV administration: A sterile preparation can be prepared with (i) 100 - 1000 mg of a desired

compound of the drug combination of this invention as a lyophilized powder, (ii) 32- 327 mg/ml sodium citrate, and (iii) 300 - 3000 mg Dextran 40. The formulation is reconstituted with sterile, injectable saline or dextrose 5% to a concentration of 10 to 20 mg/ml, which is further diluted with saline or dextrose 5% to 0.2 - 0.4 mg/ml, and is administered either IV bolus or by IV infusion over 15 - 60 minutes.

Intramuscular suspension: The following solution or suspension can be prepared, for intramuscular injection:

50 mg/ml of the desired, water-insoluble compound of the drug combination of this invention
5 mg/ml sodium carboxymethylcellulose
4 mg/ml TWEEN 80
9 mg/ml sodium chloride
9 mg/ml benzyl alcohol

Hard Shell Capsules: A large number of unit capsules are prepared by filling standard two-piece hard galantine capsules each with 100 mg of powdered active ingredient, 150 mg of lactose, 50 mg of cellulose and 6 mg of magnesium stearate.

Soft Gelatin Capsules: A mixture of active ingredient in a digestible oil such as soybean oil, cottonseed oil or olive oil is prepared and injected by means of a positive displacement pump into molten gelatin to form soft gelatin capsules containing 100 mg of the active ingredient. The capsules are washed and dried. The active ingredient can be dissolved in a mixture of polyethylene glycol, glycerin and sorbitol to prepare a water miscible medicine mix.

Tablets: A large number of tablets are prepared by conventional procedures so that the dosage unit was 100 mg of active ingredient, 0.2 mg. of colloidal silicon dioxide, 5 mg of magnesium stearate, 275 mg of microcrystalline cellulose, 11 mg. of starch, and 98.8 mg of lactose. Appropriate aqueous and non-aqueous coatings may be applied to increase palatability, improve elegance and stability or delay absorption.

Immediate Release Tablets/Capsules: These are solid oral dosage forms made by conventional and novel processes. These units are taken orally without water for immediate dissolution and delivery of the medication. The active ingredient is mixed in a liquid containing ingredient such as sugar, gelatin, pectin and sweeteners. These liquids are solidified into solid tablets or caplets by freeze drying and solid-state extraction techniques. The drug compounds may be compressed with viscoelastic and thermoelastic sugars and polymers or effervescent components to produce porous matrices intended for immediate release, without the need of water.

The invention also encompasses kits for treating mammalian cancers. Such kits can be used to treat a patient with a raf kinase stimulated cancer as well as cancers not stimulated through raf kinase. The kit can comprise a single pharmaceutical formulation containing a substitituted diaryl urea compound of Formula I (e.g., Sorafenib) and taxane, (e.g., paclitaxel) and a platinum complex (e.g., carboplatin). Alternatively, the kit can comprise a substituted diaryl urea compound a taxane and platinum complex in separate formulations. The kit can also include instructions for how to administer the compounds to a patient with cancer in need of treatment. The kit can be used to treat different cancer types which include but are not limited to NSCLC, colon, prostate, leukemia, melanoma, hepatocellular, renal, head and neck, glioma, lung, pancreatic, ovarian, and mammary.

It will be appreciated by those skilled in the art that the particular method of administration will depend on a variety of factors, all of which are routinely considered when administering therapeutics. It will also be understood, however, that the specific dose level for any given patient will depend upon a variety of factors, including, the activity of the specific compound employed, the age of the patient, the body weight of the patient, the general health of the patient, the gender of the patient, the diet of the patient, time of administration, route of administration, rate of excretion, drug combinations, and the severity of the condition undergoing therapy. It will be further appreciated by one skilled in the art that the optimal course of treatment, i.e., the mode of treatment and the daily number of doses of one or more of the drugs within the combination (or a pharmaceutically acceptable salt thereof) given for a defined number of days, can be ascertained by those skilled in the art using conventional treatment tests.

Based upon standard laboratory techniques known to evaluate compounds useful for the treatment of hyperproliferative disorders, by standard toxicity tests and by standard pharmacological assays for the determination of treatment of the conditions identified above in mammals, and by comparison of these results with the results of known medicaments that are used to treat these conditions, the effective dosage of the compounds of this invention can readily be determined for treatment of each desired indication. The amount of the active ingredient to be administered in the treatment of one of these conditions can vary widely according to such considerations as the particular compound and dosage unit employed, the mode of administration, the period of treatment, the age and gender of the patient treated,

and the nature and extent of the condition treated.

Generally, the use of the drug combination of this invention will serve to (1) yield better efficacy in reducing the growth of a tumor or even eliminate the tumor as compared to administration of a single chemotherapeutic agent, (2) provide for the administration of lesser amounts of the administered chemotherapeutic agents, (3) provide for a chemotherapeutic treatment that is well tolerated in the patient with less deleterious pharmacological complications resulting from larger doses of single chemotherapies and certain other combined therapies, (4) provide for treating a broader spectrum of different cancer types in mammals, especially humans, (5) provide for a higher response rate among treated patients, (6) provide for a longer survival time among treated patients compared to standard chemotherapy treatments, (7) provide a longer time for tumor progression, and/or (8) yield efficacy and tolerability results at least as good as those of the agents used alone, compared to known instances where other cancer agent combinations produce antagonist effects.

The substituted diaryl urea compound of Formula I can be administered to a patient at a dosage which can range from about 0.001 to about 300 mg/Kg of total body weight. A unit dosage may contain from about 0.5 mg to about 2000 mg of active ingredient, and can be administered one or more times per day. Preference is given to an amount of the substituted diarylurea compound of Formula (I) in the pharmaceutical composition from 20 to 2000 mg, preferably from 40 to 800 mg, more preferably from 50 to 600 mg. Particular preference is given to an amount of p-toluenesulfonic acid salt of 4{4-[3-(4-chloro-3-trifluoromethylphenyl)-ureido]-phenoxy}-pyridine-2-carboxylic acid methyl amide in the pharmaceutical composition from 27 to 2740 mg, preferably from 54 to 1096, more preferably from 68 to 822 mg.

The daily dose for oral administration will preferably be from 0.1 to 300 mg/kg of total body weight, typically from about 0.1 mg/kg to about 50 mg/kg body weight per day. The daily dosage for administration by injection which includes intravenous, intramuscular, subcutaneous and parenteral injection as well as infusion techniques will preferably be from 0.1 to 300 mg/kg of total body weight. The daily vaginal dosage regime will preferably be from 0.1 to 300 mg/kg of total body weight. The daily topical dosage regimen will preferably be from 0.1 to 300 mg administered between one to four times daily. The transdermal concentration will preferably be that required to maintain a daily dose of from 1 to 300 mg/kg. For all the above mentioned routes of administration, the preferred dosage is 0.1 to 300 mg/kg. The daily inhalation dosage regimen will preferably be from 0.1 to 300 mg/kg of total body weight.

The taxanes such as paclitaxel and platinum complexes such as carboplatin are each preferably administered non-orally, more preferably by intravenous infusion, in conventional amounts routinely used in cancer monotherapy or reduced amounts based on the combination of active agents.

Based on body surface area, the infusion dosage of paclitaxel may range from about 10 to 300 mg/m$^2$, preferably from about 30 to 200 mg/m$^2$ and more preferably about 100, 135 or 175 mg/m$^2$. Paclitaxel infusions should be preceded with appropriate premedications known to those skilled in the art. The paclitaxel dosage is preferably administered intravenously by infusion over a period of at least about 3 hours, preferably over a period of about 3 or 24 hours. The carboplatin dosage is preferably administered intravenously by infusion, preferably over a period of at least about 15 minutes. The infusion dosage of carboplatin may range from about 100 to 500 mg/m$^2$ preferably about 300 or 360 mg/m$^2$.

The combined dosages of carboplatin and paclitaxel can range from 50 to 500 mg/m$^2$, 100 to 400 mg/m$^2$, 150 to 300 mg/m$^2$, 175-275 mg/m$^2$ and 200-250 mg/m$^2$ for a single infusion.

For each of the substituted diaryl urea compounds of Formula I, taxanes and platinum complexes, the administered dosage of the compound may be modified depending on any superior or unexpected results which may be obtained as routinely determined with this invention. Based upon standard laboratory techniques known to evaluate compounds useful for the treatment of hyper-proliferative disorders, by standard toxicity tests and by standard pharmacological assays for the determination of treatment of the conditions identified above in mammals, and by comparison of these results with the results of known medicaments that are used to treat these conditions, the effective dosage of the pharmaceutical compositions of this invention can readily be determined by those skilled in the art. The amount of the administered active ingredient can vary widely according to such considerations as the particular compound and dosage unit employed, the mode and time of administration, the period of treatment, the age, sex, and general condition of the patient treated, the nature and extent of the condition treated, the rate of drug metabolism and excretion, the potential drug combinations and drug-drug interactions, and the like.

The substituted diaryl urea compounds of Formula I can be administered orally, topically, parenterally, rectally, by inhalation, and by injection. Administration by injection includes intravenous, intramuscular, subcutaneous, and parenterally as well as by infusion techniques. The aryl urea compound can be present in association with one or more non-toxic pharmaceutically acceptable carriers and if desired other active ingredients. A preferred route of administration for the aryl urea compound is oral administration.

The taxanes and platinum complexes can be administered to a patient by any of the conventional routes of administration for these compounds. This can include oral, topical, parenteral, rectal and inhalation administration as well as injection. Administration by injection includes intravenous, intramuscular, subcutaneous, and parenterally as well as by infusion techniques. The preferred route of administration for the taxanes and platinum complexes used in this invention is typically by injection which is the same route of administration used for the agent alone. Any of the taxanes

and platinum complexes can be administered in combination with a substituted diaryl urea compound by any of the mentioned routes of administration.

For administering the substituted diaryl urea compound of Formula I and both the taxane and platinum complex, by any of the routes of administration herein discussed, the substituted diaryl urea compound can be administered simultaneously with the taxane and platinum complex. This can be performed by administering a single formulation which contains both the substituted diaryl urea compound and the taxane and platinum complex or administering the substituted diaryl urea compound and the taxane and platinum complex in independent formulations at the same time to a patient.

Alternatively, the substituted diaryl urea compound of Formula I can be administered in tandem with the taxane and platinum complex. The substituted diaryl urea compound can be administered prior to either the taxane, the platinum complex or both. For example, the substituted aryl urea compound can be administered once or more times per day up to 28 consecutive days followed by administration of the taxane and platinum complex. Also, either the taxane, platinum complex or both can be administered first followed by administration of the substituted diaryl urea compound. The choice of sequence administration of the substituted diaryl urea compound relative to the taxane and platinum complex may vary for different agents. Also, the taxane and platinum complex can be administered using any regimen which is conventionally used for these agents.

In another regimen of administration, the substituted diaryl urea compound of Formula I and the taxane and platinum complex can be administered one or more times per day on the day of administration.

Method of Use

The present invention also relates to a method for using the combinations of this invention to manufacture medicaments or pharmaceutical composition according to the invention to treat mammalian hyper-proliferative disorders, including cancer.

Method of treating hyper-proliferative disorders

The present invention also relates to a method for using pharmaceutical composition according to the invention to treat mammalian hyper-proliferative disorders, including cancer. This method comprises administering a pharmaceutical composition containing the drug combination to a mammal in need thereof, including a human, an amount which is effective to treat the disorder.

The term "hyper-proliferative disorders" and/or "cancer" not only refers to solid tumors, such as cancers of the breast, respiratory tract, brain, reproductive organs, digestive tract, urinary tract, eye, liver, skin, head and neck, thyroid, parathyroid and their distant metastases, but also includes lymphomas, sarcomas, and leukaemias.

Drug combinations of the present invention can be utilized to treat any diseases or conditions that are associated with, or mediated by, the cellular pathways modulated by the compounds comprising the combinations. These pathways, include, but are not limited to signaling pathways which comprise, e.g., VEGFR, VEGFR2, Raf/Mek/Erk, Akt/PI3K, MTOR, PTEN, etc. (see also above). The drug combinations can be useful to treat diseases that are associated with, or mediated by, mutations in one of more genes present in these pathways, including cancer-associated mutations in PTEN, ras, Raf, Akt, PI3K, etc.

Although the compounds may be known as specific inhibitors, the present invention includes any ameliorative or therapeutic effect, regardless of the mechanism of action or how it is achieved.

The drug combination can have one or more of the following activities, including, antiproliferative; anti-tumor; anti-angiogenic; inhibiting the proliferation of endothelial or tumor cells; anti-neoplastic; immunosuppressive; immunomodulatory; apoptosis-promoting, etc. Conditions or diseases that can be treated in accordance with the present invention include proliferative disorders such as cancer.

Any tumor or cancer can be treated, including, but not limited to, cancers having one or more mutations in raf, VEGFR-2, VEGFR-3, PDGFR-beta, Flt-3, ras, PTEN, Akt, PI3K, mTOR, as well as any upstream or downstream member of the signaling pathways of which they are a part. A tumor or cancer can be treated with a compound of the present invention irrespective of the mechanism that is responsible for it. Cancers of any organ can be treated, including cancers of, but are not limited to, e.g., colon, pancreas, breast, prostate, bone, liver, kidney, lung, testes, skin, pancreas, stomach, prostate, ovary, uterus, head and neck, blood cell, lymph, etc. Cancers that can be treated in accordance with the present invention include, especially, but not limited to, brain tumors, breast cancer, bone sarcoma (e.g., osteosarcoma and Ewings sarcoma), bronchial premalignancy, endometrial cancer, glioblastoma, hematologic malignancies, hepatocellular carcinoma, Hodgkin's disease, kidney neoplasms, leukemia, leimyosarcoma, liposarcoma, lymphoma, Lhermitte-Duclose disease, malignant glioma, melanoma, malignant melanoma, metastases, multiple myeloma, myeloid metaplasia, myeloplastic syndromes, non-small cell lung cancer, pancreatic cancer, prostate cancer, renal cell carcinoma (e.g., advanced, advanced refractory), rhabdomyosarcoma, soft tissue sarcoma, squamous epithelial carcinoma of the skin,

cancers associated with loss of function of PTEN; activated Akt (e.g. PTEN null tumors and tumors with ras mutations).

Examples of breast cancer include, but are not limited to, invasive ductal carcinoma, invasive lobular carcinoma, ductal carcinoma in situ, and lobular carcinoma in situ.

Examples of cancers of the respiratory tract include, but are not limited to, small-cell, non-small-cell lung carcinoma, bronchial adenoma, and pleuropulmonary blastoma.

Examples of brain cancers include, but are not limited to, brain stem and hypophtalmic glioma, cerebellar and cerebral astrocytoma, medulloblastoma, ependymoma, and neuroectodermal and pineal tumor.

Tumors of the male reproductive organs include, but are not limited to, prostate and testicular cancer. Tumors of the female reproductive organs include, but are not limited to, endometrial, cervical, ovarian, vaginal, and vulvar cancer, as well as sarcoma of the uterus.

Tumors of the digestive tract include, but are not limited to, anal, colon, colorectal, esophageal, gallbladder, gastric, pancreatic, rectal, small intestine, and salivary gland cancers. Tumors of the urinary tract include, but are not limited to, bladder, penile, kidney, renal pelvis, ureter, and urethral cancers.

Eye cancers include, but are not limited to, intraocular melanoma and retinoblastoma. Examples of liver cancers include, but are not limited to, hepatocellular carcinoma (liver cell carcinomas with or without fibrolamellar variant), cholangiocarcinoma (intrahepatic bile duct carcinoma), and mixed hepatocellular cholangiocarcinoma.

Skin cancers include, but are not limited to, squamous cell carcinoma, Kaposi's sarcoma, malignant melanoma, Merkel cell skin cancer, and non-melanoma skin cancer. Head-and-neck cancers include, but are not limited to, laryngeal, hypopharyngeal, nasopharyngeal, and/or oropharyngeal cancers, and lip and oral cavity cancer.

Lymphomas include, but are not limited to, AIDS-related lymphoma, non-Hodgkin's lymphoma, cutaneous T-cell lymphoma, Hodgkin's disease, and lymphoma of the central nervous system.

Sarcomas include, but are not limited to, sarcoma of the soft tissue, osteosarcoma, malignant fibrous histiocytoma, lymphosarcoma, and rhabdomyosarcoma.

Leukemias include, but are not limited to, acute myeloid leukemia, acute lymphoblastic leukemia, chronic lymphocytic leukemia, chronic myelogenous leukemia, and hairy cell leukemia.

In addition to inhibiting the proliferation of tumor cells, compounds of the present invention can also cause tumor regression, e.g., a decrease in the size of a tumor, or in the extent of cancer in the body.

In addition, the drug combinations can be administered to modulate one or more the following processes, cell growth (e.g., proliferation), tumor cell growth (including, e.g., differentiation, cell survival, and/or proliferation), tumor regression, endothelial cell growth (including, e.g., differentiation, cell survival, and/or proliferation), angiogenesis (blood vessel growth), angiogenesis, and/or hematopoiesis (e.g., proliferation, T-cell development, etc.).

(The drugs can also be administered sequentially at different times. Agents can be formulated conventionally to achieve the desired rates of release over extended period of times, e.g., 12-hours, 24-hours. This can be achieved by using agents and/or their derivatives which have suitable metabolic half-lives, and/or by using controlled release formulations.

The drug combinations can be synergistic, e.g., where the joint action of the drugs is such that the combined effect is greater than the algebraic sum of their individual effects. Thus, reduced amounts of the drugs can be administered, e.g., reducing toxicity or other deleterious or unwanted effects, and/or using the same amounts as used when the agents are administered alone, but achieving greater efficacy, e.g., in having more potent antiproliferative and pro-apoptotic action. Compounds of the present invention can be further combined with any other suitable additive or pharmaceutically acceptable carrier. Such additives include any of the substances already mentioned, as well as any of those used conventionally, such as those described in Remington: The Science and Practice of Pharmacy (Gennaro and Gennaro, eds, 20th edition, Lippincott Williams & Wilkins, 2000); Theory and Practice of Industrial Pharmacy (Lachman et al., eds., 3rd edition, Lippincott Williams & Wilkins, 1986); Encyclopedia of Pharmaceutical Technology (Swarbrick and Boylan, eds., 2nd edition, Marcel Dekker, 2002). These can be referred to herein as "pharmaceutically acceptable carriers" to indicate they are combined with the active drug and can be administered safely to a subject for therapeutic purposes. In addition, compounds of the present invention can be administered with other active agents or therapies (e.g., radiation) that are utilized to treat any of the above-mentioned diseases and/or conditions.

## Assays

Activity of combinations of the present invention can be determined according to any effective in vitro or in vivo method.

## Kinase activity

Kinase activity can be determined routinely using conventional assay methods. Kinase assays typically comprise the kinase enzyme, substrates, buffers, and components of a detection system. A typical kinase assay involves the

reaction of a protein kinase with a peptide substrate and an ATP, such as $^{32}P$-ATP, to produce a phosphorylated end-product (for instance, a phosphoprotein when a peptide substrate is used). The resulting end-product can be detected using any suitable method. When radioactive ATP is utilized, a radioactively labeled phosphoprotein can be separated from the unreacted gamma-$^{32}P$-ATP using an affinity membrane or gel electrophoresis, and then visualized on the gel using autoradiography or detected with a scintillation counter. Non-radioactive methods can also be used. Methods can utilize an antibody which recognizes the phosphorylated substrate, e.g., an anti-phosphotyrosine antibody. For instance, kinase enzyme can be incubated with a substrate in the presence of ATP and kinase buffer under conditions which are effective for the enzyme to phosphorylate the substrate. The reaction mixture can be separated, e.g., electrophoretically, and then phosphorylation of the substrate can be measured, e.g., by Western blotting using an anti-phosphotyrosine antibody. The antibody can be labeled with a detectable label, e.g., an enzyme, such as HRP, avidin or biotin, chemiluminescent reagents, etc. Other methods can utilize ELISA formats, affinity membrane separation, fluorescence polarization assays, luminescent assays, etc. An alternative to a radioactive format is time-resolved fluorescence resonance energy transfer (TR-FRET). This method follows the standard kinase reaction, where a substrate, e.g., biotinylated poly (GluTyr), is phosphorylated by a protein kinase in the presence of ATP. The end-product can then detected with a europium chelate phosphospecific antibody (anti-phosphotyrosine or phosphoserine/threonine), and streptavidin-APC, which binds the biotinylated substrate. These two components are brought together spatially upon binding, and energy transfer from the phosphospecific antibody to the acceptor (SA-APC) produces fluorescent readout in the homogeneous format.

Raf/MEK/ERK activity

A c-Raf kinase assay can be performed with a c-Raf enzyme activated (phosphorylated) by Lck kinase. Lck-activated c-Raf (Lck/c-Raf) is produced in Sf9 insect cells by co-infecting cells with baculoviruses expressing, under the control of the polyhedrin promoter, GST-c-Raf (from amino acid 302 to amino acid 648) and Lck (full-length). Both baculoviruses are used at the multiplicity of infection of 2.5 and the cells are harvested 48 hours post infection. MEK-1 protein is produced in Sf9 insect cells by infecting cells with the baculovirus expressing GST-MEK-1 (full-length) fusion protein at the multiplicity of infection of 5 and harvesting the cells 48 hours post infection. Similar purification procedure is used for GST-c-Raf 302-648 and GST-MEK-1.

Transfected cells are suspended at 100 mg of wet cell biomass per mL in a buffer containing 10 mM sodium phosphate, 140 mM sodium chloride pH 7.3, 0.5% Triton X-100 and the protease inhibitor cocktail. The cells are disrupted with a Polytron homogenizer and centrifuged 30,000g for 30 minutes. The 30,000g supernatant is applied applied onto GSH-Sepharose. The resin is washed with a buffer containing 50 mM Tris, pH 8.0, 150 mM NaCl and 0.01% Triton X-100. The GST-tagged proteins are eluted with a solution containing 100 mM Glutathione, 50 mM Tris, pH 8.0, 150 mM NaCl and 0.01% Triton X-100. The purified proteins are dialyzed into a buffer containing 20 mM Tris, pH 7.5, 150 mM NaCl and 20% Glycerol.

Test compounds are serially diluted in DMSO using three-fold dilutions to stock concentrations ranging typically from 50 μM to 20 nM (e.g., final concentrations in the assay can range from 1 μM to 0.4 nM). The c-Raf biochemical assay is performed as a radioactive filtermat assay in 96-well Costar polypropylene plates (Costar 3365). The plates are loaded with 75 μL solution containing 50 mM HEPES pH 7.5, 70 mM NaCl, 80 ng of Lck/c-Raf and 1 μg MEK-1. Subsequently, 2 μL of the serially diluted individual compounds is added to the reaction, prior to the addition of ATP. The reaction is initiated with 25 μL ATP solution containing 5μM ATP and 0.3 μCi [33P]-ATP. The plates were sealed and incubated at 32°C for 1 hour. The reaction is quenched with the addition of 50 μl of 4 % Phosphoric Acid and harvested onto P30 filtermats (PerkinElmer) using a Wallac Tomtec Harvester. Filtermats are washed with 1 % Phosphoric Acid first and deinonized H2O second. The filters are dried in a microwave, soaked in scintillation fluid and read in a Wallac 1205 Betaplate Counter (Wallac Inc., Atlanta, GA, U.S.A.). The results are expressed as percent inhibition.

$$\% \text{ Inhibition} = [100-(\text{Tib}/\text{Ti})] \times 100$$

where
Tib = (counts per minute with inhibitor)-(background)
Ti = (counts per minute without inhibitor)-(background)

Raf activity can also be monitored by its ability to initiate the cascade leading to ERK phosphorylation (i.e., raf/MEK/ERK), resulting in phospho-ERK. A Bio-Plex Phospho-ERK1/2 immunoassay can be performed as follows:

A 96-well phospho-ERK (pERK) immunoassay, using laser flow cytometry platform has been established to measure inhibition of basal pERK in cell lines. MDA-MB-231 cells are plated at 50,000 cells per well in 96-well microtitre

plates in complete growth media. For effects of test compounds on basal pERK1/2 inhibition, the next day after plating, MDA-MB-231 cells are transferred to DMEM with 0.1% BSA and incubated with test compounds diluted 1:3 to a final concentration of 3 mM to 12 nM in 0.1 % DMSO. Cells are incubated with test compounds for 2 h, washed, and lysed in Bio-Plex whole cell lysis buffer A. Samples are diluted with buffer B 1:1 (v/v) and directly transferred to assay plate or frozen at -80 C degrees until processed. 50 mL of diluted MDA-MB-231 cell lysates are incubated with about 2000 of 5 micron Bio-Plex beads conjugated with an anti-ERK1/2 antibody overnight on a shaker at room temperature. The next day, biotinylated phospho-ERK1/2 sandwich immunoassay is performed, beads are washed 3 times during each incubation and then 50 mL of PE-strepavidin is used as a developing reagent. The relative fluorescence units of pERK1/2 is detected by counting 25 beads with Bio-Plex flow cell (probe) at high sensitivity. The IC50 is calculated by taking untreated cells as maximum and no cells (beads only) as background.

Cell proliferation

An example of a cell proliferation assay is described in the Examples below. However, proliferation assays can be performed by any suitable method. For example, a breast carcinoma cell proliferation assay can be performed as follows. Other cell types can be substituted for the MDA-MB-231 cell line.

Human breast carcinoma cells (MDA MB-231, NCI) are cultured in standard growth medium (DMEM) supplemented with 10% heat-inactivated FBS at 37°C in 5% $CO_2$ (vol/ vol) in a humidified incubator. Cells are plated at a density of 3000 cells per well in 90 μL growth medium in a 96 well culture dish. In order to determine $T_{0h}$ CTG values, 24 hours after plating, 100 μL of CellTiter-Glo Luminescent Reagent (Promega) is added to each well and incubated at room temperature for 30 minutes. Luminescence is recorded on a Wallac Victor II instrument. The CellTiter-Glo reagent results in cell lysis and generation of a luminescent signal proportional to the amount of ATP present, which, in turn is directly proportional to the number of cells present.

Test compounds are dissolved in 100% DMSO to prepare 10 mM stocks. Stocks are further diluted 1:400 in growth medium to yield working stocks of 25 μM test compound in 0.25% DMSO. Test compounds are serially diluted in growth medium containing 0.25% DMSO to maintain constant DMSO concentrations for all wells. 60 μL of diluted test compound are added to each culture well to give a final volume of 180 μL. The cells with and without individual test compounds are incubated for 72 hours at which time ATP dependent luminescence was measured, as described previously, to yield $T_{72h}$ values. Optionally, the $IC_{50}$ values can be determined with a least squares analysis program using compound concentration versus percent inhibition.

$\% \text{ Inhibition} = [1-(T_{72h\ test}-T_{0h})/(T_{72h\ ctrl}-T_{0h})] \times 100$, where

$T_{72h\ test}$ = ATP dependent luminescence at 72 hours in the presence of test compound

$T_{72h\ ctrl}$ = ATP dependent luminescence at 72 hours in the absence of test compound

$T_{0h}$ = ATP dependent luminescence at Time Zero.

Angiogenesis

One useful model to study angiogenesis is based on the observation that, when a reconstituted basement membrane matrix, such as Matrigel, supplemented with growth factor (e.g., FGF-1), is injected subcutaneously into a host animal, endothelial cells are recruited into the matrix, forming new blood vessels over a period of several days. See, e.g., Passaniti et al., Lab. Invest., 67:519-528, 1992. By sampling the extract at different times, angiogenesis can be temporally dissected, permitting the identification of genes involved in all stages of angiogenesis, including, e.g., migration of endothelial cells into the matrix, commitment of endothelial cells to angiogenesis pathway, cell elongation and formation of sac-like spaces, and establishment of functional capillaries comprising connected, and linear structures containing red blood cells. To stabilize the growth factor and/or slow its release from the matrix, the growth factor can be bound to heparin or another stabilizing agent. The matrix can also be periodically re-infused with growth factor to enhance and extend the angiogenic process.

Other useful systems for studying angiogenesis, include, e.g., neovascularization of tumor explants (e.g., U.S. Pat. Nos. 5,192,744; 6,024,688), chicken chorioallantoic membrane (CAM) assay (e.g., Taylor and Folkman, Nature, 297:307-312, 1982; Eliceiri et al., J. Cell Biol., 140, 1255-1263, 1998), bovine capillary endothelial (BCE) cell assay (e.g., U.S. Pat. No. 6,024,688; Polverini, P. J. et al., Methods Enzymol., 198: 440-450, 1991), migration assays, HUVEC (human umbilical cord vascular endothelial cell) growth inhibition assay (e.g., U.S. Pat. No. 6,060,449).

**Phase I data:**

"Phase I data on the combination of Sorafenib, Carboplatin and Paclitaxel in treating solid tumors is reported by Flaherty et al. in the document entitled "A Phase I Trial of the Oral, Multi-kinase Inhibitor Sorafenib in Combination with Carboplatin and Paclitaxel" and appended to US provisional application 60/859,241, filed November 16, 2006

as "Appendix A." Appendix A of US provisional application 60/859,241, filed November 16, 2006, is made part of this application and follows.

A Phase I Trial of the Oral, Multi-kinase Inhibitor Sorafenib in Combination with Carboplatin and Paclitaxel

Keith T. Flaherty, Joan Schiller, Lynn M. Schuchter, [Insert 2nd Wisconsin author], David A. Tuveson, Maryann Redlinger, Chetan Lathia, Chenghua Xia, Oana Petrenciuc, Sunil R. Hingorani, Michael A. Jacobetz, Pat VanBelle, David Elder, Marcia S. Brose, Barbara L. Weber, [Insert 3rd Wisconsin author], and Peter J. O'Dwyer

From The Abramson Cancer Center of the University of Pennsylvania, Philadelphia, PA; University of Wisconsin Cancer Center, Madison, WI; and Bayer Pharmaceuticals Corporation, West Haven, CT, USA.

**Research Support:** Authors to provide information

**Corresponding Author:** Keith T. Flaherty, M.D., Abramson Cancer Center of the University of Pennsylvania, 51 N. 39th Street, Medical Arts Building, Suite 103, 39th & Market Streets, Philadelphia, PA 19104.
Phone: +1-215-662-8624; Fax: +1-215-243-3269
Email: ktflaherty@aol.com

**Running Head:** Phase I sorafenib-carboplatin-paclitaxel trial

**Presented Previously:** ASCO 2004

**Disclaimers:** Authors to provide information

**Keywords:** Sorafenib, carboplatin, paclitaxel, metastatic melanoma, pharmacokinetics, safety

**Abstract Length:** 249 (Limit 250)

**Word Count:** 2969 (Limit 3000)

## ABSTRACT

### Purpose

The present study evaluated the safety, maximum tolerated dose, pharmacokinetics, and antitumor activity of sorafenib, a multi-kinase inhibitor, combined with paclitaxel and carboplatin in patients with solid tumors.

### Patients and Methods

Thirty nine patients with advanced cancer (24 with melanoma) received oral sorafenib 100 mg, 200 mg, or 400 mg twice daily on Days 2-19 of a 21-day cycle. All patients received carboplatin corresponding to plasma area under the curve of 6 (AUC6) and paclitaxel 225 mg/m$^2$ on Day 1. Pharmacokinetic analyses were performed for sorafenib on Days 2 and 19 of Cycle 1 and for platinum and paclitaxel on Day 1 of Cycles 1 and 2. Pretreatment tumor samples from 17 melanoma patients were analyzed for *B-Raf* mutations.

### Results

Sorafenib was well tolerated at the doses evaluated. The most frequent severe adverse events were hematologic toxicities (grade 3 or 4 in 33 patients, 85%). Twenty seven patients (69%) had sorafenib-related adverse events, the most frequent of which were dermatologic events (26 patients, 67%). Exposure to carboplatin and paclitaxel was not altered by intervening treatment with sorafenib. Treatment with sorafenib, paclitaxel, and carboplatin was associated with one complete response and nine partial responses, all among patients with melanoma. There was no clear relationship between *B-Raf* mutation status and tumor responses in patients with melanoma.

### Conclusions

The recommended Phase II doses are oral sorafenib 400 mg twice daily, carboplatin at an AUC6 dose, and paclitaxel 225 mg/m$^2$. The tumor responses observed in patients with melanoma warrant further investigation.

## INTRODUCTION

Sorafenib (BAY 43-9006; Nexavar®) is a potent oral multi-kinase inhibitor that inhibits the activities of Raf-1, wild-type B-Raf, mutant $^{V600E}$B-Raf, c-Kit, Ret, vascular endothelial growth factor receptors-1/-2/-3, and platelet-derived growth factor receptor-□ *in vitro.*[1-3] *In vivo,* sorafenib has been shown to inhibit tumor growth in several xenograft models containing *B-Raf* or *K-Ras* mutations, e.g. colon, non-small-cell lung cancer [NSCLC], and melanoma.[1] In melanoma xenograft models, sorafenib slowed tumor development by inhibiting $^{V600E}$B-Raf activity, phosphorylation of MEK and ERK, and vascular development, in a manner similar to small interfering (si)RNA directed against *B-Raf.*[4] In single-agent Phase I/II trials, sorafenib demonstrated a favorable toxicity profile.[5,6] Preliminary efficacy analysis revealed anti-tumor

activity of sorafenib in patients with a variety of solid tumors.[5,6] In addition, a Phase III trial of single-agent therapy in patients with renal cell carcinoma (RCC) showed that treatment with sorafenib resulted in median progression-free survival of 24 weeks, compared with 12 weeks in patients receiving placebo.[7] Sorafenib has now been approved in the US and Europe for the treatment of RCC.

In preclinical studies, combinations of sorafenib with other anticancer agents inhibited the growth of human cancer cells *in vitro,*[8] and in xenograft models.[9] Combinations of sorafenib with other anticancer agents have yielded encouraging results in clinical trials on patients with a variety of cancers.[10-14] However, sorafenib monotherapy had limited clinical success as stable disease was achieved in only 16% of patients with melanoma.[15] Similarly, treatment of patients with melanoma with carboplatin or paclitaxel singly yielded overall response rates (ORR) <20%.[16-22] Combination of carboplatin and paclitaxel was not better with ORR of <10% and 20% in two trials.[22,23] In a retrospective analysis, 45% of patients with melanoma treated with a carboplatin-paclitaxel combination had either partial response or stable disease suggesting that this regimen had promise, perhaps in combination with other therapies.[24] Thus, it was reasonable to consider the possible utility of a combination of sorafenib, paclitaxel and carboplatin in patients with advanced melanoma.

Before efficacy of combination therapy with sorafenib, paclitaxel and carboplatin could be evaluated, it is critical to first evaluate the safety of such a combination. Therefore, the primary objectives of the present study were to evaluate the safety and determine the maximum tolerated dose (MTD) of sorafenib administered in combination with paclitaxel and carboplatin in patients with advanced cancer. A preliminary analysis of the efficacy of treatment was also performed.

## PATIENTS AND METHODS

### *Patients*

Patients at least 18 years of age were enrolled in the study if they had histological or cytological documented cancer with an Eastern Cooperative Oncology Group (ECOG) performance status of 0 or 1, measurable disease, and life expectancy of at least 12 weeks. After establishing MTD, additional patients were enrolled to further evaluate pharmacokinetics and exclude a significant difference in exposure to carboplatin and paclitaxel with intervening sorafenib administration. Due to a change in the formulation of sorafenib from 50 mg tablets to 200 mg tablets an additional cohort of patients was enrolled to ensure that these tablets had similar pharmacokinetics. All patients were required to have adequate bone marrow, liver, and renal function.

Patients were excluded if they had clinically evident congestive heart failure greater than New York Heart Association Class 2, or had received anticancer chemotherapy or immunotherapy during the study or within 3 weeks of study entry, or mitomycin C or nitrosoureas within 6 weeks of study entry. Prior adjuvant chemotherapy was permitted. Patients could have had up to two prior chemotherapy regimens for metastatic disease (patients with three or more prior regimens could be enrolled after discussion with the sponsor). The use of concomitant investigational drugs was prohibited during or within 4 weeks of study entry. Use of ketoconazole, itraconazole, ritonavir, grapefruit products, and previous exposure to Ras pathway inhibitors were prohibited. Pregnant or nursing women were excluded. Women of childbearing potential were required to have a negative pregnancy test within 7 days of treatment initiation and to use adequate birth control measures during the study. Patients were excluded if they had a known or suspected allergy to any agent given in this study, had any condition that was unstable or could jeopardize their safety or ability to comply with study procedures, or could interfere with evaluation of the results.

All patients gave written, informed consent to participate in the study, which was conducted in accordance with the Declaration of Helsinki, the Investigational New Drug and Bioresearch Regulations of the US Food and Drug Administration, the Good Clinical Practice guidelines, and all applicable local laws and regulations. The study protocol and amendments were approved by each Institutional Review Board or Independent Ethics Committee.

### Study Design and Treatments

This was a two-center, open-label, non-placebo-controlled, dose-escalation trial. On Day 1, patients received paclitaxel by intravenous infusion over 3 hours followed by carboplatin infusion over 30 minutes. Oral sorafenib was administered twice daily (bid) on Days 2 through 19. No treatments were administered on Days 20 and 21. The starting doses were sorafenib 100 mg bid, paclitaxel 225 mg/m$^2$, and carboplatin corresponding to area under the curve of 6 (AUC6). Patients could continue to receive subsequent cycles until occurrence of unacceptable toxicity, tumor progression, or death. Sorafenib monotherapy could be continued after the sixth cycle of chemotherapy.

Dose-limiting toxicity (DLT) was defined as any one of the following: grade 4 neutropenia for at least 7 days; grade 4 neutropenia of any duration with sepsis or a fever >38.5°C; platelet count <25,000/μL; grade 3 or 4 non-hematologic toxicity (excluding nausea or vomiting not refractory to anti-emetics, and hypersensitivity reactions subsequently controlled by premedication); and any toxicity considered by the investigators to be related to the study drug

and of sufficient severity to warrant such description. Data from the first cycle of treatment were used to determine DLT. The next dose cohort was initiated if three patients enrolled at a given dose level reached Day 21 without experiencing DLT. If one patient experienced DLT, three additional patients were added to that cohort. The MTD was considered to have been exceeded if two or more patients in any cohort experienced DLT. Dose cohorts were: (i) paclitaxel 225 mg/$m^2$, carboplatin AUC6, and sorafenib 100 mg bid; (ii) paclitaxel 225 mg/$m^2$, carboplatin AUC6, and sorafenib 200 mg bid; and (iii) paclitaxel 225 mg/$m^2$, carboplatin AUC6, and sorafenib 400 mg bid.

**Study Assessments**

Before initiation of treatment, each patient was evaluated for medical history, physical examination, tumor measurement using computed tomography (CT) or magnetic resonance imaging (MRI) as appropriate, assessment of ECOG performance status, complete blood count with differential, serum chemistries, urinalysis and electrocardiogram. These assessments were also performed prior to the administration of each subsequent cycle. All observations pertinent to the safety of sorafenib were recorded, including results of physical examinations, vital signs, adverse events, concomitant medications, and laboratory tests. Complete blood counts were performed twice weekly, and serum chemistries were performed weekly during the first cycle, with more frequent monitoring in the event of severe myelosuppression. Patients were monitored every 3 weeks and as needed for adverse events. Tumor response and progression were assessed using the Response Evaluation Criteria In Solid Tumors (RECIST) with imaging studies performed after every two cycles of treatment. Blood samples were collected for analysis of sorafenib plasma concentrations on Days 2 and 19 of Cycle 1. In addition, samples were collected for analysis of platinum (total and free), paclitaxel and 6-OH-paclitaxel on Day 1 of Cycles 1 and 2. Calculated pharmacokinetic variables included the geometric means and percent coefficient of variance (% CV) of area under the concentration-time curve between time 0 and time *t* ($AUC_{0-t}$), maximum plasma concentration ($C_{max}$), the half-life of drug in plasma ($t_{1/2}$), and the time to maximum plasma concentration of drug ($t_{max}$).

Attempts were made to obtain tumor samples from all melanoma patients for *B-Raf* and *N-Ras* mutation analysis. To confirm the presence of melanoma in at least 70% of the area of interest, DNA was isolated from either paraffin-embedded tissue or frozen tissue using techniques described previously.[25] Genomic DNA was screened for *B-Raf* mutations by direct sequencing of the amplified region of interest using an ABI 3100 automated sequencer. One patient with metastatic melanoma consented to serial 4 mm punch biopsies of cutaneous lesions performed before and after 3 weeks of combined therapy with sorafenib, carboplatin, and paclitaxel. Samples were examined by hematoxylin and eosin staining to confirm that the sample was predominantly melanoma. Activation of the MAP kinase cascade was determined by immunoblotting.[26] Primary antibodies against phosphorylated and total MEK were obtained from Cell Signaling, Beverly, MA. Horseradish peroxidase-conjugated secondary antibodies were obtained from Jackson Immunoresearch (West Grove, PA).

**RESULTS**

***Patient Disposition and Baseline Characteristics***

Baseline characteristics of the thirty-nine patients enrolled in the dose-escalation phase of the trial are shown in Table 1. The primary diagnoses for these patients were melanoma (cutaneous, n = 23; ocular, 1), colon cancer (n = 4), NSCLC (n = 4), RCC (n = 2), or other tumors (n = 5). All patients received at least one dose of study medication and were included in both the safety and intent-to-treat analyses.

**Dose-limiting toxicity**

Two patients receiving sorafenib 100 mg bid had grade 3/4 toxicities during the first cycle of treatment (one grade 4 neutropenia lasting less than 7 days and one grade 3 rash considered related to sorafenib). Since the grade 3 rash was considered to be DLT, four additional patients were enrolled into this cohort. There was no DLT among the three patients receiving sorafenib 200 mg bid or among the first three patients receiving sorafenib 400 mg, 50 mg tablets. Of the additional nine patients enrolled into the third cohort, four (44%) experienced grade 3 or 4 toxicities during the first cycle of treatment of which only one (grade 3 hand-foot skin reaction (HFSR)) was considered DLT. A fourth cohort was added in which 17 patients received treatment with sorafenib 400 mg bid using 200 mg tablets, eight (47%) of whom had grade 3 toxicities during the first cycle of treatment, with five (29%) considered to be DLT (4 HFSR and 1 hypertension).

**Safety**

Treatment-related adverse events occurred in all patients. The most common individual drug-related adverse events are listed in Table 2. The most frequent adverse events were hematologic (95%), dermatologic (85%), fatigue

(59%), sensory neuropathy (59%), nausea (56%), and arthralgia (26%). Grade 4 neutropenia occurred 3 patients in the 100 mg bid cohort (43%), 1 patient in the 200 mg bid cohort (33%) and 20 patients in the 400 mg bid cohorts (69%). There were no cases of grade 4 thrombocytopenia. Rashes and plantar-palmar erythmea were reported in 71 %, 33% and 59% of the patients in the 100, 200 and 400 mg bid cohorts, respectively. Of the 29 patients who received sorafenib 400 mg bid, hand-foot skin reaction ≥grade 3 was reported in 5 (17%). However, there was no clear dose-related increment in these drug-related adverse events in this study.

**Efficacy**

There were 9 partial responses and 1 complete response by RECIST criteria, all in patients with melanoma (ORR of 26%). In addition, 19 patients had stable disease, 5 had progressive disease and 5 were removed from study before a response assessment. At the time of first assessment 29 patients (74%) were progression-free. The American Joint Committee on Cancer stage, demographic characteristics, and number of cycles of therapy administered in patients with melanoma are shown in Table 3. The complete response occurred in a male with metastatic melanoma to subcutaneous sites, lung and adrenal gland who had previously received temozolomide. The median duration of response was 467 days (range: 81 - 575 days) and did not differ significantly by dose level. Median progression-free survival was 307 days (range: 27 - 658 days) in the 24 patients with melanoma, compared with 104 days (range: 34 - 286 days) in patients with other tumor types (Fig. 1).

**Pharmacokinetics**

As expected from the long half-life (23.73 - 34.83 hours on Day 19), there was significant accumulation of sorafenib at all doses with mean values of $AUC_{0-12}$ increasing by 21.28, 15.66, and 32.61 mg·h/L from Day 2 to Day 19 of Cycle 1 for the 100, 200 and 400 mg doses, respectively, and reflected in the increases in $C_{max}$ (Table 4). These increases were accompanied by decreases in the time to reach maximum exposure for increasing doses of sorafenib. The pharmacokinetic profiles of total platinum and paclitaxel in Cycles 1 (without sorafenib) and 2 (after 18 days of sorafenib) were not notably different (Fig. 2). Mean total platinum $C_{max}$ and $AUC_{0-\infty}$ values (% CV) were 23.27 mg/L (37.33%) and 51.23 mg.h/L (18.15%) in Cycle 1 compared with 24.82 mg/L (26.39%) and 48.43 mg.h/L (13.57%) following sorafenib treatment, respectively. Mean paclitaxel and 6-OH-paclitaxel $C_{max}$ values were 9.57 mg/L (46.22%) and 1.15 mg/L (58.51%) in Cycle 1 compared with 9.93 mg/L (39.40%) and 1.28 mg/L (73.98%) following sorafenib at any dose, respectively. Mean paclitaxel and 6-OH-paclitaxel $AUC_{0-\infty}$ values were 31.49 mg.h/L (43.01%) and 3.22 mg.h/L (74.15%) in Cycle 1 compared with 29.20 mg.h/L (33.60%) and 3.81 mg.h/L (128.82%) following sorafenib at any dose, respectively.

**Correlative studies**

The $^{V600}$*B-Raf* mutation was detected in 11 of 17 melanoma patients tested (65%) with 6 of these patients (55%) achieving objective responses. One of two patients with a mutation in *N-Ras* had a partial response (50%). Three of four patients with wild-type *N-Ras* and *B-Raf* (75%) responded to treatment. Tumor samples from seven patients could not be obtained for analysis; none of these seven patients had an objective response. Melanoma samples from the patient who underwent serial biopsy of cutaneous lesions also harbored the $^{V600E}$*B-Raf* mutation. Immunoblot analyses revealed no change in MEK protein level following 3 weeks of treatment, but phosphorylated MEK was markedly decreased (Fig. 3).

**DISCUSSION**

This dose-escalation trial was designed to determine the safety and MTD of sorafenib in combination with carboplatin and paclitaxel to identify a regimen suitable for further Phase II/III evaluations. Although severe myelosuppression was observed in the present trial, the incidence is comparable to that reported with similar doses of carboplatin and paclitaxel.[27] Dermatologic adverse events (most frequently rash) were the principle toxicities associated with sorafenib treatment. Symptoms began within the first cycle and typically resolved by the end of the second cycle. There was no evidence of cumulative toxicity related to sorafenib. Neuropathy was typically mild and occurred with a frequency and severity that is expected with the use of carboplatin and paclitaxel at this dose and schedule.[27] Furthermore, the pharmacokinetics of sorafenib in this trial are consistent with previously reported results.[28] Importantly, administration of sorafenib 400 mg bid had no statistically significant effect on the pharmacokinetics of platinum or paclitaxel. This suggests that there may be no interaction between sorafenib and the combination of carboplatin and paclitaxel when sorafenib dosing is discontinued for at least 24 hours prior to administration of these chemotherapeutic agents. These data suggest that full doses of sorafenib, carboplatin and paclitaxel may be administered safely in this regimen.

Although a previous study of sorafenib monotherapy in patients with melanoma yielded a low response rate,

several clinical studies have demonstrated that sorafenib is effective when used in combination with other anticancer agents, including taxanes and platinum-containing drugs.[10-14] It was, therefore, reasonable to evaluate the efficacy of the combination of sorafenib, paclitaxel and carboplatin in patients with advanced melanoma. This combination yielded promising results, with one complete response and nine partial responses among the 39 patients, and a total of 24 patients (62%) with stable disease or objective responses. The complete response and all of the partial responses appear to be lasting since these patients remained free of progression for a median duration of at least 6 months beyond discontinuation of chemotherapy. Interestingly, all ten patients had been diagnosed with melanoma. Although this efficacy analysis is preliminary, in light of the very limited clinical success previously reported using a combination of carboplatin and paclitaxel to treat melanoma,[22-24] it is reasonable to infer that addition of sorafenib to the treatment regimen could have been the cause for the success observed in patients with melanoma. These results will need to be confirmed in larger, controlled studies.

A high frequency of activating mutations in *B-Raf* has been reported in melanoma cell lines and biopsies suggesting a causal relationship.[25,29] However, mutational analysis, available from 17 of the 24 melanoma patients, showed no correlation between clinical response and *B-Raf* status. It is possible that activated gene product, triggering a kinase cascade causing phosphorylation MEK and ERK, is more relevant to the cancer phenotype than merely the mutation of the gene. Data from the only patient with melanoma who was tested for MEK phosphorylation appear to support this hypothesis. This patient responded to therapy and demonstrated significant reduction in MEK phosphorylation in serial biopsies of the cutaneous lesions three weeks after initiation of treatment. However, this is only one patient and more data will need to be gathered before any conclusions can be drawn. The apparent discordance between *B-Raf* mutation status and response to treatment maybe due to the small sample size, suggesting a larger study could better elucidate the relationship between *B-Raf* and response to treatment. On the other hand, it may simply be that there is no predictive relationship between these two parameters.

In conclusion, this dose-escalation trial shows that sorafenib can be safely combined with carboplatin and paclitaxel. Based on the results of the present trial, the recommended Phase II doses for this combination of drugs for the treatment of melanoma are sorafenib 400 mg orally twice daily, carboplatin AUC 6, and paclitaxel 225 mg/m$^2$. This regimen is also well-suited for evaluation in NSCLC, ovarian cancer, and head and neck cancer, as carboplatin and paclitaxel are standard treatments for these tumor types. The responses seen in patients with melanoma warrant further investigation in a larger population of patients and may help clarify the relationship between response to treatment and *B-Raf* mutation status.

**Table 1.** Baseline Patient Characteristics

| Characteristic | Number of Patients | % |
|---|---|---|
| Total patients | 39 | 100 |
| Gender | | |
| Male | 26 | 67 |
| Female | 13 | 33 |
| Ethnicity | | |
| Caucasian | 37 | 95 |
| Asian | 1 | 3 |
| Hispanic | 1 | 3 |
| Age, years | | |
| Mean (SD) | 51.8 (12.9) | |
| Median (range) | 51 (25-75) | |
| Primary carcinoma | | |
| Malignant melanoma | 34 | 62 |
| Colon | 4 | 10 |
| NSCLC | 4 | 10 |
| Renal | 2 | 5 |
| Other* | 5 | 13 |
| ECOG performance status | | |
| 0 | 22 | 56 |
| 1 | 17 | 44 |

(continued)

| Characteristic | Number of Patients | % |
|---|---|---|
| Prior systemic regimens | | |
| 0 | 8 | 21 |
| 1 | 9 | 23 |
| 2 | 14 | 36 |
| 3 | 5 | 13 |
| ≥4 | 3 | 8 |
| Prior treatment | | |
| Taxane | 3 | 8 |
| Radiotherapy | 12 | 31 |
| Non-diagnostic surgery | 29 | 74 |

*Other tumors included mucinous adenocarcinoma of appenditial wall, adenocarcinoma of distal esophagus, neuroendocrine tumor of esophagus, Ewing's sarcoma of right rib and lung, and basal cell carcinoma of right shoulder.
Abbreviations: ECOG, Eastern Cooperative Oncology Group; NSCLC, non-small-cell lung cancer; SD, standard deviation.

**Table 2.** Common Treatment-Related Adverse Events

| Adverse Event | 100 mg bid (N = 7) | | | | | | 200 mg bid (N = 3) | | | | | | 400 mg* bid (N = 29) | | | | | |
|---|---|---|---|---|---|---|---|---|---|---|---|---|---|---|---|---|---|---|
| | NCI-CTC Grade, n | | | | | | NCI-CTC Grade, n | | | | | | NCI-CTC Grade, n | | | | | |
| | **1** | **2** | **3** | **4** | **5** | **All** | **1** | **2** | **3** | **4** | **5** | **All** | **1** | **2** | **3** | **4** | **5** | **All** |
| Hematologic | | | | | | | | | | | | | | | | | | |
| *Neutrophils/granulocytes* | 0 | 1 | 3 | | 0 | 7 | 0 | 0 | 2 | 1 | 0 | 3 | 0 | 2 | 2 | 20 | 0 | 24 |
| *Leukocytes* | 0 | 0 | 1 | 1 | 0 | 2 | 0 | 1 | 0 | 0 | 0 | 1 | 0 | 0 | 2 | 0 | 0 | 2 |
| *Platelets* | 1 | 0 | 1 | 0 | 0 | 2 | 0 | 0 | 3 | 0 | 0 | 3 | 2 | 5 | 12 | 0 | 0 | 19 |
| *Hemoglobin* | 0 | 0 | 0 | 0 | 0 | 0 | 0 | 1 | 0 | 0 | 0 | 1 | 1 | 9 | 5 | 1 | 0 | 16 |
| *Any* | 0 | 0 | 4 | 3 | 0 | 7 | 0 | 0 | 2 | 1 | 0 | 3 | 0 | 4 | 3 | 20 | 0 | 27 |
| Dermatologic | | | | | | | | | | | | | | | | | | |
| *Alopecia* | 0 | 5 | 0 | 0 | 0 | 5 | 0 | 1 | 0 | 0 | 0 | 1 | 2 | 10 | 0 | 0 | 0 | 12 |
| *Rash/desquamation* | 1 | 3 | 1 | 0 | 0 | 5 | 1 | 0 | 0 | 0 | 0 | 1 | 7 | 7 | 3 | 0 | 0 | 17 |
| *Any* | 0 | 4 | 1 | 0 | 1 | 6 | 1 | 1 | 0 | 0 | 0 | 2 | 5 | 14 | 5 | 0 | 1 | 25 |
| Other | | | | | | | | | | | | | | | | | | |
| *Fatigue* | 2 | 3 | 1 | 1 | 0 | 7 | 1 | 0 | 0 | 0 | 0 | 1 | 9 | 6 | 0 | 0 | 0 | 15 |
| *ausea* | 3 | 3 | 0 | 0 | 0 | 6 | 2 | 0 | 0 | 0 | 0 | 2 | 5 | 5 | 4 | 0 | 0 | 14 |
| *Constipation* | 1 | 4 | 0 | 0 | 0 | 5 | 1 | 0 | 0 | 0 | 0 | 1 | 3 | 3 | 0 | 0 | 0 | 6 |
| *Sensory neuropathy* | 3 | 2 | 1 | 0 | 0 | 6 | 1 | 1 | 0 | 0 | 0 | 2 | 10 | 4 | 1 | 0 | 0 | 15 |
| *rthralgia (Joint pain)* | 1 | 1 | 0 | 0 | 0 | 2 | 1 | 0 | 0 | 0 | 0 | 1 | 4 | 3 | 0 | 0 | 0 | 7 |
| **Any event** | **0** | **0** | **2** | **4** | **1** | **7** | **0** | **0** | **1** | **2** | **0** | **3** | **0** | **1** | **6** | **20** | **2** | **29** |

*Combined incidence of adverse events for the 4 x 50 mg and 2 x 200 mg tablet regimens. Abbreviation: NCI-CTC, National Cancer Institute-Common Toxicity Criteria.

**Table 3.** Characteristics and Tumor Responses of Patients With Melanoma

| Gender | Age (years) | ECOG Status | AJCC M Stage | Elevated LDH | Disease Sites | Prior Therapy | Mutations | Response | Treatment Duration (Months) |
|---|---|---|---|---|---|---|---|---|---|
| M | 50 | 0 | C | No | Liver, lung | 0 | *B-Raf* | PD | >1 |
| M | 26 | 0 | c | No | Adrenal, lymph node | 1 | *B-Raf* | PR | 29 |
| F | 50 | 0 | B | No | Lung, skin | 0 | *B-Raf* | SD | 3 |
| F | 52 | 0 | B | No | Lung, skin | 2 | *B-Raf* | PR | 10 |
| M | 34 | 1 | C | Yes | Liver | 1 | *N-Ras* | SD | 5 |
| F | 53 | 1 | C | Yes | Liver | 2 | *B-Raf* | SD | >1 |
| M | 69 | 1 | C | Yes | Lymph node | 1 | N/A | NE | <1 |
| F | 39 | 0 | C | Yes | Abdomen, adrenal, bowel, skin | 0 | WT | PR | >12 |
| M | 53 | 0 | C | Yes | Liver | 1 | *B-Raf* | PR | 17 |
| M | 46 | 0 | A | No | Lymph node | 1 | *B-Raf* | PR | 15 |
| M | 33 | 0 | C | No | Lung, pelvis | 1 | *B-Raf* | SD | >2 |
| M | 61 | 0 | B | No | Lung, skin | 1 | *N-Ras* | PR | 10 |
| M | 47 | 0 | C | No | Adrenal, lung, skin | 1 | *B-Raf* | CR | 21 |
| F | 51 | 0 | c | Yes | Liver, lung | 1 | N/A | SD | >3 |
| F | 51 | 0 | B | No | Lung, skin | 0 | *B-Raf* | SD | >12 |
| F | 75 | 1 | A | No | Skin | 1 | N/A | SD | >11 |
| F | 49 | 1 | C | Yes | Lymph node, pancreas, spleen | 2 | *B-Raf* | PR | 23 |
| M | 54 | 1 | C | Yes | Kidney, liver, lymph node, vertebrae | 1 | N/A | SD | 4 |

(continued)

| Gender | Age (years) | ECOG Status | AJCC M Stage | Elevated LDH | Disease Sites | Prior Therapy | Mutations | Response | Treatment Duration (Months) |
|---|---|---|---|---|---|---|---|---|---|
| M | 33 | 0 | B | No | Lung | 0 | WT | PR | 10 |
| F | 52 | 0 | A | No | Lymph node | 0 | WT | PR | >15 |
| F | 45 | 0 | C | Yes | Adrenal, liver, lung, skin, uterus | 0 | N/A | SD | 6 |
| F | 55 | 1 | C | Yes | Liver, pancreas, skin | 3 | WT | SD | 6 |
| M* | 37 | 1 | C | Yes | Bone, liver | N/A | N/A | PD | >1 |
| M | 56 | 0 | C | Yes | Liver, lung, lymph node | N/A | N/A | SD | 3 |

*Patient had ocular melanoma.
Abbreviations: AJCC M, American Joint Committee on Cancer metastasis; CR, complete response; ECOG, Eastern Cooperative Oncology Group; F, female; M, male; N/A, not available; NE, not evaluable; WT, wild type; PD, progressive disease; PR, partial response; SD, stable disease.

**Table 4.** Comparison of Sorafenib Plasma Pharmacokinetics on the Second Day of Cycle 1 and After 18 Days of Dosing (i.e. Day 19, Cycle 1)

| Sorafenib Dose | Patient Number | | $C_{max}$ (mg/L) Mean (%CV) | | $t_{½}$ (h) Mean (%CV) | | $AUC_{0-12}$ (mg.h/L) Mean (%CV) | | Calculated Clearance (L/h) | |
|---|---|---|---|---|---|---|---|---|---|---|
| | Day 2 | Day 19 | Day 2 | Day 19 | Day 2 | Day 19 | Day 2 | Day 19 | Day 2 | Day 19 |
| 100 mg bid | 7 | 4 | 2.09 (30.51) | 3.72 (27.50) | ND | 26.10 (32.9) | 8.38 (42.34) | 29.66 (24.70) | 11.93 | 3.37 |
| 200 mg bid | 3 | 2 | 2.33 (50.75) | 3.11 (15.25) | ND | 23.73 (10.67) | 9.61 (48.10) | 25.27 (19.37) | 20.81 | 7.91 |
| 400 mg bid | 25 | 19 | 3.95 (62.29) | 6.75 (41.39) | ND | 34.83 (44.85) | 23.28 (84.99) | 55.89 (42.60) | 17.18 | 7.16 |

Abbreviations: $AUC_{0-12}$, area under the concentration-time curve between time 0 and 12 hours; $C_{max}$, maximum plasma concentration; CV, coefficient of variance; $t_{½}$, the half-life of drug in plasma.

## Figure Legends

**Fig. 1.** Progression-free survival in patients with melanoma or other tumors Abbreviation: Progression-free survival (PFS).

**Fig. 2A.** Pharmacokinetics of platinum before and after sorafenib treatment Means were calculated when at least two thirds of data were above the limit of quantitation (LOQ) of 50 μg/L. In calculation of mean values, concentrations below LOQ of 50 μg/L were replaced by half of LOQ.

**Fig. 2B.** Pharmacokinetics of paclitaxel before and after sorafenib treatment Means were calculated when at least two thirds of data were above the limit of quantitation (LOQ) of 5 μg/L. In calculation of mean values, concentrations below LOQ of 5 μg/L were replaced by half of LOQ.

**Fig. 3.** Serial biopsies from responding patient with BRAF mutation

## REFERENCES

1. Wilhelm SM, Carter C, Tang L, et al: BAY 43-9006 exhibits broad spectrum oral antitumor activity and targets the RAF/MEK/ERK pathway and receptor tyrosine kinases involved in tumor progression and angiogenesis. Cancer Res 64:7099-109, 2004
2. Carlomagno F, Anaganti S, Guida T, et al: BAY 43-9006 inhibition of oncogenic RET mutants. J Natl Cancer Inst 98:326-34, 2006
3. Levy JB, Pauloski N, Braun D, et al: Analysis of transcription and protein expression changes in the 786-O human renal cell carcinoma tumor xenograft model in response to treatment with the multi-kinase inhibitor sorafenib (BAY 43-9006). AACR Meeting Abstracts 2006:213-d-214, 2006 (abstr)
4. Sharma A, Trivedi NR, Zimmerman MA, et al: Mutant V599EB-Raf Regulates Growth and Vascular Development of Malignant Melanoma Tumors. Cancer Res 65:2412-2421, 2005
5. Strumberg D, Richly H, Hilger RA, et al: Phase I clinical and pharmacokinetic study of the Novel Raf kinase and vascular endothelial growth factor receptor inhibitor BAY 43-9006 in patients with advanced refractory solid tumors. J Clin Oncol 23:965-72, 2005
6. Ratain MJ, Eisen T, Stadler WM, et al: Phase II placebo-controlled randomized discontinuation trial of sorafenib in patients with metastatic renal cell carcinoma. J Clin Oncol 24:2505-12, 2006
7. Escudier B, Szczylik C, Eisen T, et al: Randomized phase III trial of the Raf kinase and VEGFR inhibitor sorafenib (BAY 43-9006) in patients with advanced renal cell carcinoma (RCC). J Clin Oncol (Meeting Abstracts) 23:LBA4510-,

2005 (abstr)
8. Heim M, Sharifi M, Hilger RA, et al: Antitumor effect and potentiation or reduction in cytotoxic drug activity in human colon carcinoma cells by the Raf kinase inhibitor (RKI) BAY 43-9006. Int J Clin Pharmacol Ther 41:616-7, 2003
9. Carter CA, Chen C, Brink C, et al: Sorafenib is efficacious and tolerated in combination with cytotoxic or cytostatic agents in preclinical models of human non-small cell lung carcinoma. Cancer Chemother Pharmacol, 2006
10. Mross K, Steinbild S, Baas F, et al: Drug-drug interaction pharmacokinetic study with the Raf kinase inhibitor (RKI) BAY 43-9006 administered in combination with irinotecan (CPT-11) in patients with solid tumors. Int J Clin Pharmacol Ther 41:618-9, 2003
11. Kupsch P, Henning BF, Passarge K, et al: Results of a phase I trial of sorafenib (BAY 43-9006) in combination with oxaliplatin in patients with refractory solid tumors, including colorectal cancer. Clin Colorectal Cancer 5:188-96, 2005
12. Richly H, Henning BF, Kupsch P, et al: Results of a Phase I trial of sorafenib (BAY 43-9006) in combination with doxorubicin in patients with refractory solid tumors. Ann Oncol 17:866-73, 2006
13. Siu LL, Awada A, Takimoto CH, et al: Phase I trial of sorafenib and gemcitabine in advanced solid tumors with an expanded cohort in advanced pancreatic cancer. Clin Cancer Res 12:144-51, 2006
14. Richly H, Kupsch P, Passage K, et al: Results of a phase I trial of BAY 43-9006 in combination with doxorubicin in patients with primary hepatic cancer. Int J Clin Pharmacol Ther 42:650-1, 2004
15. Ahmad T, Eisen T: Kinase inhibition with BAY 43-9006 in renal cell carcinoma. Clin Cancer Res 10:6388S-92S, 2004
16. Wiernik PH, Schwartz EL, Einzig A, et al: Phase I trial of taxol given as a 24-hour infusion every 21 days: responses observed in metastatic melanoma. J Clin Oncol 5:1232-9, 1987
17. Legha SS, Ring S, Papadopoulos N, et al: A phase II trial of taxol in metastatic melanoma. Cancer 65:2478-81, 1990
18. Einzig AI, Hochster H, Wiernik PH, et al: A phase II study of taxol in patients with malignant melanoma. Invest New Drugs 9:59-64, 1991
19. Aamdal S, Wolff I, Kaplan S, et al: Docetaxel (Taxotere) in advanced malignant melanoma: a phase II study of the EORTC Early Clinical Trials Group. Eur J Cancer 30A:1061-4, 1994
20. Bedikian AY, Weiss GR, Legha SS, et al: Phase II trial of docetaxel in patients with advanced cutaneous malignant melanoma previously untreated with chemotherapy. J Clin Oncol 13:2895-9, 1995
21. Einzig AI, Schuchter LM, Recio A, et al: Phase II trial of docetaxel (Taxotere) in patients with metastatic melanoma previously untreated with cytotoxic chemotherapy. Med Oncol 13:111-7,1996
22. Zimpfer-Rechner C, Hofmann U, Figl R, et al: Randomized phase II study of weekly paclitaxel versus paclitaxel and carboplatin as second-line therapy in disseminated melanoma: a multicentre trial of the Dermatologic Co-operative Oncology Group (DeCOG). Melanoma Res 13:531-6, 2003
23. Hodi FS, Soiffer RJ, Clark J, et al: Phase II study of paclitaxel and carboplatin for malignant melanoma. Am J Clin Oncol 25:283-6, 2002
24. Rao RD, Holtan SG, Ingle JN, et al: Combination of paclitaxel and carboplatin as second-line therapy for patients with metastatic melanoma. Cancer 106:375-82, 2006
25. Brose MS, Volpe P, Feldman M, et al: BRAF and RAS mutations in human lung cancer and melanoma. Cancer Res 62:6997-7000, 2002
26. Kent LL, Hull-Campbell NE, Lau T, et al: Characterization of novel inhibitors of cyclin-dependent kinases. Biochem Biophys Res Commun 260:768-74,1999
27. Schiller JH, Harrington D, Belani CP, et al: Comparison of four chemotherapy regimens for advanced non-small-cell lung cancer. N Engl J Med 346:92-8, 2002
28. Strumberg D, Voliotis D, Moeller JG, et al: Results of phase I pharmacokinetic and pharmacodynamic studies of the Raf kinase inhibitor BAY 43-9006 in patients with solid tumors. Int J Clin Pharmacol Ther 40:580-1, 2002
29. Davies H, Bignell GR, Cox C, et al: Mutations of the BRAF gene in human cancer. Nature 417:949-54, 2002

## Claims

**1.** A combination which is therapeutically effective for the treatment of mammalian cancer comprising:

**(1)** at least one substituted-diaryl urea of Formula I , or a polymorph, solvate, hydrate, metabolite, prodrug, diastereoisomer or pharmaceutically acceptable salt thereof,

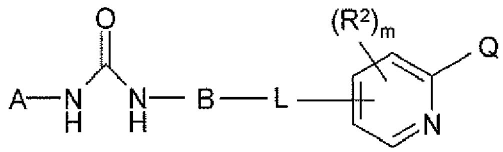

wherein

Q is -C(O)$R_x$
$R_x$ is hydroxy, $C_{1-4}$ alkyl, $C_{1-4}$ alkoxy or $NR_aR_b$,

$R_a$ and $R_b$ are independently:

**a)** hydrogen;
**b)** $C_{1-4}$ alkyl, optionally substituted by

- hydroxy,
- $C_{1-4}$ alkoxy,
- a heteroaryl group which is pyridine, pyrimidine, pyridazine, isoquinoline or quinoline;
- a heterocyclic group which is piperazine or piperidine;
- amino(-$NH_2$) optionally substituted by one or two $C_{1-4}$ alkyl groups, or
- phenyl, or

**c)** phenyl, optionally substituted with

- halogen, or
- amino(-$NH_2$) optionally substituted by one or two $C_{1-4}$ alkyl groups,

A is 3-tert butyl phenyl, 5-tert butyl-2-methoxyphenyl, 5-(trifluoromethyl)-2 phenyl, 3-(trifluoromethyl)-4-chlorophenyl, -3-trifluoromethyl)-4-bromophenyl or 5-(trifluoromethyl)-4-chloro-2 methoxyphenyl;
B is

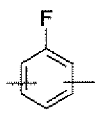

L is a bridging group which is -S- or -O-;
m is 0,1,2 or 3,
p is 0, 1, 2, 3, or 4,
n is0,1,2,3,4,5or6,

each $R^1$ is independently chlorine or fluorine;
each $R^2$ is methyl or trifluoromethyl
each $R^3$ is independently trifluoromethyl, methyl, ethyl, propyl, butyl, isopropyl, tert-butyl, chlorine, fluorine, bromine, cyano, methoxy, acetyl, trifluoromethanesulfonyl, trifluoromethoxy, or trifluoromethylthio;
and
$R^4$ and $R^5$ are independently hydrogen, $C_{1-6}$ alkyl, and up to per-halogenated $C_{1-6}$ alkyl;
**(2)** at least one taxane, or a stereoisomer thereof, a precursor (prodrug) thereof or a pharmaceutically acceptable salt thereof, and
**(3)** at least one platinum complex antineoplastic nucleic acid binding agent or a stereoisomer thereof, a precursor (prodrug) thereof or a pharmaceutically acceptable salt thereof.

**2.** A combination as in claim 1 adapted for administration of components (1), (2) and (3) to a patient in need thereof either

(a) in the same formulation,
(b) in separate formulations using the same administration route, or
(c) in separate formulations using different administration routes.

**3.** A combination as in claim 2 adapted for administration of components (1), (2) and (3) to a patient in need thereof by oral delivery and/or by intravenous injection or infusion or an intramuscular, subcutaneous or parenteral route of administration.

**4.** A combination as in claim 1 adapted for administration of the following agents to a patient in need thereof:

**(1)** at least one substituted-diaryl urea, or a polymorph, solvate, hydrate, metabolite, prodrug, diastereoisomer or pharmaceutically acceptable salt thereof, once or more per day for up to 28 consecutive days, with either concurrent or intermittent administration of
**(2)** at least one taxane, or a stereoisomer thereof, a precursor (prodrug) thereof or a pharmaceutically acceptable salt thereof, and
**(3)** at least one platinum complex antineoplastic nucleic acid binding agent or a stereoisomer thereof, a precursor (prodrug) thereof or a pharmaceutically acceptable salt thereof, over the same period.

**5.** A combination as in claim 1 adapted for administration of the following agents to a patient in need thereof:

**(1)** at least one substituted diaryl urea compound, or a polymorph, solvate, hydrate, metabolite, prodrug, diastereoisomer or pharmaceutically acceptable salt thereof, at a dosage within the range from about 0.1 to about 300 mg/kg of total body weight,
**(2)** at least one taxane or a stereoisomer thereof, a precursor (prodrug) thereof or a pharmaceutically acceptable salt thereof, at a dosage range from about 10-300 mg/$m^2$ of patient surface area, and
**(3)** at least one platinum complex or a stereoisomer thereof, a precursor (prodrug) thereof or a pharmaceutically acceptable salt thereof, within the range from about 100-500 mg/$m^2$ of patient surface area.

**6.** A combination of claim 1, where the substituted-diaryl urea of Formula I has the structure of Formula X

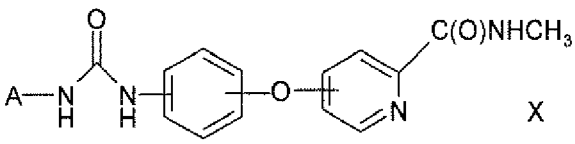
X

where A is as defined for Formula I in claim 1.

**7.** A kit for treating non-small cell lung cancer (NSCLC), colon, prostate, leukemia, hepatocellular, renal, head and neck, glioma, lung, pancreatic, and ovarian cancers comprising:

**(1)** at least one substituted-diaryl urea of Formula I, or a polymorph, solvate, hydrate, metabolite, prodrug, diastereoisomer or pharmaceutically acceptable salt thereof,

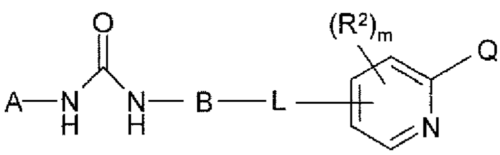

wherein

Q is -C(O)$R)_x$
$R_x$ is hydroxy, $C_{1-4}$ alkyl, $C_{1-4}$ alkoxy or $NR_aR_b$,

$R_a$ and $R_b$ are independently:

**a)** hydrogen;
**b)** C1-4 alkyl, optionally substituted by

- hydroxy,
- $C_{1-4}$ alkoxy,
- a heteroaryl group which is pyridine, pyrimidine, pyridazine, isoquinoline or quinoline;
- a heterocyclic group which is piperazine or piperidine,
- amino($-NH_2$) optionally substituted by one or two $C_{1-4}$ alkyl groups, or
- phenyl, or

**c)** phenyl optionally substituted with

- halogen, or
- amino ($-NH_2$) optionally substituted by one or two $C_{1-4}$ alkyl groups, A is 3-tert butyl phenyl, 5-tert butyl-2-methoxyphenyl,
5-(trifluoromethyl)-2 phenyl, 3-(trifluoromethyl)-4-chlorophenyl, -3-trifluoromethyl)-4-bromophenyl or 5-(trifluoromethyl)-4-chloro-2 methoxyphenyl;

B is

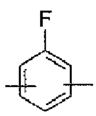

L is a bridging group which is -S- or -O-;
m is 0,1,2 or 3,
p is 0,1,2, 3, or 4,
n is 0, 1, 2, 3, 4, 5 or 6,

each $R^1$ is independently chlorine or fluorine;
each $R^2$ is methyl or trifluoromethyl
each $R^3$ is independently trifluoromethyl, methyl, ethyl, propyl, butyl, isopropyl, tert-butyl, chlorine, fluorine, bromine, cyano, methoxy, acetyl, trifluoromethanesulfonyl, trifluoromethoxy, or trifluoromethylthio;
and
$R^4$ and $R^5$ are independently hydrogen, $C_{1-6}$ alkyl, and up to per-halogenated $C_{1-6}$ alkyl;

**(2)** at least one taxane, or a stereoisomer thereof, a precursor (prodrug) thereof or a pharmaceutically acceptable salt thereof, and
**(3)** at least one platinum complex antineoplastic nucleic acid binding agent or a stereoisomer thereof, a precursor (prodrug) thereof or a pharmaceutically acceptable salt thereof.

**8.** A kit as in claim 7 wherein

**(1)** the dosage of the substituted-diaryl urea or a polymorph, solvate, hydrate, metabolite, prodrug, or pharmaceutically acceptable salt thereof is 50 to 600 mg,
**(2)** the dosage of the taxane or stereoisomer, precursor (prodrug) or pharmaceutically acceptable salt thereof is from about 30 to 200 mg/m2 and
**(3)** the dosage for the platinum complex antineoplastic nucleic acid binding agent or stereoisomer, precursor (prodrug) or pharmaceutically acceptable salt thereof is 100 to 500 mg/m2.

**9.** A pharmaceutical composition for the treatment of mammalian cancer comprising a combination as in claim 1, 2, 3, 4, 5 or 6, and at least one pharmaceutically acceptable carrier.

**10.** A pharmaceutical composition for the treatment of non-small cell lung cancer comprising a combination as in claim 1, 2, 3, 4, 5 or 6 and at least one pharmaceutically acceptable carrier.

**11.** Use of a combination of claim 1, 2, 3, 4, 5 or 6 for the manufacture of a medicament for the treatment of mammalian cancer.

**12.** Use of a combination of claim 1, 2, 3, 4, 5 or 6 in the manufacture of a medicament for the treatment of non-small cell lung cancer.

**13.** A combination as in claim 1, 2, 3 or 4 wherein the mammalian cancer to be treated is non-small cell lung cancer and cancer of the colon, pancreas, prostate, liver, kidney, lung, head and neck, pancreas and ovary.

**14.** A combination as in claim 1, 2, 3 or 4, wherein the mammalian cancer to be treated is

**(a)** small-cell lung cancer, non-small-cell lung cancer, bronchial adenoma, pleuropulmonary blastoma;
**(b)** brain stem and hypothalamic glioma, cerebellar and cerebral astrocytoma, medulloblastoma, ependymoma, and neuroectodermal, pineal tumor;
**(c)** endometrial cancer, cervical cancer, ovarian cancer, vaginal cancer, vulvar cancer, sarcoma of the uterus bone;
**(d)** anal cancer, colon cancer, colorectal cancer, esophageal cancer, gallbladder cancer, gastric cancer, pancreatic cancer, rectal cancer, small intestine cancer, salivary gland cancer;
**(e)** hepatocellular carcinoma, cholangiocarcinoma, mixed hepatocellular cholangiocarcinoma;
**(f)** bladder cancer, penile cancer, kidney cancer, kidney neoplasm, renal pelvis cancer, ureter cancer, urethral cancer;
**(g)** squamous cell carcinoma, Kaposi's sarcoma, malignant melanoma, Merkel cell skin cancer, non-melanoma skin cancer;
**(h)** intraocular melanoma, retinoblastoma;
**(i)** laryngeal, hypopharyngeal, nasopharyngeal, oropharyngeal cancers, lip and oral cavity cancer;
**(j)** testicular cancer, prostate cancer;
**(k)** AIDS-related lymphoma, non-Hodgkin's lymphoma, cutaneous T-cell lymphoma, Hodgkin's disease, lymphoma of the central nervous system;
**(l)** glioblastoma, hematologic malignancies, Lhermitte-Duclose disease, malignant glioma, multiple myeloma, myeloid metaplasia, myeloplastic syndromes;
**(m)** sarcoma of the soft tissue, osteosarcoma, malignant fibrous histiocytoma, lymphosarcoma, rhabdomyosarcoma, leimyosarcoma, liposarcoma;
**(n)** acute myeloid leukemia, acute lymphoblastic leukemia, chronic lymphocytic leukemia, chronic myelogenous leukemia, and hairy cell leukemia.

**15.** A pharmaceutical composition of claim 9 wherein the mammalian cancer to be treated is non-small cell lung cancer and cancer of the colon, pancreas, prostate, liver, kidney, lung, head and neck, pancreas and ovary.

**16.** A pharmaceutical composition of claim 9 wherein the mammalian cancer to be treated is

**(a)** small-cell lung cancer, non-small-cell lung cancer, bronchial adenoma, pleuropulmonary blastoma;
**(b)** brain stem and hypophtalmic glioma, cerebellar and cerebral astrocytoma, medulloblastoma, ependymoma, and neuroectodermal, pineal tumor;
**(c)** endometrial cancer, cervical cancer, ovarian cancer, vaginal cancer, vulvar cancer, sarcoma of the uterus bone;
**(d)** anal cancer, colon cancer, colorectal cancer, esophageal cancer, gallbladder cancer, gastric cancer, pancreatic cancer, rectal cancer, small intestine cancer, salivary gland cancer;
**(e)** hepatocellular carcinoma, cholangiocarcinoma, mixed hepatocellular cholangiocarcinoma;
**(f)** bladder cancer, penile cancer, kidney cancer, kidney neoplasm, renal pelvis cancer, ureter cancer, urethral cancer;
**(g)** squamous cell carcinoma, Kaposi's sarcoma, malignant melanoma, Merkel cell skin cancer, non-melanoma skin cancer;

**(h)** intraocular melanoma, retinoblastoma;
**(i)** laryngeal, hypopharyngeal, nasopharyngeal, oropharyngeal cancers, lip and oral cavity cancer;
**(j)** testicular cancer, prostate cancer;
**(k)** AIDS-related lymphoma, non-Hodgkin's lymphoma, cutaneous T-cell lymphoma, Hodgkin's disease, lymphoma of the central nervous system;
**(l)** glioblastoma, hematologic malignancies, Lhermitte-Duclose disease, malignant glioma, multiple myeloma, myeloid metaplasia, myeloplastic syndromes;
**(m)** sarcoma of the soft tissue, osteosarcoma, malignant fibrous histiocytoma, lymphosarcoma, rhabdomyosarcoma, leimyosarcoma, liposarcoma;
**(n)** acute myeloid leukemia, acute lymphoblastic leukemia, chronic lymphocytic leukemia, chronic myelogenous leukemia, and hairy cell leukemia.

Fig. 1.

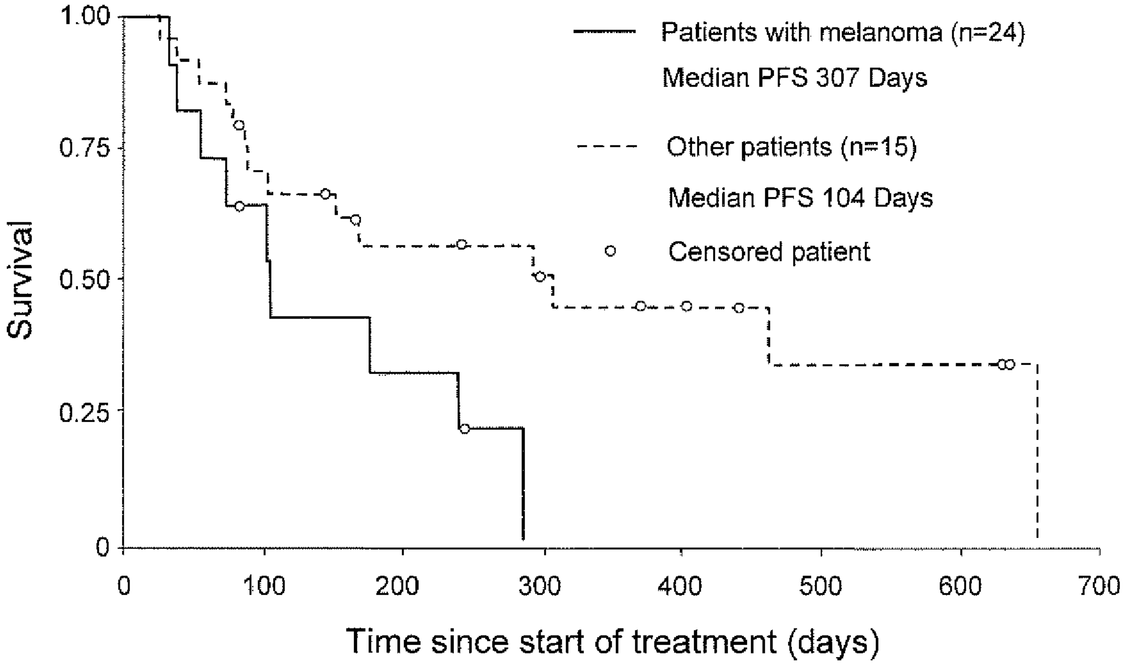
1.00
0.75
0.50
0.25
0
Survival
0
100
200
300
400
500
600
700
Time since start of treatment (days)
Patients with melanoma (n=24)
Median PFS 307 Days
Other patients (n=15)
Median PFS 104 Days
Censored patient

Fig. 2A.

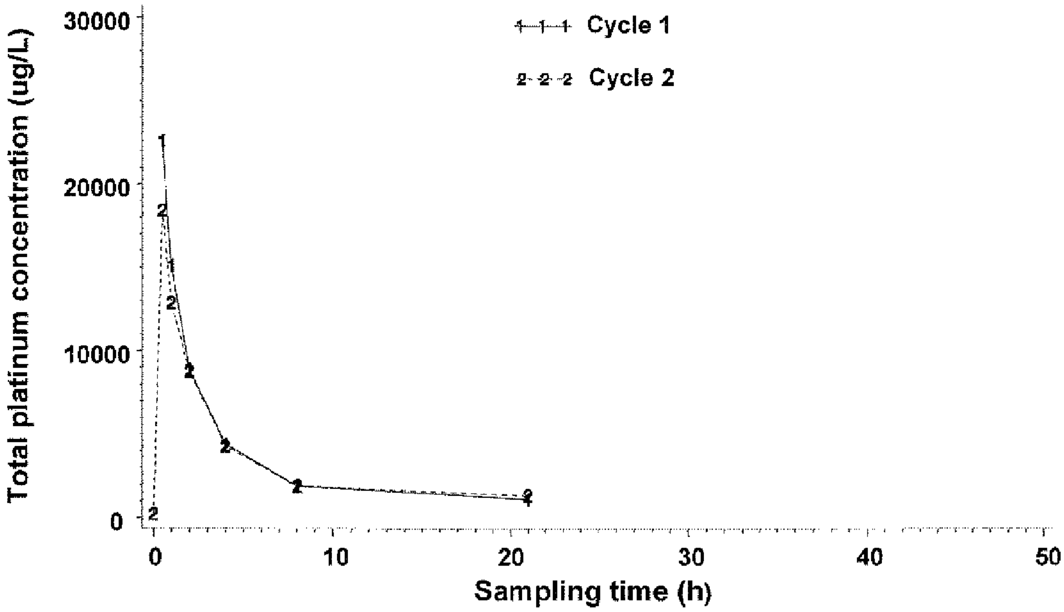
Total platinum concentration (ug/L)
30000
20000
10000
0
Cycle 1
Cycle 2
0
10
20
30
40
50
Sampling time (h)

Fig. 2B.

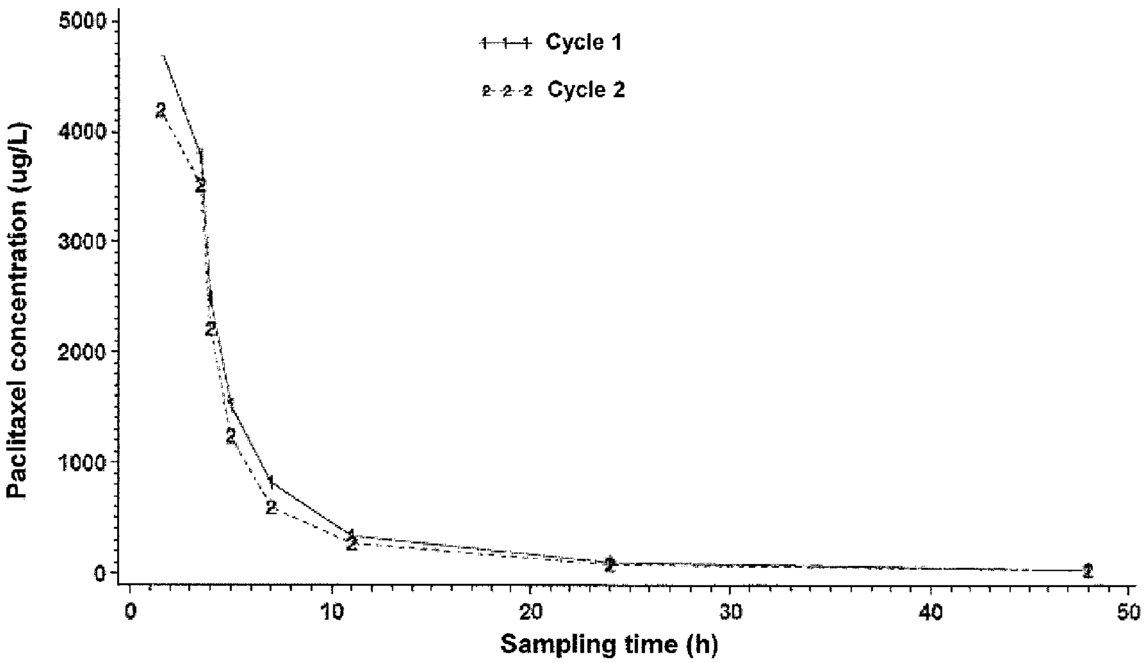
Cycle 1
Cycle 2
5000
4000
3000
2000
1000
0
Paclitaxel concentration (ug/L)
0
10
20
30
40
50
Sampling time (h)

Fig. 3.

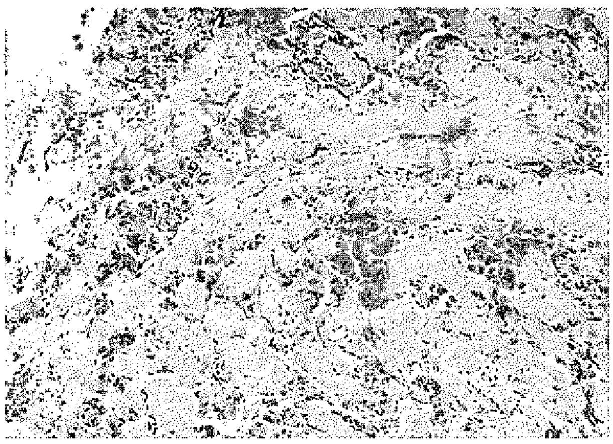

H&E stain of biopsy taken after 3 weeks

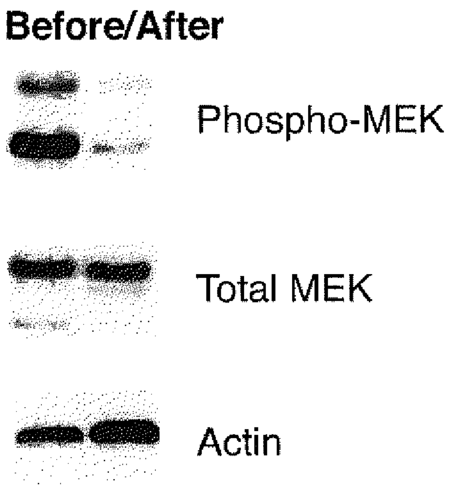

Europäisches Patentamt
European Patent Office
Office européen des brevets

# EUROPEAN SEARCH REPORT

Application Number
EP 10 18 5819

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | F.T. FLAHERTY ET AL.: "Phase I/II trial of BAY 43-9006, Carboplatin (C) and Paclitaxel (P) demonstrates preliminary antitumor activity in the expansion cohort of patients with metastatic melanoma", JOURNAL OF CLINICAL ONCOLOGY, vol. 22, no. 14S, 15 July 2004 (2004-07-15), page 7507, XP002466740, * abstract * | 1-34 | INV. A61K31/435 A61K31/337 A61K31/555 A61P35/00 A61P35/04 |
| X | SWART GUIDO W M: "International Melanoma Research Congress--Foundation for Melanoma Research. 21-24 June 2003, Philadelphia, PA, USA.", IDRUGS : THE INVESTIGATIONAL DRUGS JOURNAL AUG 2003, vol. 6, no. 8, August 2003 (2003-08), pages 752-754, XP002402150, ISSN: 1369-7056 * See page 754, column 2: therapy protocol for treating melanoma comprising administration of Bay 49-9006 (Sorafenil), carboplatinum, paclitaxel * | 1-34 | TECHNICAL FIELDS SEARCHED (IPC) A61K |
| X | WO 2006/026501 A (BAYER PHARMACEUTICALS CORP [US]; SCHUECKLER FRITZ) 9 March 2006 (2006-03-09) * See page 11, line 2, combinations with carboplatinum and cisplatinum * * See page 11, line 18: combination with paclitaxel * * claims 29-33 * | 1-34 | |

-/--

The present search report has been drawn up for all claims

1

| Place of search | Date of completion of the search | Examiner |
|---|---|---|
| Munich | 16 May 2011 | Veronese, Andrea |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 10 18 5819

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2005/009961 A (BAYER PHARMACEUTICALS CORP [US]; DUMAS JACQUES [US]; BOYER STEPHEN [DE) 3 February 2005 (2005-02-03)<br>* See page 42, lines 15 and 31, and claim 28: combinations with carboplatinum, cisplatinum and with paclitaxel *<br>* column 1 *<br>----- | 1-34 | |
| X | WO 2004/113274 A (BAYER PHARMACEUTICALS CORP [US]; WILHELM SCOTT [US]; DUMAS JACQUES [US) 29 December 2004 (2004-12-29)<br>* claims *<br>* See page 40, lines 28-29 "carboplatinum and cisplatinum" *<br>* See page 41, line 13: "paclitaxel" *<br>----- | 1-34 | |
| X | HEIM M ET AL: "Antitumor effect and potentiation or reduction in cytotoxic drug activity in human colon carcinoma cells by the Raf kinase inhibitor (RKI) BAY 43-9006.",<br>INTERNATIONAL JOURNAL OF CLINICAL PHARMACOLOGY AND THERAPEUTICS DEC 2003, vol. 41, no. 12, December 2003 (2003-12), pages 616-617, XP009079246,<br>ISSN: 0946-1965<br>* See results: page 617 *<br>----- | 1-34 | TECHNICAL FIELDS SEARCHED (IPC) |

The present search report has been drawn up for all claims

1

| Place of search | Date of completion of the search | Examiner |
|---|---|---|
| Munich | 16 May 2011 | Veronese, Andrea |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO. EP 10 18 5819

This annex lists the patent family members relating to the patent documents cited in the above-mentioned European search report.
The members are as contained in the European Patent Office EDP file on
The European Patent Office is in no way liable for these particulars which are merely given for the purpose of information.

16-05-2011

| Patent document cited in search report | Publication date | Patent family member(s) | Publication date |
|---|---|---|---|
| WO 2006026501 A | 09-03-2006 | AT 395905 T | 15-06-2008 |
| | | CA 2578442 A1 | 09-03-2006 |
| | | CN 101048140 A | 03-10-2007 |
| | | EP 1796642 A1 | 20-06-2007 |
| | | ES 2306216 T3 | 01-11-2008 |
| | | JP 2008511686 T | 17-04-2008 |
| WO 2005009961 A | 03-02-2005 | AR 048741 A1 | 24-05-2006 |
| | | AU 2004259760 A1 | 03-02-2005 |
| | | AU 2011201337 A1 | 14-04-2011 |
| | | BR PI0412219 A | 22-08-2006 |
| | | CA 2532865 A1 | 03-02-2005 |
| | | CL 18342004 A1 | 03-06-2005 |
| | | CN 1856469 A | 01-11-2006 |
| | | DE 602004010407 T2 | 16-10-2008 |
| | | DK 1663978 T3 | 07-04-2008 |
| | | EC SP066302 A | 28-07-2006 |
| | | EP 1663978 A2 | 07-06-2006 |
| | | ES 2297490 T3 | 01-05-2008 |
| | | HR 20060073 A2 | 30-06-2006 |
| | | JP 2006528196 T | 14-12-2006 |
| | | KR 20060052866 A | 19-05-2006 |
| | | NZ 544920 A | 27-11-2009 |
| | | NZ 580384 A | 31-03-2011 |
| | | PT 1663978 E | 15-02-2008 |
| | | RS P20060037 A | 05-06-2008 |
| | | SI 1663978 T1 | 29-02-2008 |
| | | US 2005038080 A1 | 17-02-2005 |
| | | US 2009192127 A1 | 30-07-2009 |
| WO 2004113274 A | 29-12-2004 | AT 384264 T | 15-02-2008 |
| | | AT 366108 T | 15-07-2007 |
| | | CA 2526617 A1 | 29-12-2004 |
| | | CA 2526636 A1 | 06-01-2005 |
| | | DE 602004007382 T2 | 17-04-2008 |
| | | DE 602004011340 T2 | 06-11-2008 |
| | | DK 1636585 T3 | 26-05-2008 |
| | | DK 1626714 T3 | 15-10-2007 |
| | | EP 1636585 A2 | 22-03-2006 |
| | | EP 1626714 A2 | 22-02-2006 |
| | | ES 2305808 T3 | 01-11-2008 |
| | | ES 2288694 T3 | 16-01-2008 |
| | | JP 2006528986 T | 28-12-2006 |
| | | JP 2007511203 T | 10-05-2007 |
| | | MX PA05012486 A | 03-07-2006 |
| | | PT 1636585 E | 27-03-2008 |

EPO FORM P0459

For more details about this annex : see Official Journal of the European Patent Office, No. 12/82

## ANNEX TO THE EUROPEAN SEARCH REPORT ON EUROPEAN PATENT APPLICATION NO. EP 10 18 5819

This annex lists the patent family members relating to the patent documents cited in the above-mentioned European search report. The members are as contained in the European Patent Office EDP file on
The European Patent Office is in no way liable for these particulars which are merely given for the purpose of information.

16-05-2011

| Patent document cited in search report | Publication date | Patent family member(s) | Publication date |
|---|---|---|---|
| WO 2004113274 A | | PT 1626714 E | 24-08-2007 |
| | | WO 2005000284 A2 | 06-01-2005 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 0042012 A
- WO 0041698 A
- US 20050038080 A
- WO 04096224 A, Boehringer; Hilberg
- WO 03047579 A
- US 09425228 B
- US 09722418 B
- US 09758547 B
- US 09838285 B
- US 09838286 B
- US 09948915 B
- EP 04023131 A
- EP 04023130 A
- WO 2005009961 A
- WO 2004078747 A
- WO 05000284 A
- WO 9932463 PCT, Miller, S
- WO 9932436 PCT, Dumas, J.
- WO 9932111 PCT, Dumas, J.
- WO 9932106 PCT, Dumas, J.
- WO 9932110 A, Dumas, J.
- WO 9932455 PCT, Riedl, B.
- WO 0042012 PCT, Riedl, B.
- WO 0041698 PCT, Dumas, J.
- US 20020065296 A, Dumas, J.
- WO 0262763 PCT, Dumas, J.
- WO 0285857 PCT, Dumas, J.
- US 20020165394 A
- WO 0042021 A
- WO 03068228 A
- WO 200500996 A
- WO 2005000284 A
- WO 94041573 A, Lipp
- US 5192744 A
- US 6024688 A
- US 6060449 A
- US 60859241 B


### Non-patent literature cited in the description


- **ALBAIN KS ; CROWLEY JJ ; LEBLANC M et al.** Survival determinants in extensive-stage non-small-cell lung cancer: the Southwest Oncology Group experience. *Journal of Clinical Oncology,* 1991, vol. 9 (9), 1618-1626
- **MACCHIARINI P ; FONTANINI G ; HARDIN MJ et al.** Blood vessel invasion by tumor cells predicts recurrence in completely resected Tl N0 M0 non-small-cell lung cancer. *Journal of Thoracic and Cardiovascular Surgery,* 1993, vol. 106 (1), 80-89
- **HARPOLE DH ; HERNDON JE ; WOLFE WG et al.** A prognostic model of recurrence and death in stage I non-small cell lung cancer utilizing presentation, histopathology, and oncoprotein expression. *Cancer Research,* 1995, vol. 55 (1), 51-56
- **ICHINOSE Y ; YANO T ; ASOH H et al.** Prognostic factors obtained by a pathologic examination in completely resected non-small-cell lung cancer: an analysis in each pathologic stage. *Journal of Thoracic and Cardiovascular Surgery,* 1995, vol. 110 (3), 601-605
- **MARTINI N ; BAINS MS ; BURT ME et al.** Incidence of local recurrence and second primary tumors in resected stage I lung cancer. *Journal of Thoracic and Cardiovascular Surgery,* 1995, vol. 109 (1), 120-129
- **STRAUSS GM ; KWIATKOWSKI DJ ; HARPOLE DH et al.** Molecular and pathologic markers in stage I non-small-cell carcinoma of the lung. *Journal of Clinical Oncology,* 1995, vol. 13 (5), 1265-1279
- **SLEBOS RJ ; KIBBELAAR RE ; DALESIO O et al.** K-RAS oncogene activation as a prognostic marker in adenocarcinoma of the lung. *New England Journal of Medicine,* 1990, vol. 323 (9), 561-565
- **FONTANINI G ; BIGINI D ; VIGNATI S et al.** Microvessel count predicts metastatic disease and survival in non-small cell lung cancer. *Journal of Pathology,* 1995, vol. 177, 57-63
- **SOUQUET PJ ; CHAUVIN F ; BOISSEL JP et al.** Polychemotherapy in advanced non small cell lung cancer: a meta-analysis. *Lancet,* 1993, vol. 342 (8862), 19-21
- Non-small Cell Lung Cancer Collaborative Group: Chemotherapy in non-small cell lung cancer: a meta-analysis using updated data on individual patients from 52 randomised clinical trials. *British Medical Journal,* 1995, vol. 311 (7010), 899-909

- **HARDY JR ; NOBLE T ; SMITH IE.** Symptom relief with moderate dose chemotherapy (mitomycin-C, vinblastine and cisplatin) in advanced non-small cell lung cancer. *British Journal of Cancer,* 1989, vol. 60 (5), 764-766
- **ELLIS PA ; SMITH IE ; HARDY JR et al.** Symptom relief with MVP (mitomycin C, vinblastine and cisplatin) chemotherapy in advanced non-small-cell lung cancer. *British Journal of Cancer,* 1995, vol. 71 (2), 366-370
- **WEICK JK ; CROWLEY J ; NATALE RB et al.** A randomized trial of five cisplatin-containing treatments in patients with metastatic non-small-cell lung cancer: a Southwest Oncology Group study. *Journal of Clinical Oncology,* 1991, vol. 9 (7), 1157-1162
- **O'CONNELL JP ; KRIS MG ; GRALLA RJ et al.** Frequency and prognostic importance of pretreatment clinical characteristics in patients with advanced non-small-cell lung cancer treated with combination chemotherapy. *Journal of Clinical Oncology,* 1986, vol. 4 (11), 1604-1614
- **LE CHEVALIER T ; BRISGAND D ; DOUILLARD JY et al.** Randomized study of vinorelbine and cisplatin versus vindesine and cisplatin versus vinorelbine alone in advanced non-small-cell lung cancer: results of a European multicenter trial including 612 patients. *Journal of Clinical Oncology,* 1994, vol. 12 (2), 360-367
- **CHANG AY ; KIM K ; GLICK J et al.** Phase II study of taxol, merbarone, and piroxantrone in stage IV non-small-cell lung cancer: the Eastern Cooperative Oncology Group results. *Journal of the National Cancer Institute,* 1993, vol. 85 (5), 388-394
- **MURPHY WK ; FOSSELLA FV ; WINN RJ et al.** Phase II study of taxol in patients with untreated advanced non-small-cell lung cancer. *Journal of the National Cancer Institute,* 1993, vol. 85 (5), 384-388
- **JOHNSON DH ; PAUL DM ; HANDE KR et al.** Paclitaxel plus carboplatin in advanced non-small-cell lung cancer: a phase II trial. *Journal of Clinical Oncology,* 1996, vol. 14 (7), 2054-2060
- **LANGER CJ ; LEIGHTON JC ; COMIS RL et al.** Paclitaxel and carboplatin in combination in the treatment of advanced non-small-cell lung cancer: a phase II toxicity, response, and survival analysis. *Journal of Clinical Oncology,* 1995, vol. 13 (8), 1860-1870
- **BONOMI P ; KIM K ; CHANG A et al.** Phase III trial comparing etoposide (E) cisplatin (C) versus taxol (T) with cisplatin-G-CSF(G) versus taxol-cisplatin in advanced non-small cell lung cancer. An Eastern Cooperative Oncology Group (ECOG) trial. *Proceedings of the American Society of Clinical Oncology,* 1996, vol. 15 (A-1145), 382
- **SOUQUET PJ ; CHAUVIN F ; BOISSEL JP et al.** Polychemotherapy in advanced non small cell lung cancer: a meta-analysis. *Lancet,* 1993, vol. 342 (8862), 19-21
- **PATCHELL RA ; TIBBS PA ; WALSH JW et al.** A randomized trial of surgery in the treatment of single metastases to the brain. *New England Journal of Medicine,* 1990, vol. 322 (8), 494-500
- **MANDELL L ; HILARIS B ; SULLIVAN M et al.** The treatment of single brain metastasis from non-oat cell lung carcinoma: surgery and radiation versus radiation therapy alone. *Cancer,* 1986, vol. 58 (3), 641-649
- **LOEFFLER JS ; KOOY HM ; WEN PY et al.** The treatment of recurrent brain metastases with stereotactic radiosurgery. *Journal of Clinical Oncology,* 1990, vol. 8 (4), 576-582
- **DEANGELIS LM ; MANDELL LR ; THALER HT et al.** The role of postoperative radiotherapy after resection of single brain metastases. *Neurosurgery,* 1989, vol. 24 (6), 798-805
- **ALEXANDER E ; MORIARTY TM ; DAVIS RB et al.** Stereotactic radiosurgery for the definitive, noninvasive treatment of brain metastases. *Journal of the National Cancer Institute,* 1995, vol. 87 (1), 34-40
- **ARBIT E ; WRONSKI M ; BURT M et al.** The treatment of patients with recurrent brain metastases: a retrospective analysis of 109 patients with nonsmall cell lung cancer. *Cancer,* 1995, vol. 76 (5), 765-773
- **HAZUKA MB ; KINZIE JJ.** Brain metastases: results and effects of re-irradiation. *International Journal of Radiation Oncology, Biology, Physics,* 1988, vol. 15 (2), 433-437
- **SALERNO TA ; MUNRO DD ; BLUNDELL PE et al.** Second primary bronchogenic carcinoma: life-table analysis of surgical treatment. *Annals of Thoracic Surgery,* 1979, vol. 27 (1), 3-6
- **YELLIN A ; HILL LR ; BENFIELD JR.** Bronchogenic carcinoma associated with upper aerodigestive cancer. *Journal ofThoracic and Cardiovascular Surgery,* 1986, vol. 91 (5), 674-683
- Medical Research Council Lung Cancer Working Party: Randomized trial of etoposide cyclophosphamide methotrexate and vincristine versus etoposide and vincristine in the palliative treatment of patients with small-cell lung cancer and poor prognosis. *British Journal of Cancer,* 1993, vol. 67 (20), A-4, 2, 14
- **VEEDER MH ; JETT JR ; SU JQ et al.** A phase III trial of mitomycin C alone versus mitomycin C, vinblastine, and cisplatin for metastatic squamous cell lung carcinoma. *Cancer,* 1992, vol. 70 (9), 2281-2287

- **BONOMI P ; KIM K ; CHANG A et al.** Phase III trial comparing etoposide (E) cisplatin (C) versus taxol (T) with cisplatin-G-CSF(G) versus taxol-cisplatin in advanced non-small cell lung cancer. An Eastern Cooperative Oncology Group (ECOG) trial. *Proceedings of the American Society of Clinical Oncology,* 1996, vol. 15, A-1145, 382
- **ROSELL R ; TONATO M ; SANDLER A.** The activity of gemcitabine plus cisplatin in randomized trials in untreated patients with advanced non-small cell lung cancer. *Seminars in Oncology,* 1998, vol. 25 (4), 27-34
- **MILLER JI ; PHILLIPS TW.** Neodymium:YAG laser and brachytherapy in the management of inoperable bronchogenic carcinoma. *Annals of Thoracic Surgery,* 1990, vol. 50 (2), 190-196
- **SMITH et al.** *Bioorg. Med. Chem. Lett.,* 2001, vol. 11, 2775-2778
- **LOWINGER et al.** *Clin. Cancer Res.,* 2000, vol. 6, 335
- **LYONS et al.** *Endocr.-Relat. Cancer,* 2001, vol. 8, 219-225
- **LOWINGER et al.** *Curr. Pharm. Design,* 2002, vol. 8, 99-110
- **LEE ; MCCUBREY.** *Curr. Opin. Investig. Drugs,* June 2003, vol. 4, 657-63
- **WILHELM et al.** *Cancer Res.,* 2004, vol. 64, 7099-7109
- *Organic Process Research and Development,* 2002, vol. 6 (6), 777-781
- **BANKSTON et al.** *Organic Process Research & Development,* 2002, vol. 6, 777-781
- **S. M. BERGE et al.** Pharmaceutical Salts. *J. Pharm. Sci.,* 1977, vol. 66, 1-19
- Prodrugs As Novel Drug Delivery Systems. American Chemical Society, 1975
- **ROCHE, E. B.** Design of Biopharmaceutical Properties through Prodrugs and Analogs. American Pharmaceutical Association, 1977
- **SINKULA, A. A. ; YALKOWSKY, S. H.** *J Pharm Sci.,* 1975, vol. 64, 181-210
- **STELLA, V. J. ; CHARMAN, W. N. ; NARINGREKAR, V. H.** *Drugs,* 1985, vol. 29, 455-473
- Design of Prodrugs. Elsevier, 1985
- **STELLA, V. J. ; HIMMELSTEIN, K. J.** *J. Med. Chem.,* 1980, vol. 23, 1275-1282
- **HAN, H-K ; AMIDON, G. L.** *AAPS Pharmsci,* 2000, vol. 2, 1-11
- **DENNY, W. A.** *Eur. J. Med. Chem.,* 2001, vol. 36, 577-595
- **WERMUTH, C. G.** The Practice of Medicinal Chemistry. Academic Press, 1996, 697-715
- **BALANT, L. P. ; DOELKER, E.** Burgers Medicinal Chemistry And Drug Discovery. John Wiley & Sons, 1997, 949-982
- **RYLANDER.** Hydrogenation Methods. Academic Press, 1985
- Reductions by the Alumino- and borohydrides. **SEYDEN-PENNE.** Organic Synthesis. VCH Publishers, 1991
- **MARCH.** Advanced Organic Chemistry. John Wiley, 1985
- **LAROCK.** Comprehensive Organic Transformations. VCH Publishers, 1989
- **MARKGRAF et al.** *Tetrahedron,* 1991, vol. 47, 183
- **COPERET et al.** *Terahedron Lett.,* 1998, vol. 39, 761
- **AMONE et al.** *Tetrahedron,* 1998, vol. 54, 7831
- **DAYAN et al.** *Synthesis,* 1427
- **ROBKER et al.** *J. Chem. Res., Synop.,* 1993, vol. 10, 412
- **KLEMM et al.** *J. Heterocylic Chem.,* 1990, vol. 6, 1537
- **LIN A.J.** *Org. Prep. Proced, Int.,* 1991, vol. 23 (1), 114
- **BOYD et al.** *J. Chem. Soc., Perkin Trans.,* 1991, vol. 9, 2189
- **J. MARCH.** Advanced Organic Chemistry. John Wiley, 1992
- **R.C. LAROCK.** Comprehensive Organic Transformations. Wiley-VCH, 1999
- **F.A. CAREY ; R.J. SUNDBERG.** Advanced Organic Chemistry. Plenum Press, 1984
- **T.W. GREENE ; P.G.M. WUTS.** Protective Groups in Organic Synthesis. John Wiley, 1999
- **L.S. HEGEDUS.** Transition Metals in the Synthesis of Complex Organic Molecules. University Science Books, 1994
- The Encyclopedia of Reagents for Organic Synthesis. John Wiley, 1994
- Comprehensive Organic Functional Group Transformations. Pergamon Press, 1995
- Comprehensive Organometallic Chemistry. Pergamon Press, 1982
- **B.M. TROST ; I. FLEMING.** Comprehensive Organic Synthesis. Pergamon Press, 1991
- Comprehensive Heterocylic Chemistry. Pergamon Press, 1984
- Comprehensive Heterocylic Chemistry II. Pergamon Press, 1996
- Comprehensive Medicinal Chemistry. Pergamon Press, 1990
- Organic Reactions. John Wiley
- Organic Syntheses. John Wiley
- Reagents for Organic Synthesis. John Wiley
- The Total Synthesis of Natural Products. John Wiley
- The Organic Chemistry of Drug Synthesis. John Wiley
- Annual Reports in Organic Synthesis. Academic Press
- **HOUBEN-WEYL.** Methoden der Organischen Chemie. Thieme

- **CHIEN.** Transdermal Controlled Systemic Medications. Marcel Dekker, Inc, 1987
- **REMINGTON.** The Science and Practice of Pharmacy. Lippincott Williams & Wilkins, 2000
- Theory and Practice of Industrial Pharmacy. Lippincott Williams & Wilkins, 1986
- Encyclopedia of Pharmaceutical Technology. Marcel Dekker, 2002
- **PASSANITI et al.** *Lab. Invest.,* 1992, vol. 67, 519-528
- **TAYLOR ; FOLKMAN.** *Nature,* 1982, vol. 297, 307-312
- **ELICEIRI et al.** *J. Cell Biol.,* 1998, vol. 140, 1255-1263
- **POLVERINI, P. J. et al.** *Methods Enzymol.,* 1991, vol. 198, 440-450
- **WILHELM SM ; CARTER C ; TANG L et al.** BAY 43-9006 exhibits broad spectrum oral antitumor activity and targets the RAF/MEK/ERK pathway and receptor tyrosine kinases involved in tumor progression and angiogenesis. *Cancer Res,* 2004, vol. 64, 7099-109
- **CARLOMAGNO F ; ANAGANTI S ; GUIDA T et al.** BAY 43-9006 inhibition of oncogenic RET mutants. *J Natl Cancer Inst,* 2006, vol. 98, 326-34
- **LEVY JB ; PAULOSKI N ; BRAUN D et al.** Analysis of transcription and protein expression changes in the 786-O human renal cell carcinoma tumor xenograft model in response to treatment with the multi-kinase inhibitor sorafenib (BAY 43-9006. *AACR Meeting Abstracts,* 2006, 213-d-214
- **SHARMA A ; TRIVEDI NR ; ZIMMERMAN MA et al.** Mutant V599EB-Raf Regulates Growth and Vascular Development of Malignant Melanoma Tumors. *Cancer Res,* 2005, vol. 65, 2412-2421
- **STRUMBERG D ; RICHLY H ; HILGER RA et al.** Phase I clinical and pharmacokinetic study of the Novel Raf kinase and vascular endothelial growth factor receptor inhibitor BAY 43-9006 in patients with advanced refractory solid tumors. *J Clin Oncol,* 2005, vol. 23, 965-72
- **RATAIN MJ ; EISEN T ; STADLER WM et al.** Phase II placebo-controlled randomized discontinuation trial of sorafenib in patients with metastatic renal cell carcinoma. *J Clin Oncol,* 2006, vol. 24, 2505-12
- **ESCUDIER B ; SZCZYLIK C ; EISEN T et al.** Randomized phase III trial of the Raf kinase and VEGFR inhibitor sorafenib (BAY 43-9006) in patients with advanced renal cell carcinoma (RCC. *J Clin Oncol (Meeting Abstracts,* 2005, vol. 23, LBA4510
- **HEIM M ; SHARIFI M ; HILGER RA et al.** Antitumor effect and potentiation or reduction in cytotoxic drug activity in human colon carcinoma cells by the Raf kinase inhibitor (RKI) BAY 43-9006. *Int J Clin Pharmacol Ther,* 2003, vol. 41, 616-7
- **CARTER CA ; CHEN C ; BRINK C et al.** Sorafenib is efficacious and tolerated in combination with cytotoxic or cytostatic agents in preclinical models of human non-small cell lung carcinoma. *Cancer Chemother Pharmacol,* 2006
- **MROSS K ; STEINBILD S ; BAAS F et al.** Drug-drug interaction pharmacokinetic study with the Raf kinase inhibitor (RKI) BAY 43-9006 administered in combination with irinotecan (CPT-11) in patients with solid tumors. *Int J Clin Pharmacol Ther,* 2003, vol. 41, 618-9
- **KUPSCH P ; HENNING BF ; PASSARGE K et al.** Results of a phase I trial of sorafenib (BAY 43-9006) in combination with oxaliplatin in patients with refractory solid tumors, including colorectal cancer. *Clin Colorectal Cancer,* 2005, vol. 5, 188-96
- **RICHLY H ; HENNING BF ; KUPSCH P et al.** Results of a Phase I trial of sorafenib (BAY 43-9006) in combination with doxorubicin in patients with refractory solid tumors. *Ann Oncol,* 2006, vol. 17, 866-73
- **SIU LL ; AWADA A ; TAKIMOTO CH et al.** Phase I trial of sorafenib and gemcitabine in advanced solid tumors with an expanded cohort in advanced pancreatic cancer. *Clin Cancer Res,* 2006, vol. 12, 144-51
- **RICHLY H ; KUPSCH P ; PASSAGE K et al.** Results of a phase I trial of BAY 43-9006 in combination with doxorubicin in patients with primary hepatic cancer. *Int J Clin Pharmacol Ther,* 2004, vol. 42, 650-1
- **AHMAD T ; EISEN T.** Kinase inhibition with BAY 43-9006 in renal cell carcinoma. *Clin Cancer Res,* 2004, vol. 10, 6388S-92S
- **WIERNIK PH ; SCHWARTZ EL ; EINZIG A et al.** Phase I trial of taxol given as a 24-hour infusion every 21 days: responses observed in metastatic melanoma. *J Clin Oncol,* 1987, vol. 5, 1232-9
- **LEGHA SS ; RING S ; PAPADOPOULOS N et al.** A phase II trial of taxol in metastatic melanoma. *Cancer,* 1990, vol. 65, 2478-81
- **EINZIG AI ; HOCHSTER H ; WIERNIK PH et al.** A phase II study of taxol in patients with malignant melanoma. *Invest New Drugs,* 1991, vol. 9, 59-64
- **AAMDAL S ; WOLFF I ; KAPLAN S et al.** Docetaxel (Taxotere) in advanced malignant melanoma: a phase II study of the EORTC Early Clinical Trials Group. *Eur J Cancer,* 1994, vol. 30A, 1061-4
- **BEDIKIAN AY ; WEISS GR ; LEGHA SS et al.** Phase II trial of docetaxel in patients with advanced cutaneous malignant melanoma previously untreated with chemotherapy. *J Clin Oncol,* 1995, vol. 13, 2895-9
- **EINZIG AI ; SCHUCHTER LM ; RECIO A et al.** Phase II trial of docetaxel (Taxotere) in patients with metastatic melanoma previously untreated with cytotoxic chemotherapy. *Med Oncol,* 1996, vol. 13, 111-7

- **ZIMPFER-RECHNER C ; HOFMANN U ; FIGL R et al.** Randomized phase II study of weekly paclitaxel versus paclitaxel and carboplatin as second-line therapy in disseminated melanoma: a multicentre trial of the Dermatologic Co-operative Oncology Group (DeCOG. *Melanoma Res,* 2003, vol. 13, 531-6
- **HODI FS ; SOIFFER RJ ; CLARK J et al.** Phase II study of paclitaxel and carboplatin for malignant melanoma. *Am J Clin Oncol,* 2002, vol. 25, 283-6
- **RAO RD ; HOLTAN SG ; INGLE JN et al.** Combination of paclitaxel and carboplatin as second-line therapy for patients with metastatic melanoma. *Cancer,* 2006, vol. 106, 375-82
- **BROSE MS ; VOLPE P ; FELDMAN M et al.** BRAF and RAS mutations in human lung cancer and melanoma. *Cancer Res,* 2002, vol. 62, 6997-7000
- **KENT LL ; HULL-CAMPBELL NE ; LAU T et al.** Characterization of novel inhibitors of cyclin-dependent kinases. *Biochem Biophys Res Commun,* 1999, vol. 260, 768-74
- **SCHILLER JH ; HARRINGTON D ; BELANI CP et al.** Comparison of four chemotherapy regimens for advanced non-small-cell lung cancer. *N Engl J Med,* 2002, vol. 346, 92-8
- **STRUMBERG D ; VOLIOTIS D ; MOELLER JG et al.** Results of phase I pharmacokinetic and pharmacodynamic studies of the Raf kinase inhibitor BAY 43-9006 in patients with solid tumors. *Int J Clin Pharmacol Ther,* 2002, vol. 40, 580-1
- **DAVIES H ; BIGNELL GR ; COX C et al.** Mutations of the BRAF gene in human cancer. *Nature,* 2002, vol. 417, 949-54